(19) Europäisches Patentamt / European Patent Office / Office européen des brevets

(11) **EP 4 379 727 A1**

(12) **EUROPEAN PATENT APPLICATION**

(43) Date of publication:
**05.06.2024 Bulletin 2024/23**

(21) Application number: **23209657.8**

(22) Date of filing: **24.09.2018**

(51) International Patent Classification (IPC):
**G16B 40/00** (2019.01)    **G16B 40/20** (2019.01)
**C12Q 1/6848** (2018.01)    **C12Q 1/6869** (2018.01)

(52) Cooperative Patent Classification (CPC):
(C-Sets available)
**G16B 40/00; C12Q 1/6848; C12Q 1/6869;**
C12Q 2549/10; G16B 20/00; G16B 25/00;
G16B 30/10                                    (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **25.09.2017 US 201762562978 P**

(62) Document number(s) of the earlier application(s) in
accordance with Art. 76 EPC:
**18786527.4 / 3 688 763**

(71) Applicant: **Becton, Dickinson and Company
Franklin Lakes, NJ 07417-1880 (US)**

(72) Inventors:
• **SHUM, Eleen
  Menlo Park, 94025 (US)**
• **FAN, Jue
  Menlo Park, 94025 (US)**
• **TSAI, Jennifer
  Menlo Park, 94025 (US)**

(74) Representative: **Carpmaels & Ransford LLP
One Southampton Row
London WC1B 5HA (GB)**

Remarks:
•The complete document including Reference
Table(s) and the Sequence Listing(s) can be
downloaded from the EPO website
•This application was filed on 14.11.2023 as a
divisional application to the application mentioned
under INID code 62.
•Claims filed after the date of receipt of the divisional
application (Rule 68(4) EPC).

(54) **IMMUNE RECEPTOR-BARCODE ERROR CORRECTION**

(57) Disclosed herein are methods and systems for determining occurrences of targets. In some embodiments, the method comprises: collapsing putative sequences of the target; collapsing molecular label sequences associated with the putative sequences of the target; and estimating the occurrence of the target, wherein the occurrence of the target estimated correlates with the occurrence of molecular label sequences associated with the putative sequences of the target in the sequencing data after collapsing the occurrence of the putative sequences of the target and the occurrence of noise molecular label sequences.

FIG. 7

(52) Cooperative Patent Classification (CPC): (Cont.)

C-Sets
**C12Q 1/6848, C12Q 2537/165, C12Q 2563/185**

**Description**

RELATED APPLICATIONS

The present application claims priority to U.S. Provisional Application No. 62/562,978, filed on September 25, 2017. The content of the related application is herein expressly incorporated by reference in its entirety.

REFERENCE TO SEQUENCE LISTING

The present application is being filed along with a Sequence Listing in electronic format. The Sequence Listing is provided as a file entitled Sequence_Listing_BDCRI_035WO.txt, created September 21, 2018, which is 32 kilobytes in size. The information in the electronic format of the Sequence Listing is incorporated herein by reference in its entirety.

BACKGROUND

Field

The present disclosure relates generally to the field of molecular barcoding and more particularly correcting substitution and non-substitution errors using molecular labels.

Description of the Related Art

Methods and techniques such as barcoding (including stochastic barcoding) are useful for cell analysis, in particular deciphering gene expression profiles to determine the states of cells using, for example, reverse transcription, polymerase chain reaction (PCR) amplification, and next generation sequencing (NGS). However, these methods and techniques can introduce errors such as substitution errors (e.g., substitution errors involving one or more base substitutions) and non-substitution errors (e.g., primer crossover errors, and PCR chimera errors), if uncorrected, may result in over-estimated molecular counts. Thus, there is a need for methods and techniques capable of correcting various errors in order to attain accurate molecular counts.

SUMMARY

Disclosed herein include methods for determining occurrences of targets. In some embodiments, the method comprises: (a) barcoding (e.g., stochastically barcoding) a plurality of targets using a plurality of barcodes (e.g., stochastic barcodes) to create a plurality of barcoded targets (e.g., stochastically barcoded targets), wherein each of the plurality of barcodes comprises a cell label and a molecular label, wherein molecular labels of at least two barcodes of the plurality of barcodes comprise different molecular label sequences, and wherein at least two barcodes of the plurality of barcodes comprise cell labels with an identical cell label sequence; (b) obtaining sequencing data of the barcoded targets; and (c) for at least one target of the plurality of targets: (i) identifying putative sequences of the target in the sequencing data; (ii) counting occurrences of molecular label sequences associated with the putative sequences of the target in the sequencing data identified in (i); (iii) identifying clusters of the putative sequences of the target; (iv) collapsing the sequencing data obtained using the clusters of putative sequences of the target identified in (iii); (v) identifying clusters of the molecular label sequences associated with the putative sequences of the target; (vi) collapsing the sequencing data using the clusters of molecular label sequences identified in (v); (vii) identifying clusters of combination sequences, wherein each combination sequence comprises a sequence of the sequences of the target and an associated molecular label sequence of the molecular label sequences; (viii) collapsing the sequencing data using the clusters of combination sequences identified in (vii); (ix) identifying one or more putative sequences of the target that correspond to one or more chimeric sequences of the target, wherein occurrences of the one or more putative sequences of the target that correspond to the one or more chimeric sequences of the target are smaller than occurrences of remaining one or more putative sequences of the target that do not correspond to the one or more chimeric sequences of the target; (x) removing the one or more putative sequences of the target corresponding to the one or more chimeric sequences of the target identified in (ix) from the sequencing data; and (xi) estimating the occurrence of the target, wherein the occurrence of the target estimated correlates with the number of molecular label sequences counted in (ii) after collapsing the sequencing data in (iv), (vi), and (viii) and removing the one or more putative sequences of the target that correspond to the one or more chimeric sequences of the target in (x).

In some embodiments, the plurality of targets comprises targets of the whole transcriptome of a cell. The plurality of targets can comprise a gene. The gene can comprise a variable sequence, such as a variable (V) region, a diversity (D) region, a joining (J) region, or any combination thereof, encoding an immune receptor. The gene can be a gene encoding a T-cell Receptor. The putative sequences of the target can differ from one another by at least one nucleotide.

In some embodiments, identifying the clusters of the putative sequences of the target comprises identifying the clusters of the putative sequences of the target using directional adjacency. Putative sequences of the target within a cluster can be within a first predetermined directional adjacency threshold of one another. The first directional adjacency threshold can be a Hamming distance of one. The putative sequences of the target within the cluster can comprise one or more parent sequences and one or more children sequences of the one or more parent sequences, and wherein an occurrence of the parent sequence is greater than or equal to a first predetermined directional adjacency occurrence threshold. The first predetermined directional adjacency occurrence threshold can be twice an occurrence of a child sequence less one.

In some embodiments, collapsing the sequencing data obtained in (b) using the clusters of putative sequences of the target identified in (iii) comprises: attributing an occurrence of a child sequence of the one or more children sequences to the parent sequence of the child sequence.

In some embodiments, identifying the clusters of the molecular label sequences associated with the putative sequences of the target comprises identifying the clusters of the molecular label sequences associated with the putative sequences of the target using directional adjacency. Molecular label sequences of the target within a cluster can be within a second predetermined directional adjacency threshold of one another. The second directional adjacency threshold can be a Hamming distance of one. The putative molecular label sequences of the target within the cluster can comprise one or more parent molecular label sequences and one or more children molecular label sequences of the one or more parent molecular label sequences, and wherein the occurrence of the parent molecular label sequence is greater than or equal to a second predetermined directional adjacency occurrence threshold. The second predetermined directional adjacency occurrence threshold can be twice an occurrence of a child molecular label sequence less one.

In some embodiments, collapsing the sequencing data using the clusters of molecular label sequences associated with the sequences of the target identified in (v) comprises: attributing an occurrence of a child molecular label sequence of the one or more children molecular label sequences to the parent molecular label of the child molecular label sequence.

In some embodiments, identifying the clusters of combination sequences comprises identifying clusters of combination sequences using directional adjacency. Combination sequences within a cluster can be within a third predetermined directional adjacency threshold of one another. The third directional adjacency threshold can be a Hamming distance of one. The combination sequences within the cluster can comprise one or more parent combination sequences and one or more children combination sequences of the one or more parent combination sequences, and wherein an occurrence of the parent combination sequence is greater than or equal to a third predetermined directional adjacency occurrence threshold. The third predetermined directional adjacency occurrence threshold can be twice an occurrence of a child combination sequence less one.

In some embodiments, collapsing the sequencing data using the clusters of combination sequences identified in (vii) comprises: attributing an occurrence of a child combination sequence of the one or more children combination sequences to the parent combination sequence of the child combination sequence.

In some embodiments, identifying the one or more putative sequences of the target corresponding to the one or more chimeric sequences of the target: identifying putative sequences of the target associated with one molecular label sequence of the plurality of molecular sequences; identifying a putative sequence of the putative sequences of the target associated with the one molecular label sequence with an occurrence smaller than a chimeric occurrence threshold as corresponding to a chimeric sequence of the one or more chimeric sequences of the target. A value of the chimeric occurrence threshold can be an occurrence of a putative sequence of the putative sequences of the target associated with the one molecular label sequence that is greater than an occurrence of any other sequence of the putative sequences of the target.

In some embodiments, the method further comprises: adjusting the sequencing data after collapsing the sequencing data in (iv), (vi), and (viii) and removing the one or more putative sequences of the target that correspond to the one or more chimeric sequences of the target in (x). Adjusting the sequencing data after collapsing the sequencing data in (iv), (vi), and (viii) and removing the one or more putative sequences of the target that correspond to the one or more chimeric sequences of the target in (x) can comprise: thresholding molecular label sequences associated with the putative sequences of the target to determine signal molecular label sequences and noise molecular label sequences associated with the sequences of the target in the sequencing data counted in (b) after collapsing the sequencing data in (iv), (vi), and (viii) and removing the one or more putative sequences of the target corresponding to the one or more chimeric sequences of the target in (x). Thresholding the molecular label sequences associated with the putative sequences of the target can comprise performing a statistical analysis on the molecular label sequences of the target. Performing the statistical analysis can comprise: fitting the molecular label sequences associated with the putative sequences of the target and their occurrences to two negative binomial distributions; determining an occurrence of signal molecular label sequences n using the two negative binomial distributions; and removing the noise molecular label sequences from the sequencing data obtained in (b) after collapsing the sequencing data in (iv), (vi), and (viii) and removing the one or more putative sequences of the target that correspond to the one or more chimeric sequences of the target in (x), wherein the noise molecular label sequences comprise molecular label sequences with occurrences lower than an occurrence of the nth most abundant molecular label, and wherein the signal molecular label sequences comprise molecular label sequences with occurrences greater than or equal to the occurrence of the nth most abundant molecular label. The two negative binomial distributions can comprise a first negative binomial distribution corresponding for the signal molecular label sequences and a second negative binomial distribution for the noise molecular label sequences.

Disclosed herein are methods for determining occurrences of targets. In some embodiments, the method comprises: (a) receiving sequencing data of a plurality of targets, wherein the sequencing data comprises putative sequences of a target of the plurality of targets and occurrences of molecular label sequences associated with the sequences of the target in the sequencing data; (b) collapsing putative sequences of the target; (c) collapsing molecular label sequences associated with the putative sequences of the target; and (d) estimating the occurrence of the target, wherein the occurrence of the target estimated correlates with the occurrence of molecular label sequences associated with the putative sequences of the target in the sequencing data after collapsing the occurrence of the putative sequences of the target in (b) and the occurrence of noise molecular label sequences determined in (c).

In some embodiments, the method comprises: identifying the sequences of the target in the sequencing data; and counting the occurrences of molecular label sequences associated with the sequences of the target in the sequencing data.

In some embodiments, the method comprises: collapsing clusters of combination sequences, wherein each combination sequence comprises a sequence of the sequences of the target and an associated molecular label sequence of the molecular label sequences, wherein the occurrence of the target estimated correlates with the occurrence of molecular label sequences associated with the sequences of the target in the sequencing data after collapsing the occurrence of the combination sequences. Collapsing the clusters of combination sequences can comprise: collapsing the clusters of combination sequences using directional adjacency. Collapsing the clusters of combination sequences using directional adjacency can comprise: identifying the clusters of combination sequences using directional adjacency; and collapsing the sequencing data using the clusters of combination sequences identified. Collapsing the putative sequences of the target comprises: collapsing the putative sequences of the target using directional adjacency. Collapsing the putative sequences of the target using directional adjacency can comprise: identifying clusters of the putative sequences of the target using directional adjacency; and collapsing the sequencing data using the clusters of putative sequences of the target identified. In some embodiments, collapsing the molecular label sequences associated with the putative sequences of the target comprises: collapsing the molecular label sequences associated with the putative sequences of the target using directional adjacency. Collapsing the molecular label sequences associated with the putative sequences of the target using directional adjacency can comprise: identifying clusters of the molecular label sequences associated with the putative sequences of the target using directional adjacency; and collapsing the sequencing data using the clusters of molecular label sequences associated with the putative sequences of the target identified.

In some embodiments, the method comprises: identifying one or more putative sequences of the target that correspond to one or more chimeric sequences of the target, wherein occurrences of the one or more putative sequences of the target that correspond to the one or more chimeric sequences of the target are smaller than occurrences of remaining one or more putative sequences of the target that do not correspond to the one or more chimeric sequences of the target; and removing from the sequencing data one or more putative sequences of the target that correspond to one or more chimeric sequences of the target. Identifying the one or more putative sequences of the target corresponding to the one or more chimeric sequences of the target can comprise: identifying putative sequences of the target associated with one molecular label sequence of the plurality of molecular sequences; identifying a putative sequence of the putative sequences of the target associated with the one molecular label sequence with an occurrence smaller than a chimeric occurrence threshold as corresponding to a chimeric sequence of the one or more chimeric sequences of the target. A value of the chimeric occurrence threshold can be an occurrence of a putative sequence of the putative sequences of the target associated with the one molecular label sequence that is greater than an occurrence of any other sequence of the putative sequences of the target.

In some embodiments, the method comprises: determining a sequencing status of the target in the sequencing data; and determining an occurrence of noise molecular label sequences associated with the putative sequences of the target in the sequencing data, wherein the occurrence of the target estimated correlates with the occurrence of the molecular label sequences associated with the putative sequences of the target in the sequencing data adjusted according to the occurrence of the noise molecular label sequences. Sequencing status of the target in the sequencing data can be saturated sequencing, under sequencing, or over sequencing.

In some embodiments, the under sequencing status can be determined by the target having a depth smaller than a predetermined under sequencing threshold, and wherein the depth of the target comprises an average, a minimum, or a maximum depth of the molecular label sequences associated with the putative sequences of the target in the sequencing data. The under sequencing threshold can be about four. The under sequencing threshold can be independent of the number of molecular label sequences. If the sequencing status of the target in the sequencing data is the under sequencing status, the number of noise molecular label sequences determined can be zero.

In some embodiments, the saturated sequencing status is determined by the number of molecular label sequences associated with the putative sequences of the target greater than a saturation threshold. The saturation threshold can be about 6557 if a molecular label sequence of the molecular label sequences associated with the putative sequences of the target has a sequence selected from about 6561 molecular label sequences. The predetermined saturation threshold can be about 65532 if a molecular label sequence of the molecular label sequences associated with the putative sequences of the target has a sequence selected from about 65536 molecular label sequences. If the sequencing status of the target in the sequencing data is the saturated sequencing status, the number of noise molecular label sequences determined can be zero.

In some embodiments, the over sequencing status is determined by the target having a depth greater than a predetermined over sequencing threshold, wherein the depth of the target comprises an average, a minimum, or a maximum depth of the molecular label sequences associated with the putative sequences of the target in the sequencing data. The over sequencing threshold can be about 250 if a molecular label sequence of the molecular label sequences associated with the putative sequences of the target has a sequence selected from about 6561 molecular label sequences. In some embodiments, the method can comprise: if the sequencing status of the target in the sequencing data is saturated sequencing status or the over sequencing status: subsampling the number of molecular label sequences associated with the sequences of the target in the sequencing data to about the predetermined over sequencing threshold.

In some embodiments, determining the occurrence of noise molecular label sequences associated with the putative sequences of the target in the sequencing data comprises: if a negative binomial distribution fitting condition is satisfied, fitting a signal negative binomial distribution to the occurrences of the molecular label sequences associated with the sequences of the target in the sequencing data, wherein the signal negative binomial distribution corresponds to an occurrence of molecular label sequences associated with the sequences of the target in the sequencing data being signal molecular label sequences; fitting a noise negative binomial distribution to the occurrences of the molecular label sequences associated with the sequences of the target in the sequencing data, wherein the noise negative binomial distribution corresponds to an occurrence of molecular label sequences associated with the sequences of the target in the sequencing data being noise molecular label sequences; and determining the occurrence of the noise molecular label sequences using the signal negative binomial distribution and the noise negative binomial distribution. In some embodiments, the negative binomial distribution fitting condition can comprise: the sequencing status of the target in the sequencing data is not the under sequencing status or the over sequencing status. Determining the number of noise molecular label sequences using the signal negative binomial distribution and the noise negative binomial distribution can comprise: for each of the molecular label sequences associated with the putative sequences of the target in the sequencing data: determining a signal probability of the molecular label sequence to be in the signal negative binomial distribution; determining a noise probability of the molecular label sequence to be in the noise negative binomial distribution; and determining the molecular label sequence to be a noise molecular label if the signal probability is smaller than the noise probability.

In some embodiments, determining the occurrence of noise molecular label sequences associated with the sequences of the target in the sequencing data comprises: adding pseudopoints to the occurrence of molecular label sequences associated with the sequences of the target in the sequencing data prior to determining the occurrence of noise molecular label sequences associated with the sequences of the target in the sequencing data, if the sequencing status of the target in the sequencing data is not the under sequencing status or the over sequencing status and the occurrence of molecular label sequences associated with the sequences of the target in the sequencing data is less than a pseudopoints threshold. The pseudopoints threshold can be ten. Determining the occurrence of noise molecular label sequences associated with the sequences of the target in the sequencing data can comprise: removing non-unique molecular label sequences when determining the occurrence of noise molecular label sequences associated with the sequences of the target in the sequencing data, if the sequencing status of the target in the sequencing data is not the under sequencing status or the over sequencing status and the occurrence of molecular label sequences associated with the sequences of the target in the sequencing data is not less than a pseudopoints threshold.

In some embodiments, receiving the sequencing data of the plurality of targets comprises: barcoding (e.g., stochastically barcoding_ a plurality of targets using a plurality of barcodes (e.g., stochastic barcodes) to create a plurality of barcoded targets (e.g., stochastically barcoded targets), wherein each of the plurality of barcodes comprises a cell label and a molecular label, wherein molecular labels of at least two barcodes of the plurality of barcodes comprise different molecular label sequences, and wherein at least two barcodes of the plurality of barcodes comprise cell labels with an identical cell label sequence; and obtaining sequencing data of the barcoded targets. Barcoding (e.g., stochastically barcoding) the plurality of targets in the plurality of cells using the plurality of barcodes to create the plurality of barcoded targets for the cells of the plurality of cells can comprise: barcoding (e.g., stochastically barcoding) the plurality of targets using the plurality of barcodes of a particle to create the plurality of barcoded targets, wherein the particle comprises a subset of the plurality of barcodes, wherein each of the subset of barcodes comprise an identical cell label sequence and with at least 100 different molecular label sequences.

In some embodiments, the particle is a bead. The bead can be selected from the group consisting of streptavidin beads, agarose beads, magnetic beads, conjugated beads, protein A conjugated beads, protein G conjugated beads, protein A/G conjugated beads, protein L conjugated beads, oligo(dT) conjugated beads, silica beads, silica-like beads, anti-biotin microbead, anti-fluorochrome microbead, and any combination thereof. The particle can comprise a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof. The barcodes (e.g., stochastic barcodes) of the particle comprise molecular labels with at least 1000, 10000, or any combination there, different molecular label sequences.

In some embodiments, the molecular labels of the barcodes (e.g., stochastic barcodes) comprise random sequences. The particle can comprise at least 10000 barcodes. Barcoding (e.g., stochastically barcoding) the plurality of targets using the plurality of barcodes (e.g., stochastic barcodes) to create the plurality of barcoded targets (e.g., stochastically barcoded targets) can comprise: (i) contacting copies of the targets with target binding regions of the barcodes; and (ii) reverse transcribing the plurality targets using the plurality of barcodes to create a plurality of reverse transcribed targets. In some embodiments, the method comprises: prior to obtaining the sequencing data of the plurality of barcoded targets, amplifying the barcoded targets to generate a plurality of barcoded targets (e.g., amplified stochastically barcoded targets_. Amplifying the barcoded targets to generate the plurality of stochastically barcoded targets can comprise: amplifying the barcoded targets by polymerase chain reaction (PCR).

In some embodiments, computer systems for determining the occurrence of targets are disclosed. The computer system can comprise: a hardware processor; and non-transitory memory having instructions stored thereon, which when executed by the hardware processor cause the processor to perform the method of any of the above claims. Computer readable media are disclosed. In some embodiments, the computer readable medium comprises executable codes for performing the method of any of the above claims.

BRIEF DESCRIPTION OF THE DRAWINGS

FIG. 1 illustrates a non-limiting exemplary stochastic barcode.
FIG. 2 shows a non-limiting exemplary workflow of stochastic barcoding and digital counting.
FIG. 3 is a schematic illustration showing a non-limiting exemplary process for generating an indexed library of the stochastically barcoded targets from a plurality of targets.
FIG. 4 is a schematic illustration showing non-limiting exemplary distributions of molecular label errors, sample label errors, and true molecular label signals.
FIG. 5 is a flowchart showing a non-limiting exemplary embodiment of correcting PCR and sequencing errors using molecular labels based on directional adjacency.
FIG. 6 is a flowchart showing a non-limiting exemplary embodiment of correcting PCR and sequencing errors based on recursive substitution error correction and distribution-based error correction.
FIG. 7 is a schematic illustration showing a non-limiting exemplary embodiment of immune receptor-barcode correction based on recursive substitution error correction.
FIG. 8 is a flowchart showing a non-limiting exemplary embodiment of correcting errors in nucleotide sequences and molecular labels using recursive substitution error correction and correcting errors in sequencing data attributable to PCR chimeras).
FIG. 9 is a schematic illustration of one possible origin of immune receptor chimeras.
FIG. 10 shows a non-limiting exemplary instrument suitable to use in the methods of the disclosure.
FIG. 11 illustrates a non-limiting exemplary architecture of a computer system that can be used in connection with embodiments of the present disclosure.
FIG. 12 illustrates a non-limiting exemplary architecture showing a network with a plurality of computer systems suitable for use in the methods of the disclosure.
FIG. 13 illustrates a non-limiting exemplary architecture of a multiprocessor computer system using a shared virtual address memory space in accordance with the methods of the disclosure.
FIG. 14 is an exemplary plot of theoretical calculation of unique molecular labels used as input molecules increases.
FIG. 15 is an exemplary plot showing molecular label coverage of each molecular label across a microwell plate for a high expressor gene - ATCB, where distinct distributions were observed between error molecular labels and real molecular labels.
FIG. 16 is an exemplary plot showing fitting two negative binomial distributions to molecular label coverage of each molecular label across a micro well plate for a high expressor gene - ATCB. The fitting of two negative binomial distributions demonstrates that molecular label errors with lower molecular label depth and true molecular label with higher molecular label depth can be statistically distinguished. The x axis is the molecular depth.

FIG. 17 shows molecular label correction, where pairwise Hamming distance of 1 was overrepresented. After molecular label correction, molecular labels with Hamming distance of one apart were clustered and collapsed to the same parent molecular label.

FIG. 18 shows the curve of corrected number of molecular labels vs. corrected number of reads coverages.

FIG. 19 shows a schematic illustration of an example of recursive substitution error correction.

FIGS. 20A-20C show exemplary results of correcting PCR and sequencing errors based on two negative binomial distributions for CD69.

FIGS. 21A-21C show exemplary results of correcting PCR and sequencing errors based on two negative binomial distributions for CD3E.

FIGS. 22A-22J show non-limiting exemplary validation of dataset corrected using two negative binomial distributions.

FIGS. 23A-23D show exemplary t-stochastic neighbor embedding (t-SNE) visualizations of Precise™ targeted assay from a 96-well of mixed Jurkat and breast cancer (BrCa) single cells (86 genes examined).

FIGS. 24A-24B are non-limiting exemplary plots showing differential expression analysis between cell clusters for genes with >0 ML in both selected clusters calculated by DBScan and determined by gene marker level in each cluster.

FIGS. 25A-25D are non-limiting exemplary plots showing t-stochastic neighbor embedding (t-SNE) visualization of a BD Precise™ targeted assay from a 96-well plate of mixed Jurkat and breast cancer (T47D) single cells with 86 genes examined.

FIG. 26A-26B are non-limiting exemplary heat maps displaying differential gene expression by molecular label counts between different cell clusters identified in FIGS. 25A-25D before any error correction steps (Raw ML shown in FIG. 26A) and after RSEC and DBEC correction (Adjusted ML shown in FIG. 26B).

FIGS. 27A-27B show a table illustrating a non-limiting example of immune receptor-barcode error correction using recursive substitution error correction.

FIG. 28 is a histogram showing non-limiting exemplary results of immune receptor-barcode error correction.

DETAILED DESCRIPTION

In the following detailed description, reference is made to the accompanying drawings, which form a part hereof. In the drawings, similar symbols typically identify similar components, unless context dictates otherwise. The illustrative embodiments described in the detailed description, drawings, and claims are not meant to be limiting. Other embodiments may be utilized, and other changes may be made, without departing from the spirit or scope of the subject matter presented herein. It will be readily understood that the aspects of the present disclosure, as generally described herein, and illustrated in the Figures, can be arranged, substituted, combined, separated, and designed in a wide variety of different configurations, all of which are explicitly contemplated herein and made part of the disclosure herein.

All patents, published patent applications, other publications, and sequences from GenBank, and other databases referred to herein are incorporated by reference in their entirety with respect to the related technology.

Quantifying small numbers of nucleic acids, for example messenger ribonucleotide acid (mRNA) molecules, is clinically important for determining, for example, the genes that are expressed in a cell at different stages of development or under different environmental conditions. However, it can also be very challenging to determine the absolute number of nucleic acid molecules (e.g., mRNA molecules), especially when the number of molecules is very small. One method to determine the absolute number of molecules in a sample is digital polymerase chain reaction (PCR). Ideally, PCR produces an identical copy of a molecule at each cycle. However, PCR can have disadvantages such that each molecule replicates with a stochastic probability, and this probability varies by PCR cycle and gene sequence, resulting in amplification bias and inaccurate gene expression measurements. Stochastic barcodes with unique molecular labels (also referred to as molecular indexes (MIs) or universal molecular indexes (UMIs)) can be used to count the number of molecules and correct for amplification bias. Stochastic barcoding such as the Precise™ assay (Cellular Research, Inc. (Palo Alto, CA)) can correct for bias induced by PCR and library preparation steps by using molecular labels (MLs) to label mRNAs during reverse transcription (RT).

The Precise™ assay can utilize a non-depleting pool of stochastic barcodes with large number, for example 6561 to 65536, unique molecular labels on poly(T) oligonucleotides to hybridize to all poly(A)-mRNAs in a sample during the RT step. In addition to molecular labels, sample label (also referred to as a sample index (SI)) of stochastic barcodes can be used to identify each well of the Precise™ plate. A stochastic barcode can comprise a universal PCR priming site. During RT, target gene molecules react randomly with stochastic barcodes. Each target molecule can hybridize to a stochastic barcode resulting to generate stochastically barcoded complementary ribonucleotide acid (cDNA) molecules). After labeling, stochastically barcoded cDNA molecules from microwells of a microwell plate can be pooled into a single tube for PCR amplification and sequencing. Raw sequencing data can be analyzed to produce the number of reads, the number of stochastic barcodes with unique molecular labels, and the numbers of mRNA molecules based on a Poisson correction or a correction method based on two negative binomial distributions.

In addition to bias correction, molecular labels can provide a better understanding of the statistical quality of the results by revealing the starting number of cDNA molecules present in the observed sequencing reads. For example, a large number of reads may indicate a statistically accurate answer, but if the reads are derived from just a small number of starting mRNA molecules, then the measurement accuracy may be compromised.

Although amplification bias induced by PCR and library preparation steps can be remedied, for example, by molecular labels, the quantification of the absolute number of molecules can still be challenging due to several other factors. First, estimation of the number of mRNA molecules may be limited by the total diversity of the molecular labels. During stochastic barcoding, mRNA molecules can react randomly with available stochastic barcodes. Thus, each mRNA molecule can hybridize to a stochastic barcode; however his molecular label might not necessarily be unique for any given gene. When the number of mRNA molecules is small relative to the number of stochastic barcodes, each mRNA molecule will likely hybridize to a stochastic barcode with a unique molecular label, and counting the number of molecules can be equivalent to counting the number of molecular labels.

As the number of mRNA molecules increases, multiple mRNA molecules become increasing likely to hybridize to stochastic barcodes with the same molecular labels. Hence using counts of unique molecular labels may underestimate the number of molecules. In some cases, the number of mRNA molecules can be estimated based on a Poisson correction or a correction based on two negative binomial distributions of the number of unique molecular labels observed in total. However, at the extreme where the entire collection of 6561 stochastic barcodes is observed, a Poisson correction or a correction based on two negative binomial distributions may no longer be possible. For example, regardless of 65000 or 100000 starting mRNA molecules, a maximum of 6561 saturated stochastic barcodes is expected in either case.

Second, PCR errors (that is, errors occurred during PCR amplification), may introduce artificial stochastic barcodes and arbitrarily inflate molecular label counts. Third, PCR amplification bias and inefficient PCR may generate low copies of barcoded molecules that are indistinguishable from errors. Fourth, sequencing errors, the inaccurate calling of stochastic barcode sequences, may introduce artificial stochastic barcodes and inflate molecular label counts. Additionally, sequencing depth may be important, especially when sequencing is too shallow to detect all of the stochastically barcoded mRNAs present in a sample library.

Substitution errors, primer crossover errors, and PCR chimera errors can occur when performing immune receptor sequencing and profiling. For example, such errors can occur when determining the numbers of occurrences or copies of mRNA molecules encoding immune receptors, such as T-cell receptors. Immune receptors are closely related genes that are highly diversified. Hence, when compared to other genes, the possibility of such errors can be higher when performing immune receptor sequencing and profiling. The errors often lead to over-quantification of immune repertoire diversity. The methods for mitigating these errors are referred to herein as immune receptor-barcode error correction. In some embodiments, immune receptor-barcode error correction utilizes recursive substitution error correction to correct substitution errors in molecular labels and nucleotide sequences (e.g., substitution errors in the complementarity-determining region 3 (CDR3)). For a given sample label or cell label, many different CDR3s can be associated with the same molecular label sequence, leading to an overestimation of immune receptor diversity. The methods can correct for PCR chimeras that cross before molecular labeling and sample labeling followed by identifying and removing error molecular labels by distribution based error correction.

Disclosed herein include methods for determining occurrences of targets. In some embodiments, the method comprises: (a) barcoding (e.g., stochastically barcoding) a plurality of targets using a plurality of barcodes (e.g., stochastic barcodes) to create a plurality of barcoded targets (e.g., stochastically barcoded targets), wherein each of the plurality of barcodes comprises a cell label and a molecular label, wherein molecular labels of at least two barcodes of the plurality of barcodes comprise different molecular label sequences, and wherein at least two barcodes of the plurality of barcodes comprise cell labels with an identical cell label sequence; (b) obtaining sequencing data of the barcoded targets; and (c) for at least one target of the plurality of targets: (i) identifying putative sequences of the target in the sequencing data; (ii) counting occurrences of molecular label sequences associated with the putative sequences of the target in the sequencing data identified in (i); (iii) identifying clusters of the putative sequences of the target; (iv) collapsing the sequencing data obtained using the clusters of putative sequences of the target identified in (iii); (v) identifying clusters of the molecular label sequences associated with the putative sequences of the target; (vi) collapsing the sequencing data using the clusters of molecular label sequences identified in (v); (vii) identifying clusters of combination sequences, wherein each combination sequence comprises a sequence of the sequences of the target and an associated molecular label sequence of the molecular label sequences; (viii) collapsing the sequencing data using the clusters of combination sequences identified in (vii); (ix) identifying one or more putative sequences of the target that correspond to one or more chimeric sequences of the target, wherein occurrences of the one or more putative sequences of the target that correspond to the one or more chimeric sequences of the target are smaller than occurrences of remaining one or more putative sequences of the target that do not correspond to the one or more chimeric sequences of the target; (x) removing the one or more putative sequences of the target corresponding to the one or more chimeric sequences of the target identified in (ix) from the sequencing data; and (xi) estimating the occurrence of the target, wherein the occurrence of the target estimated correlates with the number of molecular label sequences counted in (ii) after collapsing the sequencing data in (iv), (vi), and (viii) and removing the one or more putative sequences of the target that correspond to the one or more chimeric sequences of the target in (x).

Disclosed herein are methods for determining occurrences of targets. In some embodiments, the method comprises: (a) receiving sequencing data of a plurality of targets, wherein the sequencing data comprises putative sequences of a target of the plurality of targets and occurrences of molecular label sequences associated with the sequences of the target in the sequencing data; (b) collapsing putative sequences of the target; (c) collapsing molecular label sequences associated with the putative sequences of the target; and (d) estimating the occurrence of the target, wherein the occurrence of the target estimated correlates with the occurrence of molecular label sequences associated with the putative sequences of the target in the sequencing data after collapsing the occurrence of the putative sequences of the target in (b) and the occurrence of noise molecular label sequences determined in (c).

Computer systems for determining occurrences of targets are disclosed. A non-transitory computer readable media containing executable codes, when executed, cause one or more computing devices to determine occurrences of targets is disclosed.

Definitions

Unless defined otherwise, technical and scientific terms used herein have the same meaning as commonly understood by one of ordinary skill in the art to which the present disclosure belongs. *See, e.g.,* Singleton et al., Dictionary of Microbiology and Molecular Biology 2nd ed., J. Wiley & Sons (New York., NY 1994); Sambrook et al., Molecular Cloning, A Laboratory Manual, Cold Spring Harbor Press (Cold Spring Harbor, NY 1989). For purposes of the present disclosure, the following terms are defined below.

As used herein, the term "adaptor" can mean a sequence to facilitate amplification or sequencing of associated nucleic acids. The associated nucleic acids can comprise target nucleic acids. The associated nucleic acids can comprise one or more of spatial labels, target labels, sample labels, indexing label, or barcode sequences (e.g., molecular labels). The adapters can be linear. The adaptors can be pre-adenylated adapters. The adaptors can be double- or single-stranded. One or more adaptor can be located on the 5' or 3' end of a nucleic acid. When the adaptors comprise known sequences on the 5' and 3' ends, the known sequences can be the same or different sequences. An adaptor located on the 5' and/or 3' ends of a polynucleotide can be capable of hybridizing to one or more oligonucleotides immobilized on a surface. An adapter can, in some embodiments, comprise a universal sequence. A universal sequence can be a region of nucleotide sequence that is common to two or more nucleic acid molecules. The two or more nucleic acid molecules can also have regions of different sequence. Thus, for example, the 5' adapters can comprise identical and/or universal nucleic acid sequences and the 3' adapters can comprise identical and/or universal sequences. A universal sequence that may be present in different members of a plurality of nucleic acid molecules can allow the replication or amplification of multiple different sequences using a single universal primer that is complementary to the universal sequence. Similarly, at least one, two (e.g., a pair) or more universal sequences that may be present in different members of a collection of nucleic acid molecules can allow the replication or amplification of multiple different sequences using at least one, two (e.g., a pair) or more single universal primers that are complementary to the universal sequences. Thus, a universal primer includes a sequence that can hybridize to such a universal sequence. The target nucleic acid sequence-bearing molecules may be modified to attach universal adapters (e.g., non-target nucleic acid sequences) to one or both ends of the different target nucleic acid sequences. The one or more universal primers attached to the target nucleic acid can provide sites for hybridization of universal primers. The one or more universal primers attached to the target nucleic acid can be the same or different from each other.

As used herein the term "associated" or "associated with" can mean that two or more species are identifiable as being co-located at a point in time. An association can mean that two or more species are or were within a similar container. An association can be an informatics association. For example, digital information regarding two or more species can be stored and can be used to determine that one or more of the species were co-located at a point in time. An association can also be a physical association. In some embodiments, two or more associated species are "tethered", "attached", or "immobilized" to one another or to a common solid or semisolid surface. An association may refer to covalent or non-covalent means for attaching labels to solid or semi-solid supports such as beads. An association may be a covalent bond between a target and a label. An association can comprise hybridization between two molecules (such as a target molecule and a label).

As used herein, the term "complementary" can refer to the capacity for precise pairing between two nucleotides. For example, if a nucleotide at a given position of a nucleic acid is capable of hydrogen bonding with a nucleotide of another nucleic acid, then the two nucleic acids are considered to be complementary to one another at that position. Complementarity between two single-stranded nucleic acid molecules may be "partial," in which only some of the nucleotides bind, or it may be complete when total complementarity exists between the single-stranded molecules. A first nucleotide sequence can be said to be the "complement" of a second sequence if the first nucleotide sequence is complementary to the second nucleotide sequence. A first nucleotide sequence can be said to be the "reverse complement" of a second sequence, if the first nucleotide sequence is complementary to a sequence that is the reverse (i.e., the order of the nucleotides is reversed) of the second sequence. As used herein, the terms "complement", "complementary", and "reverse complement" can be used interchangeably. It is understood from the disclosure that if a molecule can hybridize to another molecule it may be the complement of the molecule that is hybridizing.

As used herein, the term "digital counting" can refer to a method for estimating a number of target molecules in a sample. Digital counting can include the step of determining a number of unique labels that have been associated with targets in a sample. This methodology, which can be stochastic in nature, transforms the problem of counting molecules from one of locating and identifying identical molecules to a series of yes/no digital questions regarding detection of a set of predefined labels.

As used herein, the term "label" or "labels" can refer to nucleic acid codes associated with a target within a sample. A label can be, for example, a nucleic acid label. A label can be an entirely or partially amplifiable label. A label can be entirely or partially sequencable label. A label can be a portion of a native nucleic acid that is identifiable as distinct. A label can be a known sequence. A label can comprise a junction of nucleic acid sequences, for example a junction of a native and non-native sequence. As used herein, the term "label" can be used interchangeably with the terms, "index", "tag," or "label-tag." Labels can convey information. For example, in various embodiments, labels can be used to determine an identity of a sample, a source of a sample, an identity of a cell, and/or a target.

As used herein, the term "non-depleting reservoirs" can refer to a pool of barcodes (e.g., stochastic barcodes) made up of many different labels. A non-depleting reservoir can comprise large numbers of different barcodes such that when the non-depleting reservoir is associated with a pool of targets each target is likely to be associated with a unique barcode. The uniqueness of each labeled target molecule can be determined by the statistics of random choice, and depends on the number of copies of identical target molecules in the collection compared to the diversity of labels. The size of the resulting set of labeled target molecules can be determined by the stochastic nature of the barcoding process, and analysis of the number of barcodes detected then allows calculation of the number of target molecules present in the original collection or sample. When the ratio of the number of copies of a target molecule present to the number of unique barcodes is low, the labeled target molecules are highly unique (i.e., there is a very low probability that more than one target molecule will have been labeled with a given label).

As used herein, the term "nucleic acid" refers to a polynucleotide sequence, or fragment thereof. A nucleic acid can comprise nucleotides. A nucleic acid can be exogenous or endogenous to a cell. A nucleic acid can exist in a cell-free environment. A nucleic acid can be a gene or fragment thereof. A nucleic acid can be DNA. A nucleic acid can be RNA. A nucleic acid can comprise one or more analogs (e.g. altered backbone, sugar, or nucleobase). Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, fluorophores (e.g. rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. "Nucleic acid", "polynucleotide, "target polynucleotide", and "target nucleic acid" can be used interchangeably.

A nucleic acid can comprise one or more modifications (e.g., a base modification, a backbone modification), to provide the nucleic acid with a new or enhanced feature (e.g., improved stability). A nucleic acid can comprise a nucleic acid affinity tag. A nucleoside can be a base-sugar combination. The base portion of the nucleoside can be a heterocyclic base. The two most common classes of such heterocyclic bases are the purities and the pyrimidines. Nucleotides can be nucleosides that further include a phosphate group covalently linked to the sugar portion of the nucleoside. For those nucleosides that include a pentofuranosyl sugar, the phosphate group can be linked to the 2', the 3', or the 5' hydroxyl moiety of the sugar. In forming nucleic acids, the phosphate groups can covalently link adjacent nucleosides to one another to form a linear polymeric compound. In turn, the respective ends of this linear polymeric compound can be further joined to form a circular compound; however, linear compounds are generally suitable. In addition, linear compounds may have internal nucleotide base complementarity and may therefore fold in a manner as to produce a fully or partially double-stranded compound. Within nucleic acids, the phosphate groups can commonly be referred to as forming the internucleoside backbone of the nucleic acid. The linkage or backbone can be a 3' to 5' phosphodiester linkage.

A nucleic acid can comprise a modified backbone and/or modified internucleoside linkages. A nucleic acid can comprise polynucleotide backbones that are formed by short chain alkyl or cycloalkyl internucleoside linkages, mixed heteroatom and alkyl or cycloalkyl internucleoside linkages, or one or more short chain heteroatomic or heterocyclic internucleoside linkages. A nucleic acid can comprise a nucleic acid mimetic. A nucleic acid can comprise a morpholino backbone structure. A nucleic acid can comprise linked morpholino units (i.e. morpholino nucleic acid) having heterocyclic bases attached to the morpholino ring.

A nucleic acid may also include nucleobase (often referred to simply as "base") modifications or substitutions. As used herein, "unmodified" or "natural" nucleobases can include the purine bases, (e.g. adenine (A) and guanine (G)), and the pyrimidine bases, (e.g. thymine (T), cytosine (C) and uracil (U)). Modified nucleobases can include other synthetic and natural nucleobases.

As used herein, the term "sample" can refer to a composition comprising targets. Suitable samples for analysis by the disclosed methods, devices, and systems include cells, tissues, organs, or organisms.

As used herein, the term "sampling device" or "device" can refer to a device which may take a section of a sample and/or place the section on a substrate. A sample device can refer to, for example, a fluorescence activated cell sorting (FACS) machine, a cell sorter machine, a biopsy needle, a biopsy device, a tissue sectioning device, a microfluidic device, a blade grid, and/or a microtome.

As used herein, the term "solid support" can refer to discrete solid or semi-solid surfaces to which a plurality of barcodes (e.g., stochastic barcodes) may be attached. A solid support may encompass any type of solid, porous, or hollow sphere, ball, bearing, cylinder, or other similar configuration composed of plastic, ceramic, metal, or polymeric material (e.g., hydrogel) onto which a nucleic acid may be immobilized (e.g., covalently or non-covalently). A solid support may comprise a discrete particle that may be spherical (e.g., microspheres) or have a non-spherical or irregular shape, such as cubic, cuboid, pyramidal, cylindrical, conical, oblong, or disc-shaped, and the like. A bead can be non-spherical in shape. A plurality of solid supports spaced in an array may not comprise a substrate. A solid support may be used interchangeably with the term "bead."

A solid support can refer to a "substrate." A substrate can be a type of solid support. A substrate can refer to a continuous solid or semi-solid surface on which the methods of the disclosure may be performed. A substrate can refer to an array, a cartridge, a chip, a device, and a slide, for example.

As used here, the term, "spatial label" can refer to a label which can be associated with a position in space.

As used herein, the term "stochastic barcode" can refer to a polynucleotide sequence comprising labels of the present disclosure. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "gene-specific stochastic barcode" can refer to a polynucleotide sequence comprising labels and a target-binding region that is gene-specific. A stochastic barcode can be a polynucleotide sequence that can be used for stochastic barcoding. Stochastic barcodes can be used to quantify targets within a sample. Stochastic barcodes can be used to control for errors which may occur after a label is associated with a target. For example, a stochastic barcode can be used to assess amplification or sequencing errors. A stochastic barcode associated with a target can be called a stochastic barcode-target or stochastic barcode-tag-target.

As used herein, the term "stochastic barcoding" can refer to the random labeling (e.g., barcoding) of nucleic acids. Stochastic barcoding can utilize a recursive Poisson strategy to associate and quantify labels associated with targets. As used herein, the term "stochastic barcoding" can be used interchangeably with "stochastic labeling."

As used here, the term "target" can refer to a composition which can be associated with a barcode (e.g., stochastic barcode). Exemplary suitable targets for analysis by the disclosed methods, devices, and systems include oligonucleotides, DNA, RNA, mRNA, microRNA, tRNA, and the like. Targets can be single or double stranded. In some embodiments, targets can be proteins, peptides, or polypeptides. In some embodiments, targets are lipids. As used herein, "target" can be used interchangeably with "species."

As used herein, the term "reverse transcriptases" can refer to a group of enzymes having reverse transcriptase activity (i.e., that catalyze synthesis of DNA from an RNA template). In general, such enzymes include, but are not limited to, retroviral reverse transcriptase, retrotransposon reverse transcriptase, retroplasmid reverse transcriptases, retron reverse transcriptases, bacterial reverse transcriptases, group II intron-derived reverse transcriptase, and mutants, variants or derivatives thereof. Non-retroviral reverse transcriptases include non-LTR retrotransposon reverse transcriptases, retroplasmid reverse transcriptases, retron reverse transcriptases, and group II intron reverse transcriptases. Examples of group II intron reverse transcriptases include the *Lactococcus lactis* LI.LtrB intron reverse transcriptase, the *Thermosynechococcus elongatus* TeI4c intron reverse transcriptase, or the *Geobacillus stearothermophilus* GsI-IIC intron reverse transcriptase. Other classes of reverse transcriptases can include many classes of non-retroviral reverse transcriptases (i.e., retrons, group II introns, and diversity-generating retroelements among others).

The terms "universal adaptor primer," "universal primer adaptor" or "universal adaptor sequence" are used interchangeably to refer to a nucleotide sequence that can be used to hybridize to barcodes (e.g., stochastic barcodes) to generate gene-specific barcodes. A universal adaptor sequence can, for example, be a known sequence that is universal across all barcodes used in methods of the disclosure. For example, when multiple targets are being labeled using the methods disclosed herein, each of the target-specific sequences may be linked to the same universal adaptor sequence. In some embodiments, more than one universal adaptor sequences may be used in the methods disclosed herein. For example, when multiple targets are being labeled using the methods disclosed herein, at least two of the target-specific sequences are linked to different universal adaptor sequences. A universal adaptor primer and its complement may be included in two oligonucleotides, one of which comprises a target-specific sequence and the other comprises a barcode. For example, a universal adaptor sequence may be part of an oligonucleotide comprising a target-specific sequence to generate a nucleotide sequence that is complementary to a target nucleic acid. A second oligonucleotide comprising a barcode and a complementary sequence of the universal adaptor sequence may hybridize with the nucleotide sequence and generate a target-specific barcode (e.g., a target-specific stochastic barcode). In some embodiments, a universal adaptor primer has a sequence that is different from a universal PCR primer used in the methods of this disclosure.

Disclosed herein are methods and system for detecting and/or correcting errors occurred during PCR and/or sequencing. The types of errors can vary, for example, include but not limited to, substitution errors (one or more bases) and non-substitution errors. Amongst the substitution errors, one-base substitution errors can occur much more frequently than those further than one-base apart. The methods and systems can be used, for example, to provide accurate counting of molecular targets by stochastic barcoding.

Barcodes

Barcoding, such as stochastic barcoding, has been described in, for example, US20150299784, WO2015031691, and Fu et al, Proc Natl Acad Sci U.S.A. 2011 May 31;108(22):9026-31, the content of these publications is incorporated hereby in its entirety. In some embodiments, the barcode disclosed herein can be a stochastic barcode which can be a polynucleotide sequence that may be used to stochastically label (e.g., barcode, tag) a target. Barcodes can be referred to stochastic barcodes if the ratio of the number of different barcode sequences of the stochastic barcodes and the number of occurrence of any of the targets to be labeled can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. Barcodes can be referred to as stochastic barcodes if the ratio of the number of different barcode sequences of the stochastic barcodes and the number of occurrence of any of the targets to be labeled is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1. Barcode sequences of stochastic barcodes can be referred to as molecular labels.

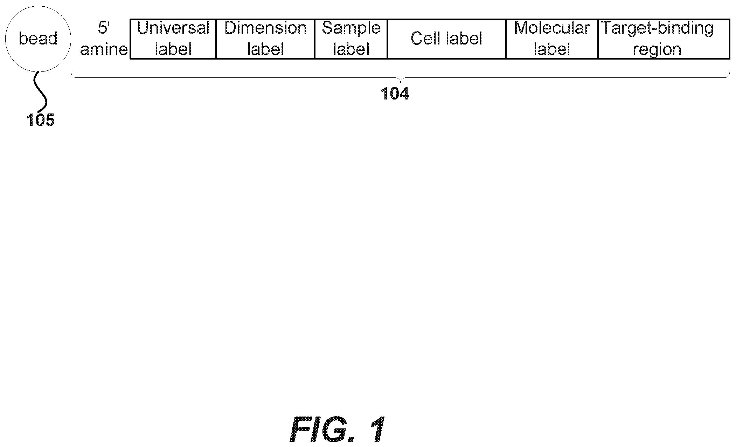

A barcode, for example a stochastic barcode, can comprise one or more labels. Exemplary labels can include a universal label, a cell label, a barcode sequence (e.g., a molecular label), a sample label, a plate label, a spatial label, and/or a pre-spatial label. FIG. 1 illustrates an exemplary barcode 104 with a spatial label. The barcode 104 can comprise a 5'amine that may link the barcode to a solid support 105. The barcode can comprise a universal label, a dimension label, a spatial label, a cell label, and/or a molecular label. The order of different labels (including but not limited to the universal label, the dimension label, the spatial label, the cell label, and the molecule label) in the barcode can vary. For example, as shown in FIG. 1, the universal label may be the 5'-most label, and the molecular label may be the 3'-most label. The spatial label, dimension label, and the cell label may be in any order. In some embodiments, the universal label, the spatial label, the dimension label, the cell label, and the molecular label are in any order. The barcode can comprise a target-binding region. The target-binding region can interact with a target (e.g., target nucleic acid, RNA, mRNA, DNA) in a sample. For example, a target-binding region can comprise an oligo(dT) sequence which can interact with poly(A) tails of mRNAs. In some instances, the labels of the barcode (e.g., universal label, dimension label, spatial label, cell label, and barcode sequence) may be separated by 1, 2, 3, 4, 5, 6, 7, 8, 9, 10, 11, 12, 13, 14, 15, 16, 17, 18, 19, or 20 or more nucleotides.

A label, for example the cell label, can comprise a unique set of nucleic acid sub-sequences of defined length, e.g., seven nucleotides each (equivalent to the number of bits used in some Hamming error correction codes), which can be designed to provide error correction capability. The set of error correction sub-sequences comprise seven nucleotide sequences can be designed such that any pairwise combination of sequences in the set exhibits a defined "genetic distance" (or number of mismatched bases), for example, a set of error correction sub-sequences can be designed to exhibit a genetic distance of three nucleotides. In this case, review of the error correction sequences in the set of sequence data for labeled target nucleic acid molecules (described more fully below) can allow one to detect or correct amplification or sequencing errors. In some embodiments, the length of the nucleic acid sub-sequences used for creating error correction codes can vary, for example, they can be, or be about 1, 2, 3, 4, 5, 6, 7, 8, 9, 10, 15, 20, 30, 31, 40, 50, or a number or a range between any two of these values, nucleotides in length. In some embodiments, nucleic acid sub-sequences of other lengths can be used for creating error correction codes.

The barcode (e.g., the stochastic barcode) can comprise a target-binding region. The target-binding region can interact with a target in a sample. The target can be, or comprise, ribonucleic acids (RNAs), messenger RNAs (mRNAs), microRNAs, small interfering RNAs (siRNAs), RNA degradation products, RNAs each comprising a poly(A) tail, or any combination thereof. In some embodiments, the plurality of targets can include deoxyribonucleic acids (DNAs).

In some embodiments, a target-binding region can comprise an oligo(dT) sequence which can interact with poly(A) tails of mRNAs. One or more of the labels of the barcode (e.g., the universal label, the dimension label, the spatial label, the cell label, and the barcode sequences (e.g., molecular label)) can be separated by a spacer from another one or two of the remaining labels of the barcode. The spacer can be, for example, 1, 2, 3, 4, 5, 6, 7, 8, 9, 10, 11, 12, 13, 14, 15, 16, 17, 18, 19, or 20, or more nucleotides. In some embodiments, none of the labels of the barcode is separated by spacer.

*Universal Labels*

A barcode can comprise one or more universal labels. In some embodiments, the one or more universal labels can be the same for all barcodes in the set of barcodes attached to a given solid support. In some embodiments, the one or more universal labels can be the same for all barcodes attached to a plurality of beads. In some embodiments, a universal label can comprise a nucleic acid sequence that is capable of hybridizing to a sequencing primer. Sequencing primers can be used for sequencing barcodes comprising a universal label. Sequencing primers (e.g., universal sequencing primers) can comprise sequencing primers associated with high-throughput sequencing platforms. In some embodiments, a universal label can comprise a nucleic acid sequence that is capable of hybridizing to a PCR primer. In some embodiments, the universal label can comprise a nucleic acid sequence that is capable of hybridizing to a sequencing primer and a PCR primer. The nucleic acid sequence of the universal label that is capable of hybridizing to a sequencing or PCR primer can be referred to as a primer binding site. A universal label can comprise a sequence that can be used to initiate transcription of the barcode. A universal label can comprise a sequence that can be used for extension of the barcode or a region within the barcode. A universal label can be, or be about, 1, 2, 3, 4, 5, 10, 15, 20, 25, 30, 35, 40, 45, 50, or a number or a range between any two of these values, nucleotides in length. For example, a universal label can comprise at least about 10 nucleotides. A universal label can be at least, or be at most, 1, 2, 3, 4, 5, 10, 15, 20, 25, 30, 35, 40, 45, 50, 100, 200, or 300 nucleotides in length. In some embodiments, a cleavable linker or modified nucleotide can be part of the universal label sequence to enable the barcode to be cleaved off from the support.

*Dimension Labels*

A barcode can comprise one or more dimension labels. In some embodiments, a dimension label can comprise a nucleic acid sequence that provides information about a dimension in which the labeling (e.g., stochastic labeling) occurred. For example, a dimension label can provide information about the time at which a target was barcoded. A dimension label can be associated with a time of barcoding (e.g., stochastic barcoding) in a sample. A dimension label can be activated at the time of labeling. Different dimension labels can be activated at different times. The dimension label provides information about the order in which targets, groups of targets, and/or samples were barcoded. For example, a population of cells can be barcoded at the G0 phase of the cell cycle. The cells can be pulsed again with barcodes (e.g., stochastic barcodes) at the G1 phase of the cell cycle. The cells can be pulsed again with barcodes at the S phase of the cell cycle, and so on. Barcodes at each pulse (e.g., each phase of the cell cycle), can comprise different dimension labels. In this way, the dimension label provides information about which targets were labelled at which phase of the cell cycle. Dimension labels can interrogate many different biological times. Exemplary biological times can include, but are not limited to, the cell cycle, transcription (e.g., transcription initiation), and transcript degradation. In another example, a sample (e.g., a cell, a population of cells) can be labeled before and/or after treatment with a drug and/or therapy. The changes in the number of copies of distinct targets can be indicative of the sample's response to the drug and/or therapy.

A dimension label can be activatable. An activatable dimension label can be activated at a specific time point. The activatable label can be, for example, constitutively activated (e.g., not turned off). The activatable dimension label can be, for example, reversibly activated (e.g., the activatable dimension label can be turned on and turned off). The dimension label can be, for example, reversibly activatable at least 1, 2, 3, 4, 5, 6, 7, 8, 9, 10, or more times. The dimension label can be reversibly activatable, for example, at least 1, 2, 3, 4, 5, 6, 7, 8, 9,, 10 or more times. In some embodiments, the dimension label can be activated with fluorescence, light, a chemical event (e.g., cleavage, ligation of another molecule, addition of modifications (e.g., pegylated, sumoylated, acetylated, methylated, deacetylated, demethylated), a photochemical event (e.g., photocaging), and introduction of a non-natural nucleotide.

The dimension label can, in some embodiments, be identical for all barcodes (e.g., stochastic barcodes) attached to a given solid support (e.g., a bead), but different for different solid supports (e.g., beads). In some embodiments, at least 60%, 70%, 80%, 85%, 90%, 95%, 97%, 99% or 100%, of barcodes on the same solid support can comprise the same dimension label. In some embodiments, at least 60% of barcodes on the same solid support can comprise the same dimension label. In some embodiments, at least 95% of barcodes on the same solid support can comprise the same dimension label.

There can be as many as $10^6$ or more unique dimension label sequences represented in a plurality of solid supports (e.g., beads). A dimension label can be, or be about 1, 2, 3, 4, 5, 10, 15, 20, 25, 30, 35, 40, 45, 50, or a number or a range between any two of these values, nucleotides in length. A dimension label can be at least, or be at most, 1, 2, 3, 4, 5, 10, 15, 20, 25, 30, 35, 40, 45, 50, 100, 200, or 300, nucleotides in length. A dimension label can comprise between about 5 to about 200 nucleotides. A dimension label can comprise between about 10 to about 150 nucleotides. A dimension label can comprise between about 20 to about 125 nucleotides in length.

*Spatial Labels*

A barcode can comprise one or more spatial labels. In some embodiments, a spatial label can comprise a nucleic acid sequence that provides information about the spatial orientation of a target molecule which is associated with the barcode. A spatial label can be associated with a coordinate in a sample. The coordinate can be a fixed coordinate. For example, a coordinate can be fixed in reference to a substrate. A spatial label can be in reference to a two or three-dimensional grid. A coordinate can be fixed in reference to a landmark. The landmark can be identifiable in space. A landmark can be a structure which can be imaged. A landmark can be a biological structure, for example an anatomical landmark. A landmark can be a cellular landmark, for instance an organelle. A landmark can be a non- natural landmark such as a structure with an identifiable identifier such as a color code, bar code, magnetic property, fluorescents, radioactivity, or a unique size or shape. A spatial label can be associated with a physical partition (e.g., A well, a container, or a droplet). In some embodiments, multiple spatial labels are used together to encode one or more positions in space.

The spatial label can be identical for all barcodes attached to a given solid support (e.g., a bead), but different for different solid supports (e.g., beads). In some embodiments, the percentage of barcodes on the same solid support comprising the same spatial label can be, or be about, 60%, 70%, 80%, 85%, 90%, 95%, 97%, 99%, 100%, or a number or a range between any two of these values. In some embodiments, the percentage of barcodes on the same solid support comprising the same spatial label can be at least, or be at most, 60%, 70%, 80%, 85%, 90%, 95%, 97%, 99%, or 100%. In some embodiments, at least 60% of barcodes on the same solid support can comprise the same spatial label. In some embodiments, at least 95% of barcodes on the same solid support can comprise the same spatial label.

There can be as many as $10^6$ or more unique spatial label sequences represented in a plurality of solid supports (e.g., beads). A spatial label can be, or be about, 1, 2, 3, 4, 5, 10, 15, 20, 25, 30, 35, 40, 45, 50, or a number or a range between any two of these values, nucleotides in length. A spatial label can be at least or at most 1, 2, 3, 4, 5, 10, 15, 20, 25, 30, 35, 40, 45, 50, 100, 200, or 300 nucleotides in length. A spatial label can comprise between about 5 to about 200 nucleotides. A spatial label can comprise between about 10 to about 150 nucleotides. A spatial label can comprise between about 20 to about 125 nucleotides in length.

*Cell labels*

A barcode (e.g., a stochastic barcode) can comprise one or more cell labels. In some embodiments, a cell label can comprise a nucleic acid sequence that provides information for determining which target nucleic acid originated from which cell. In some embodiments, the cell label is identical for all barcodes attached to a given solid support (e.g., a bead), but different for different solid supports (e.g., beads). In some embodiments, the percentage of barcodes on the same solid support comprising the same cell label can be, or be about 60%, 70%, 80%, 85%, 90%, 95%, 97%, 99%, 100%, or a number or a range between any two of these values. In some embodiments, the percentage of barcodes on the same solid support comprising the same cell label can be, or be about 60%, 70%, 80%, 85%, 90%, 95%, 97%, 99%, or 100%. For example, at least 60% of barcodes on the same solid support can comprise the same cell label. As another example, at least 95% of barcodes on the same solid support can comprise the same cell label.

There can be as many as $10^6$ or more unique cell label sequences represented in a plurality of solid supports (e.g., beads). A cell label can be, or be about, 1, 2, 3, 4, 5, 10, 15, 20, 25, 30, 35, 40, 45, 50, or a number or a range between any two of these values, nucleotides in length. A cell label can be at least, or be at most, 1, 2, 3, 4, 5, 10, 15, 20, 25, 30, 35, 40, 45, 50, 100, 200, or 300 nucleotides in length. For example, a cell label can comprise between about 5 to about 200 nucleotides. As another example, a cell label can comprise between about 10 to about 150 nucleotides. As yet another example, a cell label can comprise between about 20 to about 125 nucleotides in length.

*Barcode Sequences*

A barcode can comprise one or more barcode sequences. In some embodiments, a barcode sequence can comprise a nucleic acid sequence that provides identifying information for the specific type of target nucleic acid species hybridized to the barcode. A barcode sequence can comprise a nucleic acid sequence that provides a counter (e.g., that provides a rough approximation) for the specific occurrence of the target nucleic acid species hybridized to the barcode (e.g., target-binding region).

In some embodiments, a diverse set of barcode sequences are attached to a given solid support (e.g., a bead). In some embodiments, there can be, or be about, $10^2$, $10^3$, $10^4$, $10^5$, $10^6$, $10^7$, $10^8$, $10^9$, or a number or a range between any two of these values, unique molecular label sequences. For example, a plurality of barcodes can comprise about 6561 barcodes sequences with distinct sequences. As another example, a plurality of barcodes can comprise about 65536 barcode sequences with distinct sequences. In some embodiments, there can be at least, or be at most, $10^2$, $10^3$, $10^4$, $10^5$, $10^6$, $10^7$, $10^8$, or $10^9$, unique barcode sequences. The unique molecular label sequences can be attached to a given solid support (e.g., a bead).

The length of a barcode can be different in different implementations. For example, a barcode can be, or be about, 1, 2, 3, 4, 5, 10, 15, 20, 25, 30, 35, 40, 45, 50, or a number or a range between any two of these values, nucleotides in length. As another example, a barcode can be at least, or be at most, 1, 2, 3, 4, 5, 10, 15, 20, 25, 30, 35, 40, 45, 50, 100, 200, or 300 nucleotides in length.

*Molecular Labels*

A barcode (e.g., a stochastic barcode) can comprise one or more molecular labels. Molecular labels can include barcode sequences. In some embodiments, a molecular label can comprise a nucleic acid sequence that provides identifying information for the specific type of target nucleic acid species hybridized to the barcode. A molecular label can comprise a nucleic acid sequence that provides a counter for the specific occurrence of the target nucleic acid species hybridized to the barcode (e.g., target-binding region).

In some embodiments, a diverse set of molecular labels are attached to a given solid support (e.g., a bead). In some embodiments, there can be, or be about, $10^2$, $10^3$, $10^4$, $10^5$, $10^6$, $10^7$, $10^8$, $10^9$, or a number or a range between any two of these values, of unique molecular label sequences. For example, a plurality of barcodes can comprise about 6561 molecular labels with distinct sequences. As another example, a plurality of barcodes can comprise about 65536 molecular labels with distinct sequences. In some embodiments, there can be at least, or be at most, $10^2$, $10^3$, $10^4$, $10^5$, $10^6$, $10^7$, $10^8$, or $10^9$, unique molecular label sequences. Barcodes with unique molecular label sequences can be attached to a given solid support (e.g., a bead).

For stochastic barcoding using a plurality of stochastic barcodes, the ratio of the number of different molecular label sequences and the number of occurrence of any of the targets can be, or be about, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, 100:1, or a number or a range between any two of these values. A target can be an mRNA species comprising mRNA molecules with identical or nearly identical sequences. In some embodiments, the ratio of the number of different molecular label sequences and the number of occurrence of any of the targets is at least, or is at most, 1:1, 2:1, 3:1, 4:1, 5:1, 6:1, 7:1, 8:1, 9:1, 10:1, 11:1, 12:1, 13:1, 14:1, 15:1, 16:1, 17:1, 18:1, 19:1, 20:1, 30:1, 40:1, 50:1, 60:1, 70:1, 80:1, 90:1, or 100:1.

A molecular label can be, or be about, 1, 2, 3, 4, 5, 10, 15, 20, 25, 30, 35, 40, 45, 50, or a number or a range between any two of these values, nucleotides in length. A molecular label can be at least, or be at most, 1, 2, 3, 4, 5, 10, 15, 20, 25, 30, 35, 40, 45, 50, 100, 200, or 300 nucleotides in length.

*Target-Binding Region*

A barcode can comprise one or more target binding regions, such as capture probes. In some embodiments, a target-binding region can hybridize with a target of interest. In some embodiments, the target binding regions can comprise a nucleic acid sequence that hybridizes specifically to a target (e.g., target nucleic acid, target molecule, e.g., a cellular nucleic acid to be analyzed), for example to a specific gene sequence. In some embodiments, a target binding region can comprise a nucleic acid sequence that can attach (e.g., hybridize) to a specific location of a specific target nucleic acid. In some embodiments, the target binding region can comprise a nucleic acid sequence that is capable of specific hybridization to a restriction enzyme site overhang (e.g., an EcoRI sticky-end overhang). The barcode can then ligate to any nucleic acid molecule comprising a sequence complementary to the restriction site overhang.

In some embodiments, a target binding region can comprise a non-specific target nucleic acid sequence. A non-specific target nucleic acid sequence can refer to a sequence that can bind to multiple target nucleic acids, independent of the specific sequence of the target nucleic acid. For example, target binding region can comprise a random multimer sequence, or an oligo(dT) sequence that hybridizes to the poly(A) tail on mRNA molecules. A random multimer sequence can be, for example, a random dimer, trimer, quatramer, pentamer, hexamer, septamer, octamer, nonamer, decamer, or higher multimer sequence of any length. In some embodiments, the target binding region is the same for all barcodes attached to a given bead. In some embodiments, the target binding regions for the plurality of barcodes attached to a given bead can comprise two or more different target binding sequences. A target binding region can be, or be about, 5, 10, 15, 20, 25, 30, 35, 40, 45, 50, or a number or a range between any two of these values, nucleotides in length. A target binding region can be at most about 5, 10, 15, 20, 25, 30, 35, 40, 45, 50 or more nucleotides in length.

In some embodiments, a target-binding region can comprise an oligo(dT) which can hybridize with mRNAs comprising polyadenylated ends. A target-binding region can be gene-specific. For example, a target-binding region can be configured to hybridize to a specific region of a target. A target-binding region can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these values, nucleotides in length. A target-binding region can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30, nucleotides in length. A target-binding region can be about 5-30 nucleotides in length. When a barcode comprises a gene-specific target-binding region, the barcode can be referred to herein as a gene-specific barcode.

*Orientation Property*

A stochastic barcode (e.g., a stochastic barcode) can comprise one or more orientation properties which can be used to orient (e.g., align) the barcodes. A barcode can comprise a moiety for isoelectric focusing. Different barcodes can comprise different isoelectric focusing points. When these barcodes are introduced to a sample, the sample can undergo isoelectric focusing in order to orient the barcodes into a known way. In this way, the orientation property can be used to develop a known map of barcodes in a sample. Exemplary orientation properties can include, electrophoretic mobility (e.g., based on size of the barcode), isoelectric point, spin, conductivity, and/or self-assembly. For example, barcodes with an orientation property of self-assembly, can self-assemble into a specific orientation (e.g., nucleic acid nanostructure) upon activation.

*Affinity Property*

A barcode (e.g., a stochastic barcode) can comprise one or more affinity properties. For example, a spatial label can comprise an affinity property. An affinity property can include a chemical and/or biological moiety that can facilitate binding of the barcode to another entity (e.g., cell receptor). For example, an affinity property can comprise an antibody, for example, an antibody specific for a specific moiety (e.g., receptor) on a sample. In some embodiments, the antibody can guide the barcode to a specific cell type or molecule. Targets at and/or near the specific cell type or molecule can be labeled (e.g., stochastically labeled). The affinity property can, in some embodiments, provide spatial information in addition to the nucleotide sequence of the spatial label because the antibody can guide the barcode to a specific location. The antibody can be a therapeutic antibody, for example a monoclonal antibody or a polyclonal antibody. The antibody can be humanized or chimeric. The antibody can be a naked antibody or a fusion antibody.

The antibody can be a full-length (i.e., naturally occurring or formed by normal immunoglobulin gene fragment recombinatorial processes) immunoglobulin molecule (e.g., an IgG antibody) or an immunologically active (i.e., specifically binding) portion of an immunoglobulin molecule, like an antibody fragment.

The antibody fragment can be, for example, a portion of an antibody such as F(ab')2, Fab', Fab, Fv, sFv and the like. In some embodiments, the antibody fragment can bind with the same antigen that is recognized by the full-length antibody. The antibody fragment can include isolated fragments consisting of the variable regions of antibodies, such as the "Fv" fragments consisting of the variable regions of the heavy and light chains and recombinant single chain polypeptide molecules in which light and heavy variable regions are connected by a peptide linker ("scFv proteins"). Exemplary antibodies can include, but are not limited to, antibodies for cancer cells, antibodies for viruses, antibodies that bind to cell surface receptors (CD8, CD34, CD45), and therapeutic antibodies.

*Universal Adaptor Primer*

A barcode can comprise one or more universal adaptor primers. For example, a gene-specific barcode, such as a gene-specific stochastic barcode, can comprise a universal adaptor primer. A universal adaptor primer can refer to a nucleotide sequence that is universal across all barcodes. A universal adaptor primer can be used for building gene-specific barcodes. A universal adaptor primer can be, or be about, 1, 2, 3, 4, 5, 6, 7, 8, 9, 10, 11, 12, 13, 14, 15, 16, 17, 18, 19, 20, 21, 22, 23, 24, 25, 26 27, 28, 29, 30, or a number or a range between any two of these nucleotides in length. A universal adaptor primer can be at least, or be at most, 1, 2, 3, 4, 5, 6, 7, 8, 9, 10, 11, 12, 13, 14, 15, 16, 17, 18, 19, 20, 21, 22, 23, 24, 25, 26 27, 28, 29, or 30 nucleotides in length. A universal adaptor primer can be from 5-30 nucleotides in length.

Solid Supports

Barcodes, such as stochastic barcodes, disclosed herein can, in some embodiments, be associated with a solid support. The solid support can be, for example, a synthetic particle. In some embodiments, some or all of the barcode sequences, such as molecular labels for stochastic barcodes (e.g., the first barcode sequences) of a plurality of barcodes (e.g., the first plurality of barcodes) on a solid support differ by at least one nucleotide. The cell labels of the barcodes on the same solid support can be the same. The cell labels of the barcodes on different solid supports can differ by at least one nucleotide. For example, first cell labels of a first plurality of barcodes on a first solid support can have the same sequence, and second cell labels of a second plurality of barcodes on a second solid support can have the same sequence. The first cell labels of the first plurality of barcodes on the first solid support and the second cell labels of the second plurality of barcodes on the second solid support can differ by at least one nucleotide. A cell label can be, for example, about 5-20 nucleotides long. A barcode sequence can be, for example, about 5-20 nucleotides long. The synthetic particle can be, for example, a bead.

The bead can be, for example, a silica gel bead, a controlled pore glass bead, a magnetic bead, a Dynabead, a Sephadex/Sepharose bead, a cellulose bead, a polystyrene bead, or any combination thereof. The bead can comprise a material such as polydimethylsiloxane (PDMS), polystyrene, glass, polypropylene, agarose, gelatin, hydrogel, paramagnetic, ceramic, plastic, glass, methylstyrene, acrylic polymer, titanium, latex, Sepharose, cellulose, nylon, silicone, or any combination thereof.

In some embodiments, the bead can be a polymeric bead, for example a deformable bead or a gel bead, functionalized with barcodes or stochastic barcodes (such as gel beads from 10X Genomics (San Francisco, CA). In some implementation, a gel bead can comprise a polymer based gels. Gel beads can be generated, for example, by encapsulating one or more polymeric precursors into droplets. Upon exposure of the polymeric precursors to an accelerator (e.g., tetramethylethylenediamine (TEMED)), a gel bead may be generated.

In some embodiments, the particle can be degradable. For example, the polymeric bead can dissolve, melt, or degrade, for example, under a desired condition. The desired condition can include an environmental condition. The desired condition may result in the polymeric bead dissolving, melting, or degrading in a controlled manner. A gel bead may dissolve, melt, or degrade due to a chemical stimulus, a physical stimulus, a biological stimulus, a thermal stimulus, a magnetic stimulus, an electric stimulus, a light stimulus, or any combination thereof.

Analytes and/or reagents, such as oligonucleotide barcodes, for example, may be coupled/immobilized to the interior surface of a gel bead (e.g., the interior accessible via diffusion of an oligonucleotide barcode and/or materials used to generate an oligonucleotide barcode) and/or the outer surface of a gel bead or any other microcapsule described herein. Coupling/immobilization may be via any form of chemical bonding (e.g., covalent bond, ionic bond) or physical phenomena (e.g., Van der Waals forces, dipole-dipole interactions, etc.). In some embodiments, coupling/immobilization of a reagent to a gel bead or any other microcapsule described herein may be reversible, such as, for example, via a labile moiety (e.g., via a chemical cross-linker, including chemical cross-linkers described herein). Upon application of a stimulus, the labile moiety may be cleaved and the immobilized reagent set free. In some embodiments, the labile moiety is a disulfide bond. For example, in the case where an oligonucleotide barcode is immobilized to a gel bead via a disulfide bond, exposure of the disulfide bond to a reducing agent can cleave the disulfide bond and free the oligonucleotide barcode from the bead. The labile moiety may be included as part of a gel bead or microcapsule, as part of a chemical linker that links a reagent or analyte to a gel bead or microcapsule, and/or as part of a reagent or analyte. In some embodiments, at least one barcode of the plurality of barcodes can be immobilized on the particle, partially immobilized on the particle, enclosed in the particle, partially enclosed in the particle, or any combination thereof.

In some embodiments, a gel bead can comprise a wide range of different polymers including but not limited to: polymers, heat sensitive polymers, photosensitive polymers, magnetic polymers, pH sensitive polymers, salt-sensitive polymers, chemically sensitive polymers, polyelectrolytes, polysaccharides, peptides, proteins, and/or plastics. Polymers may include but are not limited to materials such as poly(N-isopropylacrylamide) (PNIPAAm), poly(styrene sulfonate) (PSS), poly(allyl amine) (PAAm), poly(acrylic acid) (PAA), poly(ethylene imine) (PEI), poly(diallyldimethyl-ammonium chloride) (PDADMAC), poly(pyrolle) (PPy), poly(vinylpyrrolidone) (PVPON), poly(vinyl pyridine) (PVP), poly(methacrylic acid) (PMAA), poly(methyl methacrylate) (PMMA), polystyrene (PS), poly(tetrahydrofuran) (PTHF), poly(phthaladehyde) (PTHF), poly(hexyl viologen) (PHV), poly(L-lysine) (PLL), poly(L-arginine) (PARG), poly(lactic-co-glycolic acid) (PLGA).

Numerous chemical stimuli can be used to trigger the disruption, dissolution, or degradation of the beads. Examples of these chemical changes may include, but are not limited to pH-mediated changes to the bead wall, disintegration of the bead wall via chemical cleavage of crosslink bonds, triggered depolymerization of the bead wall, and bead wall switching reactions. Bulk changes may also be used to trigger disruption of the beads.

Bulk or physical changes to the microcapsule through various stimuli also offer many advantages in designing capsules to release reagents. Bulk or physical changes occur on a macroscopic scale, in which bead rupture is the result of mechano-physical forces induced by a stimulus. These processes may include, but are not limited to pressure induced rupture, bead wall melting, or changes in the porosity of the bead wall.

Biological stimuli may also be used to trigger disruption, dissolution, or degradation of beads. Generally, biological triggers resemble chemical triggers, but many examples use biomolecules, or molecules commonly found in living systems such as enzymes, peptides, saccharides, fatty acids, nucleic acids and the like. For example, beads may comprise polymers with peptide cross-links that are sensitive to cleavage by specific proteases. More specifically, one example may comprise a microcapsule comprising GFLGK peptide cross links. Upon addition of a biological trigger such as the protease Cathepsin B, the peptide cross links of the shell well are cleaved and the contents of the beads are released. In other cases, the proteases may be heat-activated. In another example, beads comprise a shell wall comprising cellulose. Addition of the hydrolytic enzyme chitosan serves as biologic trigger for cleavage of cellulosic bonds, depolymerization of the shell wall, and release of its inner contents.

The beads may also be induced to release their contents upon the application of a thermal stimulus. A change in temperature can cause a variety changes to the beads. A change in heat may cause melting of a bead such that the bead wall disintegrates. In other cases, the heat may increase the internal pressure of the inner components of the bead such that the bead ruptures or explodes. In still other cases, the heat may transform the bead into a shrunken dehydrated state. The heat may also act upon heatsensitive polymers within the wall of a bead to cause disruption of the bead.

Inclusion of magnetic nanoparticles to the bead wall of microcapsules may allow triggered rupture of the beads as well as guide the beads in an array. A device of this disclosure may comprise magnetic beads for either purpose. In one example, incorporation of $Fe_3O_4$ nanoparticles into polyelectrolyte containing beads triggers rupture in the presence of an oscillating magnetic field stimulus.

A bead may also be disrupted, dissolved, or degraded as the result of electrical stimulation. Similar to magnetic particles described in the previous section, electrically sensitive beads can allow for both triggered rupture of the beads as well as other functions such as alignment in an electric field, electrical conductivity or redox reactions. In one example, beads containing electrically sensitive material are aligned in an electric field such that release of inner reagents can be controlled. In other examples, electrical fields may induce redox reactions within the bead wall itself that may increase porosity.

A light stimulus may also be used to disrupt the beads. Numerous light triggers are possible and may include systems that use various molecules such as nanoparticles and chromophores capable of absorbing photons of specific ranges of wavelengths. For example, metal oxide coatings can be used as capsule triggers. UV irradiation of polyelectrolyte capsules coated with $SiO_2$ may result in disintegration of the bead wall. In yet another example, photo switchable materials such as azobenzene groups may be incorporated in the bead wall. Upon the application of UV or visible light, chemicals such as these undergo a reversible cis-to-trans isomerization upon absorption of photons. In this aspect, incorporation of photon switches result in a bead wall that may disintegrate or become more porous upon the application of a light trigger.

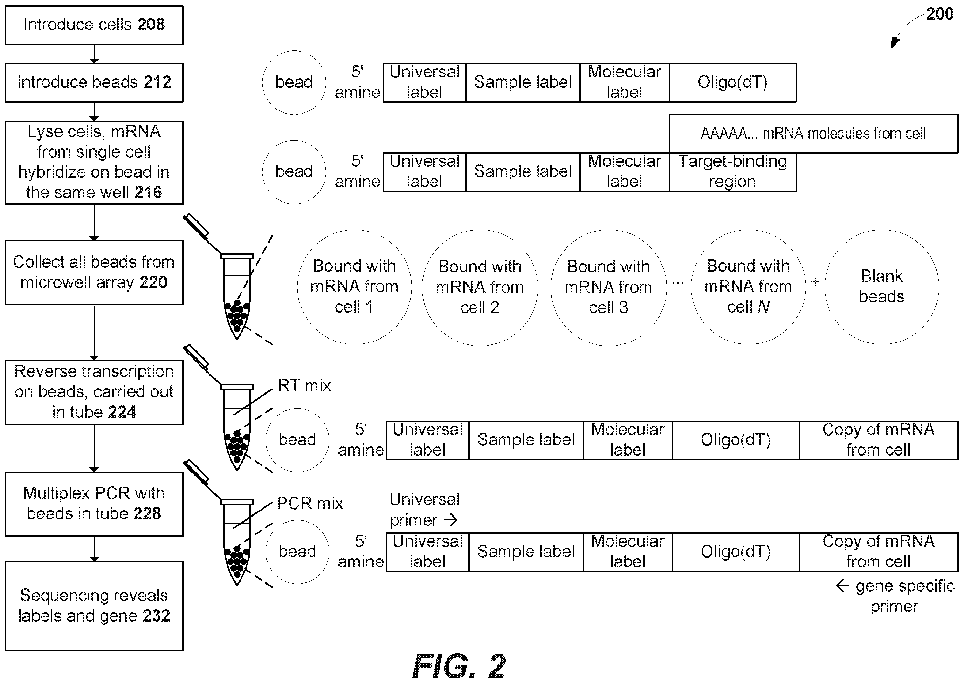

For example, in a non-limiting example of barcoding (e.g., stochastic barcoding) illustrated in FIG. 2, after introducing cells such as single cells onto a plurality of microwells of a microwell array at block 208, beads can be introduced onto the plurality of microwells of the micro well array at block 212. Each microwell can comprise one bead. The beads can comprise a plurality of arcodes. A barcode can comprise a 5' amine region attached to a bead. The barcode can comprise a universal label, a barcode sequence (e.g., a molecular label), a target-binding region, or any combination thereof.

The barcodes disclosed herein can be associated with (e.g., attached to) a solid support (e.g., a bead). The barcodes associated with a solid support can each comprise a barcode sequence selected from a group comprising at least 100 or 1000 barcode sequences with unique sequences. In some embodiments, different barcodes associated with a solid support can comprise barcode with different sequences. In some embodiments, a percentage of barcodes associated with a solid support comprises the same cell label. For example, the percentage can be, or be about 60%, 70%, 80%, 85%, 90%, 95%, 97%, 99%, 100%, or a number or a range between any two of these values. As another example, the percentage can be at least, or be at most 60%, 70%, 80%, 85%, 90%, 95%, 97%, 99%, or 100%. In some embodiments, barcodes associated with a solid support can have the same cell label. The barcodes associated with different solid supports can have different cell labels selected from a group comprising at least 100 or 1000 cell labels with unique sequences.

The barcodes disclosed herein can be associated to (e.g., attached to) a solid support (e.g., a bead). In some embodiments, barcoding the plurality of targets in the sample can be performed with a solid support including a plurality of synthetic particles associated with the plurality of barcodes. In some embodiments, the solid support can include a plurality of synthetic particles associated with the plurality of barcodes. The spatial labels of the plurality of barcodes on different solid supports can differ by at least one nucleotide. The solid support can, for example, include the plurality of barcodes in two dimensions or three dimensions. The synthetic particles can be beads. The beads can be silica gel beads, controlled pore glass beads, magnetic beads, Dynabeads, Sephadex/Sepharose beads, cellulose beads, polystyrene beads, or any combination thereof. The solid support can include a polymer, a matrix, a hydrogel, a needle array device, an antibody, or any combination thereof. In some embodiments, the solid supports can be free floating. In some embodiments, the solid supports can be embedded in a semi-solid or solid array. The barcodes may not be associated with solid supports. The barcodes can be individual nucleotides. The barcodes can be associated with a substrate.

As used herein, the terms "tethered," "attached," and "immobilized," are used interchangeably, and can refer to covalent or non-covalent means for attaching barcodes to a solid support. Any of a variety of different solid supports can be used as solid supports for attaching pre-synthesized barcodes or for *in situ* solid-phase synthesis of barcode.

In some embodiments, the solid support is a bead. The bead can comprise one or more types of solid, porous, or hollow sphere, ball, bearing, cylinder, or other similar configuration which a nucleic acid can be immobilized (e.g., covalently or non-covalently). The bead can be, for example, composed of plastic, ceramic, metal, polymeric material, or any combination thereof. A bead can be, or comprise, a discrete particle that is spherical (e.g., microspheres) or have a non-spherical or irregular shape, such as cubic, cuboid, pyramidal, cylindrical, conical, oblong, or disc-shaped, and the like. In some embodiments, a bead can be non-spherical in shape.

Beads can comprise a variety of materials including, but not limited to, paramagnetic materials (e.g., magnesium, molybdenum, lithium, and tantalum), superparamagnetic materials (e.g., ferrite ($Fe_3O_4$; magnetite) nanoparticles), ferromagnetic materials (e.g., iron, nickel, cobalt, some alloys thereof, and some rare earth metal compounds), ceramic, plastic, glass, polystyrene, silica, methylstyrene, acrylic polymers, titanium, latex, Sepharose, agarose, hydrogel, polymer, cellulose, nylon, or any combination thereof.

In some embodiments, the bead (e.g., the bead to which the labels are attached) is a hydrogel bead. In some embodiments, the hydrogel bead is dissolvable. In some embodiments, the bead comprises hydrogel.

Some embodiments disclosed herein include one or more particles (for example, beads). Each of the particles can comprise a plurality of oligonucleotides (e.g., barcodes). Each of the plurality of oligonucleotides can comprise a barcode sequence (e.g., a molecular label sequence), a cell label, and a target-binding region (e.g., an oligo(dT) sequence, a gene-specific sequence, a random multimer, or a combination thereof). The cell label sequence of each of the plurality of oligonucleotides can be the same. The cell label sequences of oligonucleotides on different particles can be different such that the oligonucleotides on different particles can be identified. The number of different cell label sequences can be different in different implementations. In some embodiments, the number of cell label sequences can be, can be about, can be at least, or can be at most, 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, $10^6$, $10^7$, $10^8$, $10^9$, a number or a range between any two of these values, or more. In some embodiments, no more than, or no more than about, 1, 2, 3, 4, 5, 6, 7, 8, 9, 10, 20, 30, 40, 50, 60, 70, 80, 90, 100, 200, 300, 400, 500, 600, 700, 800, 900, 1000, or more of the plurality of the particles include oligonucleotides with the same cell sequence.

The plurality of oligonucleotides on each particle can comprise different barcode sequences (e.g., molecular labels). In some embodiments, the number of barcode sequences can be, can be about, can be at least, or can be at most, 10, 100, 200, 300, 400, 500, 600, 700, 800, 900, 1000, 2000, 3000, 4000, 5000, 6000, 7000, 8000, 9000, 10000, 20000, 30000, 40000, 50000, 60000, 70000, 80000, 90000, 100000, $10^6$, $10^7$, $10^8$, $10^9$, or a number or a range between any two of these values. As another example, in a single particle, at least 100, 500, 1000, 5000, 10000, 15000, 20000, 50000, a number or a range between any two of these values, or more of the plurality of oligonucleotides comprise different barcode sequences. Some embodiments provide a plurality of the particles comprising barcodes. In some embodiments, the ratio of an occurrence (or a copy or a number) of a target to be labeled and the different barcode sequences can be at least 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, or more. In some embodiments, each of the plurality of oligonucleotides further comprises a sample label, a universal label, or both. The particle can be, for example, a nanoparticle or microparticle.

Methods of Barcoding

The disclosure provides for methods for estimating the number of distinct targets at distinct locations in a physical sample (e.g., tissue, organ, tumor, cell). The methods can comprise placing barcodes (e.g., stochastic barcodes) in close proximity with the sample, lysing the sample, associating distinct targets with the barcodes, amplifying the targets and/or digitally counting the targets. The method can further comprise analyzing and/or visualizing the information obtained from the spatial labels on the barcodes. In some embodiments, a method comprises visualizing the plurality of targets in the sample. Mapping the plurality of targets onto the map of the sample can include generating a two dimensional map or a three dimensional map of the sample. The two dimensional map and the three dimensional map can be generated prior to or after barcoding (e.g., stochastically barcoding) the plurality of targets in the sample. Visualizing the plurality of targets in the sample can include mapping the plurality of targets onto a map of the sample. Mapping the plurality of targets onto the map of the sample can include generating a two dimensional map or a three dimensional map of the sample. The two dimensional map and the three dimensional map can be generated prior to or after barcoding the plurality of targets in the sample. in some embodiments, the two dimensional map and the three dimensional map can be generated before or after lysing the sample. Lysing the sample before or after generating the two dimensional map or the three dimensional map can include heating the sample, contacting the sample with a detergent, changing the pH of the sample, or any combination thereof.

In some embodiments, barcoding the plurality of targets comprises hybridizing a plurality of barcodes with a plurality of targets to create barcoded targets (e.g., stochastically barcoded targets). Barcoding the plurality of targets can comprise generating an indexed library of the barcoded targets. Generating an indexed library of the barcoded targets can be performed with a solid support comprising the plurality of barcodes (e.g., stochastic barcodes).

*Contacting a Sample and a Barcode*

The disclosure provides for methods for contacting a sample (e.g., cells) to a substrate of the disclosure. A sample comprising, for example, a cell, organ, or tissue thin section, can be contacted to barcodes (e.g., stochastic barcodes). The cells can be contacted, for example, by gravity flow wherein the cells can settle and create a monolayer. The sample can be a tissue thin section. The thin section can be placed on the substrate. The sample can be one-dimensional (e.g., formsa planar surface). The sample (e.g., cells) can be spread across the substrate, for example, by growing/culturing the cells on the substrate.

When barcodes are in close proximity to targets, the targets can hybridize to the barcode. The barcodes can be contacted at a non-depletable ratio such that each distinct target can associate with a distinct barcode of the disclosure. To ensure efficient association between the target and the barcode, the targets can be cross-linked to barcode.

*Cell Lysis*

Following the distribution of cells and barcodes, the cells can be lysed to liberate the target molecules. Cell lysis can be accomplished by any of a variety of means, for example, by chemical or biochemical means, by osmotic shock, or by means of thermal lysis, mechanical lysis, or optical lysis. Cells can be lysed by addition of a cell lysis buffer comprising a detergent (e.g., SDS, Li dodecyl sulfate, Triton X-100, Tween-20, or NP-40), an organic solvent (e.g., methanol or acetone), or digestive enzymes (e.g., proteinase K, pepsin, or trypsin), or any combination thereof. To increase the association of a target and a barcode, the rate of the diffusion of the target molecules can be altered by for example, reducing the temperature and/or increasing the viscosity of the lysate.

In some embodiments, lysis can be performed by mechanical lysis, heat lysis, optical lysis, and/or chemical lysis. A lysed cell can comprise at least about 100000, 200000, 300000, 400000, 500000, 600000, or 700000 or more target nucleic acid molecules. A lysed cell can comprise at most about 100000, 200000, 300000, 400000, 500000, 600000, or 700000 or more target nucleic acid molecules.

*Attachment of Barcodes to Target Nucleic Acid Molecules*

Following lysis of the cells and release of nucleic acid molecules therefrom, the nucleic acid molecules can randomly associate with the barcodes of the co-localized solid support. Association can comprise hybridization of a barcode's target recognition region to a complementary portion of the target nucleic acid molecule (e.g., oligo(dT) of the barcode can interact with a poly(A) tail of a target). The assay conditions used for hybridization (e.g., buffer pH, ionic strength, temperature, etc.) can be chosen to promote formation of specific, stable hybrids. In some embodiments, the nucleic acid molecules released from the lysed cells can associate with the plurality of probes on the substrate (e.g., hybridize with the probes on the substrate). When the probes comprise oligo(dT), mRNA molecules can hybridize to the probes and be reverse transcribed. The oligo(dT) portion of the oligonucleotide can act as a primer for first strand synthesis of the cDNA molecule. For example, in a non-limiting example of barcoding illustrated in FIG. 2, at block 216, mRNA molecules can hybridize to barcodes on beads. For example, single-stranded nucleotide fragments can hybridize to the target-binding regions of barcodes.

Attachment can further comprise ligation of a barcode's target recognition region and a portion of the target nucleic acid molecule. For example, the target binding region can comprise a nucleic acid sequence that can be capable of specific hybridization to a restriction site overhang (e.g., an EcoRI sticky-end overhang). The assay procedure can further comprise treating the target nucleic acids with a restriction enzyme (e.g., EcoRI) to create a restriction site overhang. The barcode can then be ligated to any nucleic acid molecule comprising a sequence complementary to the restriction site overhang. A ligase (e.g., T4 DNA ligase) can be used to join the two fragments.

For example, in a non-limiting example of barcoding illustrated in FIG. 2, at block 220, the labeled targets from a plurality of cells (or a plurality of samples) (e.g., target-barcode molecules) can be subsequently pooled, for example, into a tube. The labeled targets can be pooled by, for example, retrieving the barcodes and/or the beads to which the target-barcode molecules are attached.

The retrieval of solid support--based collections of attached target-barcode molecules can be implemented by use of magnetic beads and an externally-applied magnetic field. Once the target-barcode molecules have been pooled, all further processing can proceed in a single reaction vessel. Further processing can include, for example, reverse transcription reactions, amplification reactions, cleavage reactions, dissociation reactions, and/or nucleic acid extension reactions. Further processing reactions can be performed within the microwells, that is, without first pooling the labeled target nucleic acid molecules from a plurality of cells.

*Reverse Transcription*

The disclosure provides for a method to create a target-barcode conjugate using reverse transcription (e.g., at block 224 of FIG. 2). The target-barcode conjugate can comprise the barcode and a complementary sequence of all or a portion of the target nucleic acid (i.e., a barcoded cDNA molecule, such as a stochastically barcoded cDNA molecule). Reverse transcription of the associated RNA molecule can occur by the addition of a reverse transcription primer along with the reverse transcriptase. The reverse transcription primer can be an oligo(dT) primer, a random hexanucleotide primer, or a target-specific oligonucleotide primer. Oligo(dT) primers can be, or can be about, 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

In some embodiments, reverse transcription of the labeled-RNA molecule can occur by the addition of a reverse transcription primer. In some embodiments, the reverse transcription primer is an oligo(dT) primer, random hexanucleotide primer, or a target-specific oligonucleotide primer. Generally, oligo(dT) primers are 12-18 nucleotides in length and bind to the endogenous poly(A) tail at the 3' end of mammalian mRNA. Random hexanucleotide primers can bind to mRNA at a variety of complementary sites. Target-specific oligonucleotide primers typically selectively prime the mRNA of interest.

Reverse transcription can occur repeatedly to produce multiple labeled-cDNA molecules. The methods disclosed herein can comprise conducting at least about 1, 2, 3, 4, 5, 6, 7, 8, 9, 10, 11, 12, 13, 14, 15, 16, 17, 18, 19, or 20 reverse transcription reactions. The method can comprise conducting at least about 25, 30, 35, 40, 45, 50, 55, 60, 65, 70, 75, 80, 85, 90, 95, or 100 reverse transcription reactions.

*Amplification*

One or more nucleic acid amplification reactions (e.g., at block 228 of FIG. 2) can be performed to create multiple copies of the labeled target nucleic acid molecules. Amplification can be performed in a multiplexed manner, wherein multiple target nucleic acid sequences are amplified simultaneously. The amplification reaction can be used to add sequencing adaptors to the nucleic acid molecules. The amplification reactions can comprise amplifying at least a portion of a sample label, if present. The amplification reactions can comprise amplifying at least a portion of the cellular label and/or barcode sequence (e.g., a molecular label). The amplification reactions can comprise amplifying at least a portion of a sample tag, a cell label, a spatial label, a barcode sequence (e.g., a molecular label), a target nucleic acid, or a combination thereof. The amplification reactions can comprise amplifying 0.5%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95%, 97%, 100%, or a range or a number between any two of these values, of the plurality of nucleic acids. The method can further comprise conducting one or more cDNA synthesis reactions to produce one or more cDNA copies of target-barcode molecules comprising a sample label, a cell label, a spatial label, and/or a barcode sequence (e.g., a a molecular label).

In some embodiments, amplification can be performed using a polymerase chain reaction (PCR). As used herein, PCR can refer to a reaction for the in vitro amplification of specific DNA sequences by the simultaneous primer extension of complementary strands of DNA. As used herein, PCR can encompass derivative forms of the reaction, including but not limited to, RT-PCR, real-time PCR, nested PCR, quantitative PCR, multiplexed PCR, digital PCR, and assembly PCR.

Amplification of the labeled nucleic acids can comprise non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerasedriven RNA transcription amplification or RNA-directed DNA synthesis and transcription to amplify DNA or RNA targets, a ligase chain reaction (LCR), and a Qβ replicase (Qβ) method, use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and ramification extension amplification (RAM). In some embodiments, the amplification does not produce circularized transcripts.

In some embodiments, the methods disclosed herein further comprise conducting a polymerase chain reaction on the labeled nucleic acid (e.g., labeled-RNA, labeled-DNA, labeled-cDNA) to produce a labeled amplicon (e.g., a stochastically labeledamplicon). The labeled amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample label, a spatial label, a cell label, and/or a barcode sequence (e.g., a molecular label). The labeled amplicon can be a single-stranded molecule.

The single-stranded molecule can comprise DNA, RNA, or a combination thereof. The nucleic acids of the disclosure can comprise synthetic or altered nucleic acids.

Amplification can comprise use of one or more non-natural nucleotides. Non-natural nucleotides can comprise photolabile or triggerable nucleotides. Examples of non-natural nucleotides can include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

The one or more primers can comprise a universal primer. The universal primer can anneal to a universal primer binding site. The one or more custom primers can anneal to a first sample label, a second sample label, a spatial label, a cell label, a barcode sequence (e.g., a molecular label), a target, or any combination thereof. The one or more primers can comprise a universal primer and a custom primer. The custom primer can be designed to amplify one or more targets. The targets can comprise a subset of the total nucleic acids in one or more samples. The targets can comprise a subset of the total labeled targets in one or more samples. The one or more primers can comprise at least 96 or more custom primers. The one or more primers can comprise at least 960 or more custom primers. The one or more primers can comprise at least 9600 or more custom primers. The one or more custom primers can anneal to two or more different labeled nucleic acids. The two or more different labeled nucleic acids can correspond to one or more genes.

Any amplification scheme can be used in the methods of the present disclosure. For example, in one scheme, the first round PCR can amplify molecules attached to the bead using a gene specific primer and a primer against the universal Illumina sequencing primer 1 sequence. The second round of PCR can amplify the first PCR products using a nested gene specific primer flanked by Illumina sequencing primer 2 sequence, and a primer against the universal Illumina sequencing primer 1 sequence. The third round of PCR adds P5 and P7 and sample index to turn PCR products into an Illumina sequencing library. Sequencing using 150 bp x 2 sequencing can reveal the cell label and barcode sequence (e.g., molecular label) on read 1, the gene on read 2, and the sample index on index 1 read.

In some embodiments, nucleic acids can be removed from the substrate using chemical cleavage. For example, a chemical group or a modified base present in a nucleic acid can be used to facilitate its removal from a solid support. For example, an enzyme can be used to remove a nucleic acid from a substrate. For example, a nucleic acid can be removed from a substrate through a restriction endonuclease digestion. For example, treatment of a nucleic acid containing a dUTP or ddUTP with uracil-d-glycosylase (UDG) can be used to remove a nucleic acid from a substrate. For example, a nucleic acid can be removed from a substrate using an enzyme that performs nucleotide excision, such as a base excision repair enzyme, such as an apurinic/apyrimidinic (AP) endonuclease. In some embodiments, a nucleic acid can be removed from a substrate using a photocleavable group and light. In some embodiments, a cleavable linker can be used to remove a nucleic acid from the substrate. For example, the cleavable linker can comprise at least one of biotin/avidin, biotin/streptavidin, biotin/neutravidin, Ig-protein A, a photo--labile linker, acid or base labile linker group, or an aptamer.

When the probes are gene-specific, the molecules can hybridize to the probes and be reverse transcribed and/or amplified. In some embodiments, after the nucleic acid has been synthesized (e.g., reverse transcribed), it can be amplified. Amplification can be performed in a multiplex manner, wherein multiple target nucleic acid sequences are amplified simultaneously. Amplification can add sequencing adaptors to the nucleic acid.

In some embodiments, amplification can be performed on the substrate, for example, with bridge amplification. cDNAs can be homopolymer tailed in order to generate a compatible end for bridge amplification using oligo(dT) probes on the substrate. In bridge amplification, the primer that is complementary to the 3' end of the template nucleic acid can be the first primer of each pair that is covalently attached to the solid particle. When a sample containing the template nucleic acid is contacted with the particle and a single thermal cycle is performed, the template molecule can be annealed to the first primer and the first primer is elongated in the forward direction by addition of nucleotides to form a duplex molecule consisting of the template molecule and a newly formed DNA strand that is complementary to the template. In the heating step of the next cycle, the duplex molecule can be denatured, releasing the template molecule from the particle and leaving the complementary DNA strand attached to the particle through the first primer. In the annealing stage of the annealing and elongation step that follows, the complementary strand can hybridize to the second primer, which is complementary to a segment of the complementary strand at a location removed from the first primer. This hybridization can cause the complementary strand to form a bridge between the first and second primers secured to the first primer by a covalent bond and to the second primer by hybridization. In the elongation stage, the second primer can be elongated in the reverse direction by the addition of nucleotides in the same reaction mixture, thereby converting the bridge to a double-stranded bridge. The next cycle then begins, and the double-stranded bridge can be denatured to yield two single-stranded nucleic acid molecules, each having one end attached to the particle surface via the first and second primers, respectively, with the other end of each unattached. In the annealing and elongation step of this second cycle, each strand can hybridize to a further complementary primer, previously unused, on the same particle, to form new single-strand bridges. The two previously unused primers that are now hybridized elongate to convert the two new bridges to double-strand bridges.

Amplification of the labeled nucleic acids can comprise PCR-based methods or non-PCR based methods. Amplification of the labeled nucleic acids can comprise exponential amplification of the labeled nucleic acids. Amplification of the labeled nucleic acids can comprise linear amplification of the labeled nucleic acids. Amplification can be performed by polymerase chain reaction (PCR). PCR can refer to a reaction for the in vitro amplification of specific DNA sequences by the simultaneous primer extension of complementary strands of DNA. PCR can encompass derivative forms of the reaction, including but not limited to, RT-PCR, real-time PCR, nested PCR, quantitative PCR, multiplexed PCR, digital PCR, suppression PCR, semi-suppressive PCR and assembly PCR.

In some embodiments, amplification of the labeled nucleic acids comprises non-PCR based methods. Examples of non-PCR based methods include, but are not limited to, multiple displacement amplification (MDA), transcription-mediated amplification (TMA), nucleic acid sequence-based amplification (NASBA), strand displacement amplification (SDA), real-time SDA, rolling circle amplification, or circle-to-circle amplification. Other non-PCR-based amplification methods include multiple cycles of DNA-dependent RNA polymerase-driven RNA transcription amplification or RNA-directed DNA synthesis and transcription to amplify DNA or RNA targets, a ligase chain reaction (LCR), a Qβ replicase (Qβ), use of palindromic probes, strand displacement amplification, oligonucleotide-driven amplification using a restriction endonuclease, an amplification method in which a primer is hybridized to a nucleic acid sequence and the resulting duplex is cleaved prior to the extension reaction and amplification, strand displacement amplification using a nucleic acid polymerase lacking 5' exonuclease activity, rolling circle amplification, and/or ramification extension amplification (RAM).

In some embodiments, the methods disclosed herein further comprise conducting a nested polymerase chain reaction on the amplified amplicon (e.g., target). The amplicon can be double-stranded molecule. The double-stranded molecule can comprise a double-stranded RNA molecule, a double-stranded DNA molecule, or a RNA molecule hybridized to a DNA molecule. One or both of the strands of the double-stranded molecule can comprise a sample tag or molecular identifier label. Alternatively, the amplicon can be a single-stranded molecule. The single-stranded molecule can comprise DNA, RNA, or a combination thereof. The nucleic acids of the present invention can comprise synthetic or altered nucleic acids.

In some embodiments, the method comprises repeatedly amplifying the labeled nucleic acid to produce multiple amplicons. The methods disclosed herein can comprise conducting at least about 1, 2, 3, 4, 5, 6, 7, 8, 9, 10, 11, 12, 13, 14, 15, 16, 17, 18, 19, or 20 amplification reactions. Alternatively, the method comprises conducting at least about 25, 30, 35, 40, 45, 50, 55, 60, 65, 70, 75, 80, 85, 90, 95, or 100 amplification reactions.

Amplification can further comprise adding one or more control nucleic acids to one or more samples comprising a plurality of nucleic acids. Amplification can further comprise adding one or more control nucleic acids to a plurality of nucleic acids. The control nucleic acids can comprise a control label.

Amplification can comprise use of one or more non-natural nucleotides. Non-natural nucleotides can comprise photolabile and/or triggerable nucleotides. Examples of non-natural nucleotides include, but are not limited to, peptide nucleic acid (PNA), morpholino and locked nucleic acid (LNA), as well as glycol nucleic acid (GNA) and threose nucleic acid (TNA). Non-natural nucleotides can be added to one or more cycles of an amplification reaction. The addition of the non-natural nucleotides can be used to identify products as specific cycles or time points in the amplification reaction.

Conducting the one or more amplification reactions can comprise the use of one or more primers. The one or more primers can comprise one or more oligonucleotides. The one or more oligonucleotides can comprise at least about 7-9 nucleotides. The one or more oligonucleotides can comprise less than 12-15 nucleotides. The one or more primers can anneal to at least a portion of the plurality of labeled nucleic acids. The one or more primers can anneal to the 3' end and/or 5' end of the plurality of labeled nucleic acids. The one or more primers can anneal to an internal region of the plurality of labeled nucleic acids. The internal region can be at least about 50, 100, 150, 200, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 650, 700, 750, 800, 850, 900 or 1000 nucleotides from the 3' ends the plurality of labeled nucleic acids. The one or more primers can comprise a fixed panel of primers. The one or more primers can comprise at least one or more custom primers. The one or more primers can comprise at least one or more control primers. The one or more primers can comprise at least one or more housekeeping gene primers. The one or more primers can comprise a universal primer. The universal primer can anneal to a universal primer binding site. The one or more custom primers can anneal to the first sample tag, the second sample tag, the molecular identifier label, the nucleic acid or a product thereof. The one or more primers can comprise a universal primer and a custom primer. The custom primer can be designed to amplify one or more target nucleic acids. The target nucleic acids can comprise a subset of the total nucleic acids in one or more samples. In some embodiments, the primers are the probes attached to the array of the disclosure.

In some embodiments, barcoding (e.g., stochastically barcoding) the plurality of targets in the sample further comprises generating an indexed library of the barcoded targets (e.g., stochastically barcoded targets) or barcoded fragments of the targets. The barcode sequences of different barcodes (e.g., the molecular labels of different stochastic barcodes) can be different from one another. Generating an indexed library of the barcoded targets includes generating a plurality of indexed polynucleotides from the plurality of targets in the sample. For example, for an indexed library of the barcoded targets comprising a first indexed target and a second indexed target, the label region of the first indexed polynucleotide can differ from the label region of the second indexed polynucleotide by, by about, by at least, or by at most, 1, 2, 3, 4, 5, 6, 7, 8, 9, 10, 20, 30, 40, 50, or a number or a range between any two of these values, nucleotides. In some embodiments, generating an indexed library of the barcoded targets includes contacting a plurality of targets, for example mRNA molecules, with a plurality of oligonucleotides including a poly(T) region and a label region; and conducting a first strand synthesis using a reverse transcriptase to produce single-strand labeled cDNA molecules each comprising a cDNA region and a label region, wherein the plurality of targets includes at least two mRNA molecules of different sequences and the plurality of oligonucleotides includes at least two oligonucleotides of different sequences. Generating an indexed library of the barcoded targets can further comprise amplifying the single-strand labeled cDNA molecules to produce double-strand labeled cDNA molecules, and conducting nested PCR on the double-strand labeled cDNA molecules to produce labeled amplicons. In some embodiments, the method can include generating an adaptor-labeled amplicon.

Barcoding (e.g., stochastic barcoding) can include using nucleic acid barcodes or tags to label individual nucleic acid (e.g., DNA or RNA) molecules. In some embodiments, it involves adding DNA barcodes or tags to cDNA molecules as they are generated from mRNA. Nested PCR can be performed to minimize PCR amplification bias. Adaptors can be added for sequencing using, for example, next generation sequencing (NGS). The sequencing results can be used to determine cell labels, molecular labels, and sequences of nucleotide fragments of the one or more copies of the targets, for example at block 232 of FIG. 2.

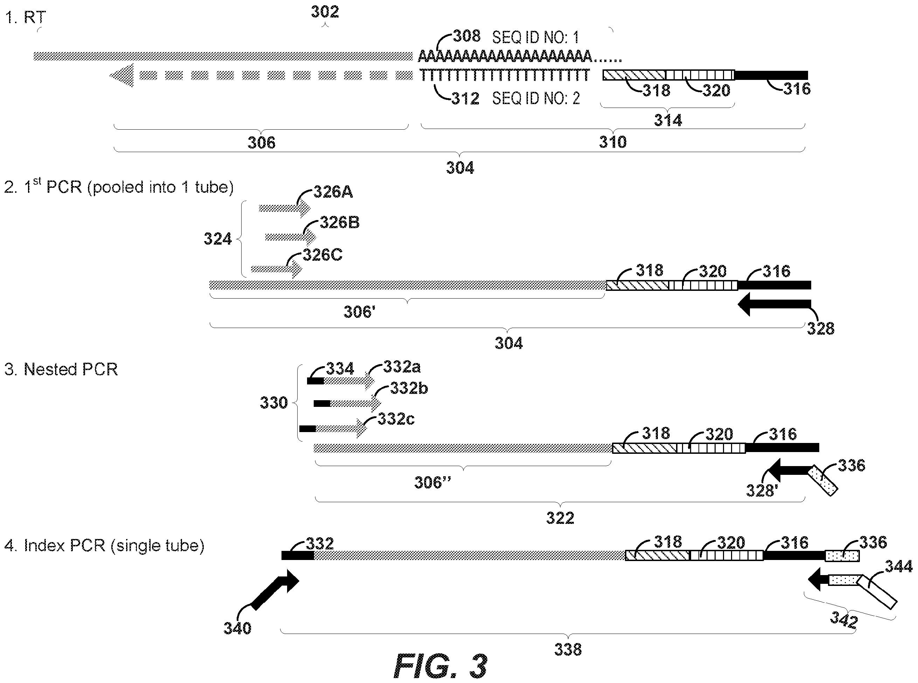

FIG. 3 is a schematic illustration showing a non-limiting exemplary process of generating an indexed library of the barcoded targets (e.g., stochastically barcoded targets), such as barcoded mRNAs or fragments thereof. As shown in step 1, the reverse transcription process can encode each mRNA molecule with a unique molecular label, a cell label, and a universal PCR site. In particular, RNA molecules 302 can be reverse transcribed to produce labeled cDNA molecules 304, including a cDNA region 306, by hybridization (e.g., stochastic hybridization) of a set of barcodes (e.g., stochastic barcodes) 310 to the poly(A) tail region 308 of the RNA molecules 302. Each of the barcodes 310 can comprise a target-binding region, for example a poly(dT) region 312, a label region 314 (e.g., a barcode sequence or a molecule), and a universal PCR region 316.

In some embodiments, the cell label can include 3 to 20 nucleotides. In some embodiments, the molecular label can include 3 to 20 nucleotides. In some embodiments, each of the plurality of stochastic barcodes further comprises one or more of a universal label and a cell label, wherein universal labels are the same for the plurality of stochastic barcodes on the solid support and cell labels are the same for the plurality of stochastic barcodes on the solid support. In some embodiments, the universal label can include 3 to 20 nucleotides. In some embodiments, the cell label comprises 3 to 20 nucleotides.

In some embodiments, the label region 314 can include a barcode sequence or a molecular label 318 and a cell label 320. In some embodiments, the label region 314 can include one or more of a universal label, a dimension label, and a cell label. The barcode sequence or molecular label 318 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The cell label 320 can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. The universal label can be, can be about, can be at least, or can be at most, 1, 2, 3, 4, 5, 6, 7, 8, 9, 10, 20, 30, 40, 50, 60, 70, 80, 90, 100, or a number or a range between any of these values, of nucleotides in length. Universal labels can be the same for the plurality of stochastic barcodes on the solid support and cell labels are the same for the plurality of stochastic barcodes on the solid support. The dimension label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length.

In some embodiments, the label region 314 can comprise, comprise about, comprise at least, or comprise at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different labels, such as a barcode sequence or a molecular label 318 and a cell label 320. Each label can be, can be about, can be at least, or can be at most 1, 2, 3, 4, 5, 6, 7, 8, 9, 10, 20, 30, 40, 50, 60, 70, 80, 90, 100, or a number or a range between any of these values, of nucleotides in length. A set of barcodes or stochastic barcodes 310 can contain, contain about, contain at least, or can be at most, 10, 20, 40, 50, 70, 80, 90, $10^2$, $10^3$, $10^4$, $10^5$, $10^6$, $10^7$, $10^8$, $10^9$, $10^{10}$, $10^{11}$, $10^{12}$, $10^{13}$, $10^{14}$, $10^{15}$, $10^{20}$, or a number or a range between any of these values, barcodes or stochastic barcodes 310. And the set of barcodes or stochastic barcodes 310 can, for example, each contain a unique label region 314. The labeled cDNA molecules 304 can be purified to remove excess barcodes or stochastic barcodes 310. Purification can comprise Ampure bead purification.

As shown in step 2, products from the reverse transcription process in step 1 can be pooled into 1 tube and PCR amplified with a 1st PCR primer pool and a 1st universal PCR primer. Pooling is possible because of the unique label region 314. In particular, the labeled cDNA molecules 304 can be amplified to produce nested PCR labeled amplicons 322. Amplification can comprise multiplex PCR amplification. Amplification can comprise a multiplex PCR amplification with 96 multiplex primers in a single reaction volume. In some embodiments, multiplex PCR amplification can utilize, utilize about, utilize at least, or utilize at most, 10, 20, 40, 50, 70, 80, 90, $10^2$, $10^3$, $10^4$, $10^5$, $10^6$, $10^7$, $10^8$, $10^9$, $10^{10}$, $10^{11}$, $10^{12}$, $10^{13}$, $10^{14}$, $10^{15}$, $10^{20}$, or a number or a range between any of these values, multiplex primers in a single reaction volume. Amplification can comprise using a $1^{st}$ PCR primer pool 324 comprising custom primers 326A-C targeting specific genes and a universal primer 328. The custom primers 326 can hybridize to a region within the cDNA portion 306' of the labeled cDNA molecule 304. The universal primer 328 can hybridize to the universal PCR region 316 of the labeled cDNA molecule 304.

As shown in step 3 of FIG. 3, products from PCR amplification in step 2 can be amplified with a nested PCR primers pool and a $2^{nd}$ universal PCR primer. Nested PCR can minimize PCR amplification bias. In particular, the nested PCR labeled amplicons 322 can be further amplified by nested PCR. The nested PCR can comprise multiplex PCR with nested PCR primers pool 330 of nested PCR primers 332a-c and a $2^{nd}$ universal PCR primer 328' in a single reaction volume. The nested PCR primer pool 328 can contain, contain about, contain at least, or contain at most, 1, 2, 3, 4, 5, 6, 7, 8, 9, 10, 20, 30, 40, 50, 60, 70, 80, 90, 100, 200, 300, 400, 500, 600, 700, 800, 900, 1000, or a number or a range between any of these values, different nested PCR primers 330. The nested PCR primers 332 can contain an adaptor 334 and hybridize to a region within the cDNA portion 306" of the labeled amplicon 322. The universal primer 328' can contain an adaptor 336 and hybridize to the universal PCR region 316 of the labeled amplicon 322. Thus, step 3 produces adaptor-labeled amplicon 338. In some embodiments, nested PCR primers 332 and the $2^{nd}$ universal PCR primer 328' may not contain the adaptors 334 and 336. The adaptors 334 and 336 can instead be ligated to the products of nested PCR to produce adaptor-labeled amplicon 338.

As shown in step 4, PCR products from step 3 can be PCR amplified for sequencing using library amplification primers. In particular, the adaptors 334 and 336 can be used to conduct one or more additional assays on the adaptor-labeled amplicon 338. The adaptors 334 and 336 can be hybridized to primers 340 and 342. The one or more primers 340 and 342 can be PCR amplification primers. The one or more primers 340 and 342 can be sequencing primers. The one or more adaptors 334 and 336 can be used for further amplification of the adaptor-labeled amplicons 338. The one or more adaptors 334 and 336 can be used for sequencing the adaptor-labeled amplicon 338. The primer 342 can contain a plate index 344 so that amplicons generated using the same set of barcodes or stochastic barcodes 310 can be sequenced in one sequencing reaction using next generation sequencing (NGS).

Sequencing Data Errors

Methods disclosed herein can be used for identifying and/or correcting sequencing data errors, for example the errors occurring in the methods for counting one or more target nucleic acids. In some embodiments, the error can comprise, or be, a deletion of one or more nucleotides, a substitution of one or more nucleotides, an addition of one or more nucleotides, or any combination thereof. The error can be present on a molecular label (ML), a sample label (SL), or another other label on a barcode (e.g., a stochastic barcode). In some embodiments, a sequencing data error can comprise, or be, a PCR-introduced error, a sequencing-introduced error, a reverse transcription (RT) primer contamination error, or any combination thereof. The PCR-introduced error can comprise, or be, a result of a PCR amplification error, PCR amplification bias, insufficient PCR amplification, or any combination thereof. The sequencing-introduced error can comprise, or be, a result of inaccurate base calling, insufficient sequencing, or any combination thereof. The RT primer contamination error may be an error caused by a reverse transcription primer entering PCR.

As used herein, the term "coverage" or "sequencing depth" can refer to the number of reads of a barcoded target with a particular ML and a particular SL in sequencing data. For example, a barcoded target may be sequenced multiple times. Accordingly, the barcoded target with a particular ML and SL can be observed multiple times. As another example, a cell may contain multiple copies of a target (for example, multiple copies of mRNA molecules of a gene). These multiple copies of the target can be barcoded. After PCR amplification (for example, block 228 in FIG. 2), there can be multiple copies of a barcoded target with a particular ML and SL. During sequencing, some or all of the multiple copies of the barcoded target with the particular ML and SL may be sequenced. The number of reads the barcoded target with the same ML and SL observed in sequencing data may be referred to as "coverage" or "sequencing depth."

In some embodiments, sequencing data errors can be identified and/or corrected. For example, copies of a target from a cell can be barcoded with different MLs and the same SL. The barcoded target with a ML can have multiple reads in sequencing data. The barcoded target with a different ML can have only a few reads (e.g., one read). The former barcoded target can be more likely to have a true ML (or real or signal ML), compared to the latter barcoded target. The latter barcoded target can include an error ML (or false or noise ML). This may be because that the two MLs can be expected to have similar coverages or sequencing depths. The latter barcoded target with only a few reads can be an artifact or error generated during sequencing or PCR.

As another example, barcode (e.g., a stochastic barcode) entering PCR can result in a RT primer contamination error. In some embodiments, after reverse transcribing mRNA molecules into cDNA molecules (e.g., at block 224 of FIG. 2), barcodes not incorporated into cDNA molecules can be removed by, for example, Ampure bead purification. The removal method, for example Ampure bead purification, may not completely remove the barcodes that are not extended by reverse transcription to be incorporated into barcoded cDNA molecules (e.g., stochastically barcoded cDNA molecules). For example, 15%, 10%, 9%, 8%, 7%, 6%, 5%, 4%, 3%, 2%, 1%, 0.5%, 0.1%, or a range between any of these two values of barcodes that are not extended by reverse transcription to be incorporated into barcoded cDNA molecules may not be removed by Ampure bead purification. These unremoved barcodes can result in sequencing data error during amplification of cDNA molecules (e.g., at block 228 of FIG. 2). The barcodes between samples can be highly similar. For example, sample labels of barcodes can be identical for a sample. Thus, PCR cross over can occur because these unremoved barcodes can hybridize to other nucleic acid molecules from the same sample (for example, the SL regions of barcoded mRNA molecules, such as stochastically barcoded mRNA molecules) during PCR and can result in sequencing data errors referred to as SL errors.

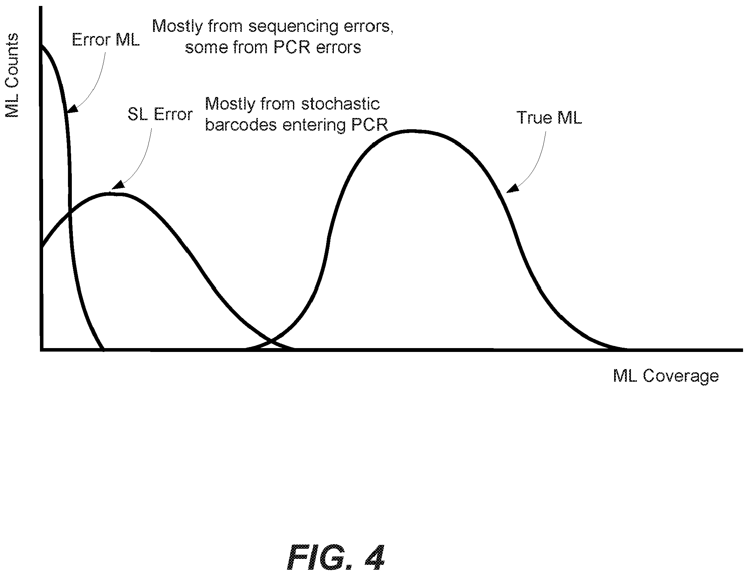

True MLs, error MLs, and SL errors can have distinct distributions. FIG. 4 is a schematic illustration showing non-limiting exemplary distributions of molecular label errors, sample label errors, and true molecular label signals. As illustrated in FIG. 4, error MLs may be more likely to have lower ML coverage because error MLs may be results of PCR or sequencing errors. For example, error MLs may be results of mostly sequencing errors and some PCR errors. SL errors may be more likely to have lower ML coverage because SL errors may be results mostly from barcodes (e.g., stochastic barcodes) entering PCR.

Correcting PCR and Sequencing Errors Based on Directional Adjacency

Disclosed herein are methods for correcting PCR or sequencing errors. In some embodiments, the method comprises: (a) receiving sequencing data of barcoded targets (e.g., stochastically barcoded targets). The barcoded targets can be obtained by barcoding (e.g., stochastically barcoding) a plurality of targets using a plurality of barcodes (e.g., stochastic barcodes) to create a plurality of barcoded targets (e.g., stochastically barcoded targets), wherein each of the plurality of barcodes comprises a molecular label. In some embodiments, the method comprises: (b) for one or more of the plurality of targets: (i) counting the number of molecular labels with distinct sequences associated with the target in the sequencing data; (ii) identifying clusters of molecular labels of the target using directional adjacency; (iii) collapsing the sequencing data received in (b) using the clusters of molecular labels of the target identified in (ii); and (iv) estimating the number of the target, wherein the number of the target estimated correlates with the number of molecular labels with distinct sequences associated with the target in the sequencing data counted in (i) after collapsing the sequencing data in (ii). The plurality of targets can comprise targets of the whole transcriptome of a cell. In some embodiments, the method further comprises: (c) barcoding (e.g., stochastically barcoding) the plurality of targets using the plurality of barcodes to create the plurality of barcoded targets; and (d) sequencing the barcoded targets to generate the sequencing data of stochastically barcoded targets received.

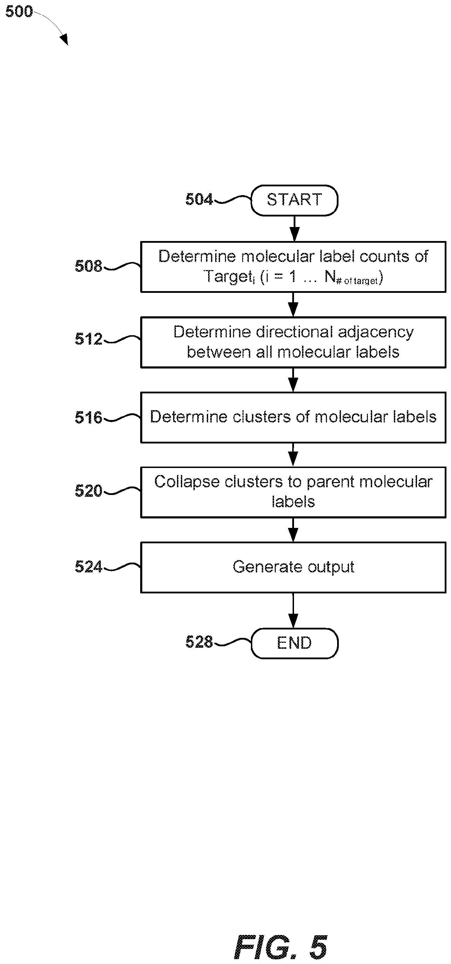

FIG. 5 is a flowchart showing a non-limiting exemplary embodiment 500 of correcting PCR and sequencing errors using molecular labels based on directional adjacency. Correcting PCR and sequencing errors using molecular labels based on directional adjacency may be referred to as recursive substitution error correction (RSEC) The method 500 starts at block 504 after receiving sequencing data of a plurality of barcoded targets (e.g., stochastically barcoded targets). In some embodiments, the method 500 further comprises stochastically barcoding a plurality of targets using a plurality of barcodes (e.g., stochastic barcodes) to create the plurality of barcoded targets (e.g., stochastically barcoded targets), wherein each of the plurality of barcodes comprises a molecular label. In some embodiments, the method 500 further comprises sequencing the plurality of barcoded targets to obtain the sequencing data.

At block 508, for one or more of the plurality of targets: the number of molecular labels with distinct sequences associated with the target in the sequencing data can be counted. At block 512, clusters of molecular labels of the target can be identified using directional adjacency. Molecular labels of the target within a cluster can be within a predetermined directional adjacency threshold of one another. The directional adjacency threshold can vary. In some embodiments, the predetermined directional adjacency threshold can be, be about, be at least, or be at most, a Hamming distance of one or two.

In some embodiments, the molecular labels of the target within the cluster can comprise one or more parent molecular labels and children molecular labels of the one or more parent molecular labels. The occurrence of the parent molecular label can be greater than or equal to a predetermined directional adjacency occurrence threshold. In some embodiments, the predetermined directional adjacency occurrence threshold can be, be about, be at least, or be at most, twice the occurrence of a child molecular label less one. In some embodiments, the predetermined directional adjacency occurrence threshold can be, or be about 1.5 time, 2 times, 3 times, 4 times, 5 times, 6 times, 7 times, 8 times, 9 times, 10 times, or a number or a range between any two of these values, the occurrence of a child molecular label. In some embodiments, the predetermined directional adjacency occurrence threshold can be, at least or at most 1.5 time, 2 times, 3 times, 4 times, 5 times, 6 times, 7 times, 8 times, 9 times, or 10 times, the occurrence of a child molecular label.

At block 520, the sequencing data is collapsed using the clusters of molecular labels of the target. Collapsing the sequencing data can comprise attributing the occurrence of the child molecular label to the parent molecular label. At block 532, the number of the target can be estimated to generate output after collapsing the sequencing data. The method 500 ends at block 536.

In some embodiments, the methods further comprise: determining a sequencing depth of the target. Estimating the number of the target, if the sequencing depth of the target is above a predetermined sequencing depth threshold, comprises adjusting the sequencing data counted in (i). The predetermined sequencing depth threshold can be between 15 and 20. Adjusting the sequencing data counted in (i) comprises: thresholding molecular labels of the target to determine true molecular labels and false molecular labels associated with the target in the sequencing data obtained in (b). Thresholding the molecular labels of the target comprises performing a statistical analysis on the molecular labels of the target. Performing the statistical analysis comprises: fitting the distribution of the molecular labels of the target and their occurrences to two distributions such as two negative binomial distributions; determining the number of true molecular labels n using the two negative binomial distributions; and removing the false molecular labels from the sequencing data obtained in (b), wherein the false molecular labels comprise molecular labels with occurrences lower than the occurrence of the nth most abundant molecular label, and wherein the true molecular labels comprise molecular labels with occurrences greater than or equal to the occurrence of the *n*th most abundant molecular label.

Correcting PCR and Sequencing Errors Based on Directional Adjacency and Distribution-Based Error Correction

Disclosed herein are methods for correcting PCR or sequencing errors. The methods can be used to determine the number of targets. In some embodiments, the method comprises: (a) receiving sequencing data of barcoded targets (e.g., stochastically barcoded targets). The barcoded targets can be obtained by barcoding (e.g., stochastically barcoding) a plurality of targets using a plurality of barcodes (e.g., stochastic barcodes) to create a plurality of barcoded targets (e.g., stochastically barcoded targets), wherein each of the plurality of barcodes comprises a molecular label. In some embodiments, the method comprises (b) for one or more of the plurality of targets: (i) counting the number of molecular labels with distinct sequences associated with the target in the sequencing data; (ii) determining a number of noise molecular labels with distinct sequences associated with the target in the sequencing data; and (iii) estimating the number of the target, wherein the number of the target estimated correlates with the number of molecular labels with distinct sequences associated with the target in the sequencing data counted in (i) adjusted according to the number of noise molecular labels determined in (ii). In some embodiments, the method comprises determining a sequencing status of the target in the sequencing data. In some embodiments, the method further comprises: (c) barcoding (e.g., stochastically barcoding) the plurality of targets using the plurality of barcodes to create the plurality of barcoded targets; and (d) sequencing the barcoded targets to generate the sequencing data of barcoded targets received.

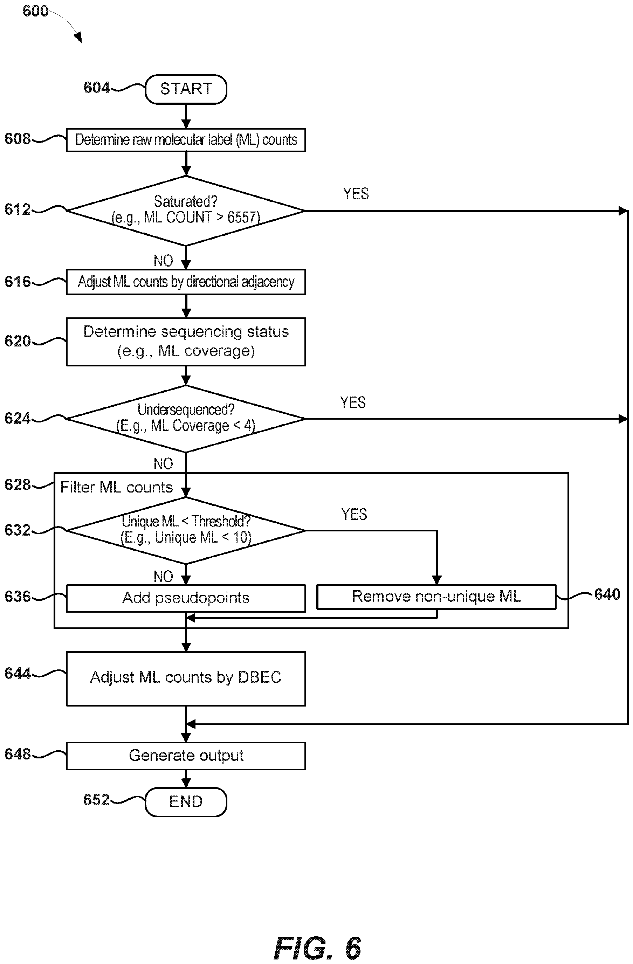

FIG. 6 is a flowchart showing a non-limiting exemplary embodiment 600 of correcting PCR and sequencing errors based on recursive substitution error correction and distribution-based error correction. The method 600 starts at block 604 after receiving sequencing data of a plurality of barcoded targets (e.g., stochastically barcoded targets). In some embodiments, the method 600 further comprises barcoding (e.g., stochastically barcoding) a plurality of targets using a plurality of barcodes (e.g., stochastic barcodes) to create the plurality of barcoded targets, wherein each of the plurality of barcodes comprises a molecular label. In some embodiments, the method 600 further comprises sequencing the plurality of barcoded targets to obtain the sequencing data.

At block 608, for one or more of the plurality of targets: the number of molecular labels with distinct sequences associated with the target in the sequencing data can be counted. At decision block 612, whether the sequencing data has a saturated sequencing status can be determined. For example, the target can be considered to have the saturated sequencing status if it has a number of molecular labels with distinct sequences greater than 1000, 2000, 3000, 4000, 5000, 6000, 7000, 8000, 9000, 10000, 20000, 30000, 40000, 50000, 60000, 70000, 80000, 90000, 100000, or a number or a range between any two of these. As another example, the target can be considered to have the saturated sequencing status if it has a number of molecular labels with distinct sequences greater than 50%, 60%, 70%, 80%, 90%, 95%, 99%, 99.9%, or a number or a range between any two of these, of the molecular labels of the barcodes (e.g., stochastic barcodes) with distinct sequences.

In some embodiments, the saturated sequencing status can be determined by the target having a number of molecular labels with distinct sequences greater than a predetermined saturation threshold. The predetermined saturation threshold can be different in different implementations. For example, the predetermined saturation threshold can be, or be about, 1000, 2000, 3000, 4000, 5000, 6000, 6557, 7000, 8000, 9000, 10000, 20000, 30000, 40000, 50000, 60000, 65532, 70000, 80000, 90000, 100000, or a number or a range between any two of these values. As another example, the predetermined saturation threshold can be at least, or at most, 1000, 2000, 3000, 4000, 5000, 6000, 6557, 7000, 8000, 9000, 10000, 20000, 30000, 40000, 50000, 60000, 65532, 70000, 80000, 90000, or 100000.

In some embodiments, the saturated sequencing status can depend on the number of molecular labels of the barcodes (e.g., stochastic barcodes) with distinct sequences. For example, the predetermined saturation threshold can be about 6557 if the barcodes comprise about 6561 molecular labels with distinct sequences. As another example, the predetermined saturation threshold can be about 65532 if the barcodes (e.g., stochastic barcodes comprise about 65536 molecular labels with distinct sequences. In some embodiments, the saturated sequencing status may not depend on the number of molecular labels of the barcodes with distinct sequences.

If the sequencing data does not have a saturated sequencing status at decision block 612, the method 600 can proceed to block 616, where the molecular label counts can be adjusted based on directional adjacency. In some embodiments, adjusting the molecular label counts based on directional adjacency can be as described with reference to FIG. 5. For example, adjusting the molecular label counts based on directional adjacency can include identifying clusters of molecular labels of the target using directional adjacency; collapsing the sequencing data using the clusters of molecular labels of the target identified; and estimating the number of the target, wherein the number of the target estimated correlates with the number of molecular labels with distinct sequences associated with the target in the sequencing data counted after collapsing the sequencing data.

At block 620, the sequencing status of the target in the sequencing data can be determined. The sequencing status of the target in the sequencing data can include, or be, under sequencing. At decision block 624, whether the sequencing status of the target in the sequencing data is the under sequencing status can be determined. For example, the target can be considered to have the under sequencing status if its depth (e.g., an average, a minimum, or a maximum depth) is smaller than, or smaller than about, 1, 2, 3, 4, 5, 6, 7, 8, 9, 10, 20, 30, 40, 50, 60, 70, 80, 90, 100, or a number or a range between any two of these. As another example, the target can be considered to have the under sequencing status if its depth is smaller than at least, or at most, 1, 2, 3, 4, 5, 6, 7, 8, 9, 10, 20, 30, 40, 50, 60, 70, 80, 90, or 100.

In some embodiments, the under sequencing status can be determined by the target having a depth (e.g., an average, a minimum, or a maximum depth) smaller than a predetermined under sequencing threshold. The under sequencing threshold can be different in different implementations. For example, the under sequencing threshold can be, or be about, 1, 2, 3, 4, 5, 6, 7, 8, 9, 10, 20, 30, 40, 50, 60, 70, 80, 90, 100, or a number or a range between any two of these values. As another example, the under sequencing threshold can be at least, or at most, 1, 2, 3, 4, 5, 6, 7, 8, 9, 10, 20, 30, 40, 50, 60, 70, 80, 90, or 100.

In some embodiments, the under sequencing status may depend on the number of molecular labels of the barcodes (e.g., stochastic barcodes) with distinct sequences. For example, the under sequencing threshold can be 10 (or another threshold number) if the barcodes comprise, or about, 1000, 2000, 3000, 4000, 5000, 6000, 6561, 7000, 8000, 9000, 10000, 20000, 30000, 40000, 50000, 60000, 65532, 70000, 80000, 90000, 100000, or a number or range between any two of these values, molecular labels with distinct sequences. As another example, the under sequencing threshold can be 10 (or another threshold number) if the barcodes comprise at least, or at most, 1000, 2000, 3000, 4000, 5000, 6000, 6561, 7000, 8000, 9000, 10000, 20000, 30000, 40000, 50000, 60000, 65532, 70000, 80000, 90000, or 100000. In some embodiments, the under sequencing status may not depend on the number of molecular labels of the barcodes (e.g., stochastic barcodes) with distinct sequences.

At decision block 624, if the sequencing status of the target in the sequencing data is not the under sequencing status, the method 600 can proceed to block 628 to filter molecular label counts. Filtering molecular label counts can include, at decision block 632, determining the number of molecular labels with distinct sequences associated with the target in the sequencing data being less than a pseudopoints threshold. The pseudopoints threshold can be different in different implementations. For example, the pseudopoints threshold can be, or be about, 1, 2, 3, 4, 5, 6, 7, 8, 9, 10, 20, 30, 40, 50, 60, 70, 80, 90, 100, or a number or a range between any two of these values if the barcodes (e.g., stochastic barcodes) comprise about 6561 molecular labels with distinct sequences. As another example, the pseudopoints sequencing threshold can be at least, or at most, 1, 2, 3, 4, 5, 6, 7, 8, 9, 10, 20, 30, 40, 50, 60, 70, 80, 90, or 100, if the barcodes (e.g., stochastic barcodes) comprise about 6561 molecular labels with distinct sequences.

At decision block 632, if the number of molecular labels with distinct sequences associated with the target in the sequencing data is less than the pseudopoints threshold, the method 600 can optionally proceed to block 636, where pseudopoints can be added to the number of molecular labels with distinct sequences associated with the target in the sequencing data prior to determining the number of noise molecular labels with distinct sequences associated with the target in the sequencing data. The pseudopoints can have different molecular label counts in different implementations. For example, the molecular label count of a pseudopoint can be, or be about, 0.0001, 0.001, 0.01, 0.1, 1, 2, 3, 4, 5, 6, 7, 8, 9, 10, 20, 30, 40, 50, 60, 70, 80, 90, 100, or a number or a range between any two of these values. As another example, the molecular label count of a pseudopoint can be at least, or at most, 0.0001, 0.001, 0.01, 0.1, 1, 2, 3, 4, 5, 6, 7, 8, 9, 10, 20, 30, 40, 50, 60, 70, 80, 90, or 100.

At decision block 632, if the number of molecular labels with distinct sequences associated with the target in the sequencing data is not less than the pseudopoints threshold, non-unique molecular labels can be removed at block 640. The non-unique molecular labels can be removed by determining the number of noise molecular labels with distinct sequences associated with the target in the sequencing data at block 644. The non-unique molecular labels can include molecular labels with distinct sequences associated with the target in the sequencing data being greater than a predetermined recycled molecular label threshold. The recycled molecular label threshold can be different in different implementations. For example, the recycled molecular label threshold can be, or be about, 100, 200, 300, 400, 500, 600, 650, 700, 900, 1000, 2000, or a number or a range between any two of these values, if the barcodes (e.g., stochastic barcodes_ comprise about 6561 molecular labels with distinct sequences. As another example, the recycled molecular label threshold can be at least, or at most, 100, 200, 300, 400, 500, 600, 650, 700, 900, 1000, or 2000, if the barcodes (e.g., stochastic barcodes) comprise about 6561 molecular labels with distinct sequences.

In some embodiments, removing the non-unique molecular labels comprises: determining a theoretical number of non-unique molecular labels for the number of molecular labels with distinct sequences associated with the target in the sequencing data. Removing the non-unique molecular labels can comprise removing a molecular label with an occurrence greater than the nth most abundant molecular label of the molecular labels with distinct sequences associated with the target in the sequencing data. The number $n$ can be the theoretical number of non-unique molecular labels.

At block 644, the molecular label counts can be adjusted using a distribution-based error correction method. The distribution-based error correction method can include determining the number of noise molecular labels with distinct sequences associated with the target in the sequencing data. Determining the number of noise molecular labels can comprise: fitting two negative binomial distributions to the number of molecular labels with distinct sequences associated with the target in the sequencing data. For example, determining the number of noise molecular labels can comprise: fitting a signal negative binomial distribution (one of the two negative binomial distributions) to the number of molecular labels with distinct sequences associated with the target in the sequencing data counted, wherein the signal negative binomial distribution corresponds to a number of molecular labels with distinct sequences associated with the target in the sequencing data counted being signal molecular labels. Determining the number of noise molecular labels can comprise: fitting a noise negative binomial distribution (the other of the two negative binomial distributions) to the number of molecular labels with distinct sequences associated with the target in the sequencing data counted, wherein the noise negative binomial distribution corresponds to a number of molecular labels with distinct sequences associated with the target in the sequencing data counted being noise molecular labels. Determining the number of noise molecular labels can comprise determining the number of noise molecular labels using the signal negative binomial distribution fitted and the noise negative binomial distribution fitted.

In some embodiments, determining the number of noise molecular labels using the signal negative binomial distribution fitted and the noise negative binomial distribution fitted comprises, for each of the distinct sequences associated with the target in the sequencing data: determining a signal probability of the distinct sequence to be in the signal negative binomial distribution. And a noise probability of the distinct sequence to be in the noise negative binomial distribution can be determined. Furthermore, the distinct sequence can be determined to be a noise molecular label if the signal probability is smaller than the noise probability. In some embodiments, adjusting the molecular label counts at block 644 can include removing singletons (e.g., single base substitutions) if fewer than two peaks are found (because two peaks may be required to determine the signal negative binomial distribution and the noise negative binomial distribution).

At block 648, the number of the target can be estimated to generate output after adjacency-based and distribution-based error corrections. At decision block 612, if the sequencing status of the target in the sequencing data is the saturated sequencing status, the method 600 can proceed to block 648 to generate output without adjusting molecular labels based on directional adjacency and distribution-based error correction. For example, the number of noise molecular labels determined can be zero.

At decision block 624, if the sequencing status of the target in the sequencing data is the under sequencing status, the method 600 can proceed to block 648 to generate output without adjusting molecular labels based on distribution-based error correction. For example, the number of noise molecular labels determined can be zero. The method 600 ends at block 652.

Immune Receptor-Barcode Error Correction

Substitution errors, primer crossover errors, and PCR chimera errors can occur when performing sequencing and profiling, such as immune receptor sequencing and profiling. For example, errors can occur when determining the numbers of occurrences or copies of RNA molecules encoding immune receptors, such as T-cell receptors. Immune receptors include closely related genes that are highly diversified. Hence, when compared to other genes, the possibility of errors in sequencing data can be higher when performing immune receptor sequencing and profiling. The errors often lead to over-quantification of immune repertoire diversity. The methods for mitigating these errors are referred to herein as immune receptor-barcode error correction. In some embodiments, immune receptor-barcode error correction utilizes recursive substitution error correction to correct substitution errors in molecular labels and nucleotide sequences (e.g., substitution errors in the complementarity-determining region 3 (CDR3)). For a given sample label or cell label, many different CDR3s can be associated with the same molecular label sequence, leading to an overestimation of immune receptor diversity. The correction methods disclosed herein can correct for PCR chimeras that cross before molecular labeling and sample labeling followed by identifying and removing error molecular labels.

Disclosed herein include methods for determining occurrences of targets. In some embodiments, the method comprises: (a) barcoding (e.g., stochastically barcoding) a plurality of targets using a plurality of barcodes (e.g., stochastic barcodes) to create a plurality of barcoded targets (e.g., stochastically barcoded targets), wherein each of the plurality of barcodes comprises a cell label and a molecular label, wherein molecular labels of at least two barcodes of the plurality of barcodes comprise different molecular label sequences, and wherein at least two barcodes of the plurality of barcodes comprise cell labels with an identical cell label sequence; (b) obtaining sequencing data of the barcoded targets; and (c) for at least one target of the plurality of targets: (i) identifying putative sequences of the target in the sequencing data; (ii) counting occurrences of molecular label sequences associated with the putative sequences of the target in the sequencing data identified in (i); (iii) identifying clusters of the putative sequences of the target; (iv) collapsing the sequencing data obtained using the clusters of putative sequences of the target identified in (iii); (v) identifying clusters of the molecular label sequences associated with the putative sequences of the target; (vi) collapsing the sequencing data using the clusters of molecular label sequences identified in (v); (vii) identifying clusters of combination sequences, wherein each combination sequence comprises a sequence of the sequences of the target and an associated molecular label sequence of the molecular label sequences; (viii) collapsing the sequencing data using the clusters of combination sequences identified in (vii); (ix) identifying one or more putative sequences of the target that correspond to one or more chimeric sequences of the target, wherein occurrences of the one or more putative sequences of the target that correspond to the one or more chimeric sequences of the target are smaller than occurrences of remaining one or more putative sequences of the target that do not correspond to the one or more chimeric sequences of the target; (x) removing the one or more putative sequences of the target corresponding to the one or more chimeric sequences of the target identified in (ix) from the sequencing data; and (xi) estimating the occurrence of the target, wherein the occurrence of the target estimated correlates with the number of molecular label sequences counted in (ii) after collapsing the sequencing data in (iv), (vi), and (viii) and removing the one or more putative sequences of the target that correspond to the one or more chimeric sequences of the target in (x).

Disclosed herein are methods for determining occurrences of targets. In some embodiments, the method comprises: (a) receiving sequencing data of a plurality of targets, wherein the sequencing data comprises putative sequences of a target of the plurality of targets and occurrences of molecular label sequences associated with the sequences of the target in the sequencing data; (b) collapsing putative sequences of the target; (c) collapsing molecular label sequences associated with the putative sequences of the target; and (d) estimating the occurrence of the target, wherein the occurrence of the target estimated correlates with the occurrence of molecular label sequences associated with the putative sequences of the target in the sequencing data after collapsing the occurrence of the putative sequences of the target in (b) and the occurrence of noise molecular label sequences determined in (c).

The complementarity-determining regions (CDRs) are part of the variable chains in immunoglobulins (antibodies), generated by B-cells, and T cell receptors, generated by T-cells. There are three CDRs (CDR1, CDR2, and CDR3), arranged non-consecutively, on the amino acid sequence of a variable domain of an antigen receptor or an immune receptor. Since the antigen receptors are typically composed of two variable domains (on two different polypeptide chains, heavy and light chain), there are six CDRs, for each pair of heavy chain and light chain, that can collectively come into contact with the antigen. Since most sequence variation associated with immunoglobulins and T cell receptors are found in the CDRs, variation in the nucleotide sequence encoding the CDRs that results from one or more errors during sequencing and variation that exists in the nucleotide sequence encoding the CDRs cannot be easily distinguished. Within the variable domain, CDR1 and CDR2 are found in the variable (V) region of a polypeptide chain, and CDR3 includes some of the V region, and all of diversity (D) region and joining (J) region for a heavy chain. A light chain contains a V region and a J region, not a D region. CDR3 is the most variable of the CDRs.

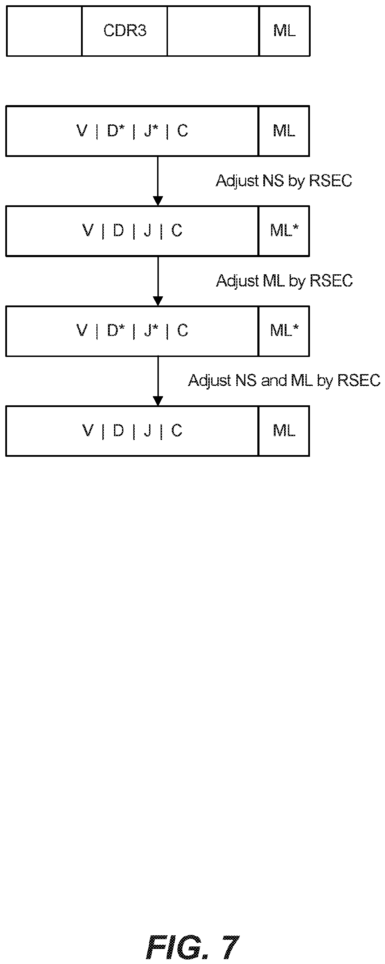

FIG. 7 is a schematic illustration showing a non-limiting exemplary embodiment of immune receptor-barcode correction based on recursive substitution error correction (RSEC, also referred to herein as directional adjacency). Barcodes (e.g., stochastic barcodes) comprising cell labels and molecular labels can be used to determine the sequences of mRNA molecules encoding immune receptors, or targets of interest generally. CDR3 includes some of the V region and all of the D region and J region. As illustrated, substitution errors during sample preparation and sequencing can occur in the D and J regions (indicated with an "*"). Although not illustrated, substitution errors can also occur in the V region. Additionally, sequencing errors can occur in the molecular labels (MLs, indicated with an "*"). Recursive Substitution Error Correction (RSEC) can be used to correct such errors. For example, RSEC can be used to first adjust counts of the CDR3 sequences. Subsequently, RSEC can be used to adjust counts of the molecular labels. Optionally, RSEC can be used to adjust counts of both the CDR3 sequences and the molecular labels simultaneously by considering each CDR3 sequence and the associated molecular label in the sequencing data as one sequence. In some embodiments, the counts of the molecular labels can be adjusted using RSEC first.

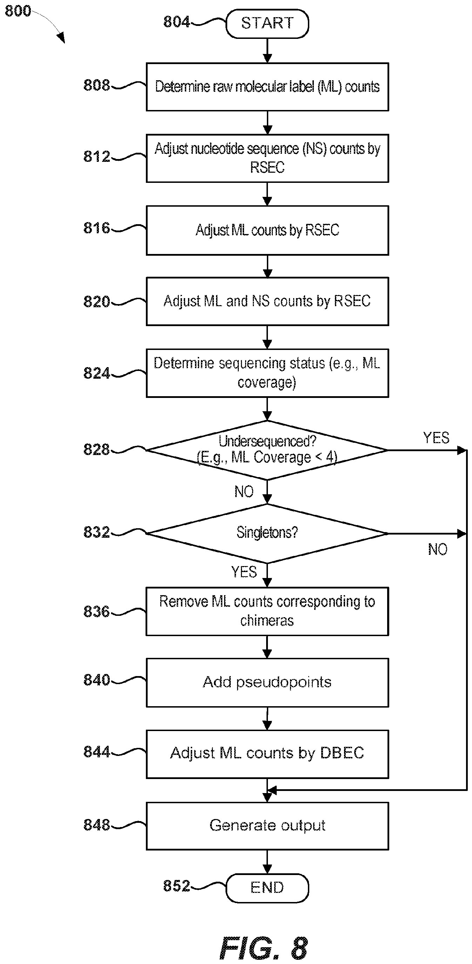

FIG. 8 is a flowchart showing a non-limiting exemplary embodiment 800 of correcting errors in nucleotide sequences and molecular labels based on recursive substitution error correction and correcting errors in sequencing data attributable to one or more PCR chimeras. The method 800 starts at block 804 after receiving sequencing data of a plurality of barcoded targets (e.g., stochastically barcoded targets). In some embodiments, the method 800 further comprises barcoding (e.g., stochastically barcoding) a plurality of targets using a plurality of barcodes (e.g., stochastic barcodes) to create the plurality of barcoded targets, wherein each of the plurality of barcodes comprises a molecular label and/or a cell label. In some embodiments, the method 800 further comprises sequencing the plurality of barcoded targets to obtain the sequencing data. The plurality of targets can include targets of the whole transcriptome of a cell, a gene (e.g., a gene encoding an immune receptor, such as a T-cell receptor), a variable sequence (e.g., a variable (V) region, a diversity (D) region, a joining (J) region encoding an immune receptor), or any combination thereof.

At block 808, the method 800 can include: for one or more of the plurality of targets, determining (e.g., counting) the number of molecular labels with distinct sequences associated with the target in the sequencing data. In some embodiments, counting the number of molecular labels with distinct sequences associated with the target in the sequencing data can include: identifying putative sequences of the target (e.g., an immune receptor sequence, such as the CDR3 sequence) in the sequencing data; and counting occurrences of molecular label sequences associated with the putative sequences of the target in the sequencing data identified.

The putative sequences of the target (e.g., CDR3) can differ from each other by one or more nucleotides. The sequences are putative in the sense that only one sequence is the real or correct sequence (e.g., there is only one correct CDR3 sequence per cell). Putative sequences of the target can differ from one another by at least one nucleotide.

At block 812, the method 800 can include adjusting the counts of the putative nucleotide sequences of the target of interest based on Recursive Substitution Error Correction (also referred to herein as directional adjacency). In some embodiments, adjusting the nucleotide sequence counts based on RSEC can be similar to adjusting the molecular label counts based on directional adjacency described with reference to FIG. 5. For example, adjusting the nucleotide sequence counts based on directional adjacency can include: identifying putative sequences of the target in the sequencing data; counting occurrences of molecular label sequences associated with the putative sequences of the target in the sequencing data identified; identifying clusters of the putative sequences of the target; and collapsing the sequencing data obtained using the clusters of putative sequences of the target identified. Identifying the clusters of the putative sequences of the target can comprise identifying the clusters of the putative sequences of the target using RSEC. Collapsing the sequencing data obtained using the clusters of putative sequences of the target identified can comprise: attributing an occurrence of a child sequence of the one or more children sequences to the parent sequence of the child sequence.

In some embodiments, the putative sequences of the target within a cluster can be within a first predetermined directional adjacency threshold of one another. The first predetermined directional adjacency threshold can be different in different implementations. In some embodiments, the first directional adjacency threshold can be a Hamming distance, or about, 1, 2, 3, 4, 5, 6, 7, 8, 9, 10, or a number or a range between any two of these values. In some embodiments, the first directional adjacency threshold can be a Hamming distance at least, or at most, 1, 2, 3, 4, 5, 6, 7, 8, 9, or 10. The putative sequences of the target within the cluster can comprise one or more parent sequences and one or more children sequences of the one or more parent sequences. A number of occurrence of the parent sequence can be greater than or equal to a first predetermined directional adjacency occurrence threshold. The first predetermined directional adjacency occurrence threshold can be different in different implementations. In some embodiments, the first predetermined directional adjacency occurrence threshold can be twice of a number of occurrence of a child sequence less one. In some embodiments, the first predetermined directional adjacency occurrence threshold can be, or be about 1.5 time, 2 times, 3 times, 4 times, 5 times, 6 times, 7 times, 8 times, 9 times, 10 times, or a number or a range between any two of these values, the occurrence of a child sequence. In some embodiments, the first predetermined directional adjacency occurrence threshold can be, at least or at most 1.5 time, 2 times, 3 times, 4 times, 5 times, 6 times, 7 times, 8 times, 9 times, or 10 times, the occurrence of a child sequence.

At block 816, the method 800 can include adjusting counts of the molecular labels based on RSEC. In some embodiments, adjusting the molecular label counts based on directional adjacency can be as described with reference to FIG. 5. For example, adjusting the molecular label counts based on directional adjacency can include: identifying clusters of the molecular label sequences associated with the putative sequences of the target; and collapsing the sequencing data using the clusters of molecular label sequences identified. Identifying the clusters of the molecular label sequences associated with the putative sequences of the target can comprise identifying the clusters of the molecular label sequences associated with the putative sequences of the target using directional adjacency. Molecular label sequences of the target within a cluster can be within a second predetermined directional adjacency threshold of one another. The second predetermined directional adjacency threshold can be different in different implementations. In some embodiments, the second directional adjacency threshold can be a Hamming distance, or about, 1, 2, 3, 4, 5, 6, 7, 8, 9, 10, or a number or a range between any two of these values. In some embodiments, the second directional adjacency threshold can be a Hamming distance at least, or at most, 1, 2, 3, 4, 5, 6, 7, 8, 9, or 10. The putative molecular label sequences of the target within the cluster can comprise one or more parent molecular label sequences and one or more children molecular label sequences of the one or more parent molecular label sequences. The occurrence of the parent molecular label sequence can be greater than or equal to a second predetermined directional adjacency occurrence threshold. The second predetermined directional adjacency occurrence threshold can be different in different implementations. In some embodiments, the second predetermined directional adjacency occurrence threshold is twice an occurrence of a child molecular label sequence less one. In some embodiments, the second predetermined directional adjacency occurrence threshold can be, or be about 1.5 time, 2 times, 3 times, 4 times, 5 times, 6 times, 7 times, 8 times, 9 times, 10 times, or a number or a range between any two of these values, the occurrence of a child sequence. In some embodiments, the second predetermined directional adjacency occurrence threshold can be, at least or at most 1.5 time, 2 times, 3 times, 4 times, 5 times, 6 times, 7 times, 8 times, 9 times, or 10 times, the occurrence of a child sequence. Collapsing the sequencing data using the clusters of molecular label sequences associated with the sequences of the target identified can comprise: attributing an occurrence of a child molecular label sequence of the one or more children molecular label sequences to the parent molecular label of the child molecular label sequence.

At block 820, the method 800 can include optionally adjusting counts of the nucleotide sequences and molecular labels at the same time based on RSEC. Adjusting counts of the nucleotide sequences and molecular labels at the same time based on directional adjacency can include identifying clusters of combination sequences, wherein each combination sequence comprises a sequence of the sequences of the target and an associated molecular label sequence of the molecular label sequences; and collapsing the sequencing data using the clusters of combination sequences identified. Identifying the clusters of combination sequences can comprise identifying clusters of combination sequences using directional adjacency. Combination sequences within a cluster can be within a third predetermined directional adjacency threshold of one another. In some embodiments, the third directional adjacency threshold can be a Hamming distance, or about, 1, 2, 3, 4, 5, 6, 7, 8, 9, 10, or a number or a range between any two of these values. In some embodiments, the third directional adjacency threshold can be a Hamming distance at least, or at most, 1, 2, 3, 4, 5, 6, 7, 8, 9, or 10. The combination sequences within the cluster can comprise one or more parent combination sequences and one or more children combination sequences of the one or more parent combination sequences, and wherein an occurrence of the parent combination sequence is greater than or equal to a third predetermined directional adjacency occurrence threshold. The third predetermined directional adjacency occurrence threshold can be different in different implementations. In some embodiments, the third predetermined directional adjacency occurrence threshold is twice an occurrence of a child molecular label sequence less one. In some embodiments, the third predetermined directional adjacency occurrence threshold can be, or be about 1.5 time, 2 times, 3 times, 4 times, 5 times, 6 times, 7 times, 8 times, 9 times, 10 times, or a number or a range between any two of these values, the occurrence of a child sequence. In some embodiments, the third predetermined directional adjacency occurrence threshold can be, at least or at most 1.5 time, 2 times, 3 times, 4 times, 5 times, 6 times, 7 times, 8 times, 9 times, or 10 times, the occurrence of a child sequence. Collapsing the sequencing data using the clusters of combination sequences identified in (vii) can comprise: attributing an occurrence of a child combination sequence of the one or more children combination sequences to the parent combination sequence of the child combination sequence.

At block 824, the sequencing status of the target in the sequencing data can optionally be determined. The sequencing status of the target in the sequencing data can include, or be, under sequencing. At decision block 828, whether the sequencing status of the target in the sequencing data is the under sequencing status can optionally be determined. For example, the target can be considered to have the under sequencing status if its depth (e.g., an average, a minimum, or a maximum depth) is smaller than, or smaller than about, 1, 2, 3, 4, 5, 6, 7, 8, 9, 10, 20, 30, 40, 50, 60, 70, 80, 90, 100, or a number or a range between any two of these. As another example, the target can be considered to have the under sequencing status if its depth is smaller than at least, or at most, 1, 2, 3, 4, 5, 6, 7, 8, 9, 10, 20, 30, 40, 50, 60, 70, 80, 90, or 100.

In some embodiments, the under sequencing status can be determined by the target having a depth (e.g., an average, a minimum, or a maximum depth) smaller than a predetermined under sequencing threshold. The under sequencing threshold can be different in different implementations. For example, the under sequencing threshold can be, or be about, 1, 2, 3, 4, 5, 6, 7, 8, 9, 10, 20, 30, 40, 50, 60, 70, 80, 90, 100, or a number or a range between any two of these values. As another example, the under sequencing threshold can be at least, or at most, 1, 2, 3, 4, 5, 6, 7, 8, 9, 10, 20, 30, 40, 50, 60, 70, 80, 90, or 100.

In some embodiments, the under sequencing status may depend on the number of molecular labels of the barcodes (e.g., stochastic barcodes) with distinct sequences. For example, the under sequencing threshold can be 10 (or another threshold number) if the barcodes comprise, or about, 1000, 2000, 3000, 4000, 5000, 6000, 6561, 7000, 8000, 9000, 10000, 20000, 30000, 40000, 50000, 60000, 65532, 70000, 80000, 90000, 100000, or a number or range between any two of these values, molecular labels with distinct sequences. As another example, the under sequencing threshold can be 10 (or another threshold number) if the barcodes comprise at least, or at most, 1000, 2000, 3000, 4000, 5000, 6000, 6561, 7000, 8000, 9000, 10000, 20000, 30000, 40000, 50000, 60000, 65532, 70000, 80000, 90000, or 100000. In some embodiments, the under sequencing status may not depend on the number of molecular labels of the barcodes (e.g., stochastic barcodes) with distinct sequences.

At decision block 828, if the sequencing status of the target in the sequencing data is not the under sequencing status, the method 800 can proceed to decision block 832 to determine whether there is any singleton (e.g., single base substitutions) in the nucleotide sequences and/or molecular labels remaining after adjustments at blocks 812, 816, and 820. If at least one singleton remains, the method 800 can proceed to block 836, wherein molecular label counts corresponding to chimeras can be removed.

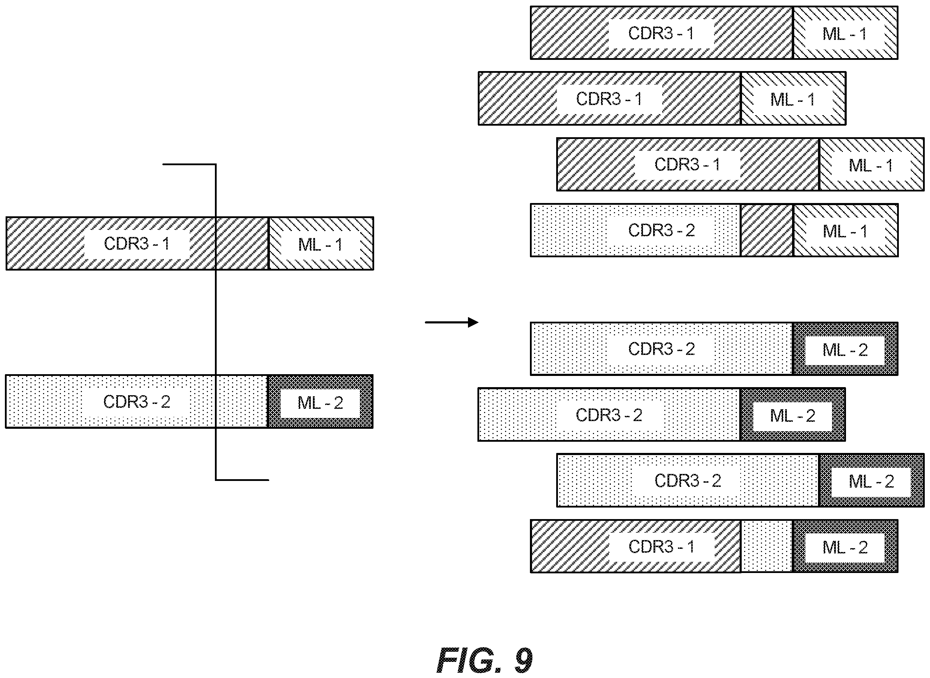

At block 836, the method 800 can include removing molecular label counts corresponding to chimeras. FIG. 9 is a schematic illustration of one possible origin of immune receptor chimeras (or target chimeras). As illustrated, many different CDR3 sequences (or putative sequences of a target) can have or be associated with the same ML (and sample label or cell label), which can result in overestimated TCR diversity. Two or more real CDR3 sequences can cross during PCR. For example, two real CDR3 sequences, labeled CDR3-1 and CDR3-2 in FIG. 9, can be associated with two different molecular labels, labeled ML-1 and ML-2, after barcoding (e.g., after block 224 in FIG. 2). Two (or more) real CDR3 sequences can be two different CDR3 sequences from two different cells not reslted from PCR cross over. Immune receptor chimeras can form during PCR (e.g., at block 228 in FIG. 2) such that multiple different CDR3 sequences (e.g., two different CDR3 sequences) can have the same ML. For example, ML-1 can be associated with CDR3-1 and a chimeric of CDR3-1 and CDR3-2. As another example, ML-2 can be associated with CDR3-2. and a chimeric of CDR3-1 and CDR3-2. It can be advantageous to remove molecular label counts corresponding to chimeras.

Referring to FIG. 8, removing molecular label counts corresponding to chimeras can include identifying one or more putative sequences of the target that correspond to one or more chimeric sequences of the target, wherein occurrences of the one or more putative sequences of the target that correspond to the one or more chimeric sequences of the target are smaller than occurrences of remaining one or more putative sequences of the target that do not correspond to the one or more chimeric sequences of the target; and removing the one or more putative sequences of the target corresponding to the one or more chimeric sequences of the target identified in the sequencing data. Identifying the one or more putative sequences of the target corresponding to the one or more chimeric sequences of the target can include: identifying putative sequences of the target associated with one molecular label sequence of the plurality of molecular sequences; and identifying a putative sequence of the putative sequences of the target associated with the one molecular label sequence with an occurrence smaller than a chimeric occurrence threshold as corresponding to a chimeric sequence of the one or more chimeric sequences of the target. The chimeric occurrence threshold can be different in different implementations. In some embodiments, the chimeric occurrence threshold can be an occurrence of a putative sequence of the putative sequences of the target associated with the one molecular label sequence that is greater than an occurrence of any other sequence of the putative sequences of the target. In some embodiments, the chimeric occurrence threshold can be an occurrence of a putative sequence of the putative sequences of the target associated with the one molecular label sequence that is greater than an occurrence of any other sequence of the putative sequences of the target adjusted (e.g., subtracting) by an offset. The offset can be different in different implementations. In some embodiments, the offset can be, or be about, 1, 2, 3, 4, 5, 6, 7, 8, 9, 10, 20, 30, 40, 50, 60, 70, 80, 90, 100, or a number or a range between any two of these values. In some embodiments, the offset can be at least, or at most, 1, 2, 3, 4, 5, 6, 7, 8, 9, 10, 20, 30, 40, 50, 60, 70, 80, 90, or 100.

The occurrence of the target estimated after block 824 correlates with the number of molecular label sequences counted at block 808 after collapsing the sequencing data at blocks 812, 816, and 820. The occurrence of the target estimated after block 836 correlates with the number of molecular label sequences counted at block 808 after collapsing the sequencing data at blocks 812, 816, and 820 and removing the one or more putative sequences of the target that correspond to the one or more chimeric sequences of the target at block 836.

At block 840, pseudopoints can be optionally added to the number of molecular labels with distinct sequences associated with the target in the sequencing data prior to determining the number of noise molecular labels with distinct sequences associated with the target in the sequencing data. For example, if the number of molecular labels with distinct sequences associated with the target in the sequencing data is less than a pseudopoints threshold, the method 800 can include adding pseudopoints to the number of molecular labels with distinct sequences associated with the target in the sequencing data prior to determining the number of noise molecular labels with distinct sequences associated with the target in the sequencing data. The pseudopoints threshold can be different in different implementations. For example, the pseudopoints threshold can be, or be about, 1, 2, 3, 4, 5, 6, 7, 8, 9, 10, 20, 30, 40, 50, 60, 70, 80, 90, 100, or a number or a range between any two of these values if the barcodes (e.g., stochastic barcodes) comprise about 6561 molecular labels with distinct sequences. As another example, the pseudopoints sequencing threshold can be at least, or at most, 1, 2, 3, 4, 5, 6, 7, 8, 9, 10, 20, 30, 40, 50, 60, 70, 80, 90, or 100, if the barcodes (e.g., stochastic barcodes) comprise about 6561 molecular labels with distinct sequences. The pseudopoints added can have different molecular label counts in different implementations. For example, the molecular label count of a pseudopoint can be, or be about, 0.0001, 0.001, 0.01, 0.1, 1, 2, 3, 4, 5, 6, 7, 8, 9, 10, 20, 30, 40, 50, 60, 70, 80, 90, 100, or a number or a range between any two of these values. As another example, the molecular label count of a pseudopoint can be at least, or at most, 0.0001, 0.001, 0.01, 0.1, 1, 2, 3, 4, 5, 6, 7, 8, 9, 10, 20, 30, 40, 50, 60, 70, 80, 90, or 100.

At block 844, the molecular label counts can be adjusted using a distribution-based error correction method. Performing the distribution-based error correction method can include determining the number of noise molecular labels with distinct sequences associated with the target in the sequencing data. Determining the number of noise molecular labels can comprise: fitting two distributions (such as two negative binomial distributions) to the number of molecular labels with distinct sequences associated with the target in the sequencing data. For example, determining the number of noise molecular labels can comprise: fitting a signal negative binomial distribution (one of the two negative binomial distributions) to the number of molecular labels with distinct sequences associated with the target in the sequencing data counted, wherein the signal negative binomial distribution corresponds to a number of molecular labels with distinct sequences associated with the target in the sequencing data counted being signal molecular labels. Determining the number of noise molecular labels can comprise: fitting a noise negative binomial distribution (the other of the two negative binomial distributions) to the number of molecular labels with distinct sequences associated with the target in the sequencing data counted, wherein the noise negative binomial distribution corresponds to a number of molecular labels with distinct sequences associated with the target in the sequencing data counted being noise molecular labels. Determining the number of noise molecular labels can comprise determining the number of noise molecular labels using the signal negative binomial distribution fitted and the noise negative binomial distribution fitted.

In some embodiments, determining the number of noise molecular labels using the signal negative binomial distribution fitted and the noise negative binomial distribution fitted comprises, for each of the distinct sequences associated with the target in the sequencing data: determining a signal probability of the distinct sequence to be in the signal negative binomial distribution. And a noise probability of the distinct sequence to be in the noise negative binomial distribution can be determined. Furthermore, the distinct sequence can be determined to be a noise molecular label if the signal probability is smaller than the noise probability. In some embodiments, adjusting the molecular label counts at block 644 can include removing singletons (e.g., single base substitutions) if fewer than two peaks are found (because two peaks may be required to determine the signal negative binomial distribution and the noise negative binomial distribution).

Performing the distribution-based error correction after collapsing the sequencing data at blocks 812, 816, and 820 and removing the one or more putative sequences of the target that correspond to the one or more chimeric sequences of the target at block 836 can include: thresholding molecular label sequences associated with the putative sequences of the target to determine signal molecular label sequences and noise molecular label sequences associated with the sequences of the target in the sequencing data counted at block 808 after collapsing the sequencing data at blocks 812, 816, and 820 and removing the one or more putative sequences of the target corresponding to the one or more chimeric sequences of the target at block 836. Thresholding the molecular label sequences associated with the putative sequences of the target can comprise performing a statistical analysis on the molecular label sequences of the target. Performing the statistical analysis can comprise: fitting the molecular label sequences associated with the putative sequences of the target and their occurrences to two negative binomial distributions; determining an occurrence of signal molecular label sequences n using the two negative binomial distributions; and removing the noise molecular label sequences from the sequencing data obtained in (b) after collapsing the sequencing data in (iv), (vi), and (viii) and removing the one or more putative sequences of the target that correspond to the one or more chimeric sequences of the target in (x), wherein the noise molecular label sequences comprise molecular label sequences with occurrences lower than an occurrence of the nth most abundant molecular label, and wherein the signal molecular label sequences comprise molecular label sequences with occurrences greater than or equal to the occurrence of the nth most abundant molecular label. The two negative binomial distributions can comprise a first negative binomial distribution corresponding for the signal molecular label sequences and a second negative binomial distribution for the noise molecular label sequences.

At block 848, the number of the target can be estimated to generate output after adjacency-based and distribution-based error corrections. At decision block 828, if the sequencing status of the target in the sequencing data is the under sequencing status, the method 800 can proceed to block 848 to generate output without adjusting molecular labels based on distribution-based error correction. For example, the number of noise molecular labels can be zero. At decision block 832, if no singletons remain in the adjusted sequencing data, the method 800 can proceed to block 848 to generate output without adjusting molecular labels based on distribution-based error correction. The method 800 ends at block 852.

Sequencing

In some embodiments, estimating the number of different labeled or barcoded targets (e.g., stochastically barcoded targets) can comprise determining the sequences of the labeled targets, the spatial label, the molecular label, the sample label, the cell label, or any product thereof (e.g. labeled-amplicons, or labeled-cDNA molecules). An amplified target can be subjected to sequencing. Determining the sequence of the barcoded target (e.g., stochastically barcoded target) or any product thereof can comprise conducting a sequencing reaction to determine the sequence of at least a portion of a sample label, a spatial label, a cell label, a molecular label, at least a portion of the barcoded target, a complement thereof, a reverse complement thereof, or any combination thereof.

Determination of the sequence of a barcoded target or a stochastically barcoded target (e.g. amplified nucleic acid, labeled nucleic acid, cDNA copy of a labeled nucleic acid, etc.) can be performed using variety of sequencing methods including, but not limited to, sequencing by hybridization (SBH), sequencing by ligation (SBL), quantitative incremental fluorescent nucleotide addition sequencing (QIFNAS), stepwise ligation and cleavage, fluorescence resonance energy transfer (FRET), molecular beacons, TaqMan reporter probe digestion, pyrosequencing, fluorescent in situ sequencing (FISSEQ), FISSEQ beads, wobble sequencing, multiplex sequencing, polymerized colony (POLONY) sequencing; nanogrid rolling circle sequencing (ROLONY), allele-specific oligo ligation assays (e.g., oligo ligation assay (OLA), single template molecule OLA using a ligated linear probe and a rolling circle amplification (RCA) readout, ligated padlock probes, or single template molecule OLA using a ligated circular padlock probe and a rolling circle amplification (RCA) readout), and the like.

In some embodiments, determining the sequence of the barcoded target (e.g., a stochastically barcoded target) or any product thereof comprises paired-end sequencing, nanopore sequencing, high-throughput sequencing, shotgun sequencing, dye-terminator sequencing, multiple-primer DNA sequencing, primer walking, Sanger dideoxy sequencing, Maxim-Gilbert sequencing, pyrosequencing, true single molecule sequencing, or any combination thereof. Alternatively, the sequence of the barcoded target or any product thereof can be determined by electron microscopy or a chemical-sensitive field effect transistor (chemFET) array.

High-throughput sequencing methods, such as cyclic array sequencing using platforms such as Roche 454, Illumina Solexa, ABI-SOLiD, ION Torrent, Complete Genomics, Pacific Bioscience, Helicos, or the Polonator platform, can also be utilized. In some embodiment, sequencing can comprise MiSeq sequencing. In some embodiment, sequencing can comprise HiSeq sequencing.

The barcoded targets (e.g., stochastically barcoded targets can comprise nucleic acids representing from about 0.01% of the genes of an organism's genome to about 100% of the genes of an organism's genome. For example, about 0.01% of the genes of an organism's genome to about 100% of the genes of an organism's genome can be sequenced using a target complimentary region comprising a plurality of multimers by capturing the genes containing a complimentary sequence from the sample. In some embodiments, the barcoded targets comprise nucleic acids representing from about 0.01% of the transcripts of an organism's transcriptome to about 100% of the transcripts of an organism's transcriptome. For example, about 0.501% of the transcripts of an organism's transcriptome to about 100% of the transcripts of an organism's transcriptome can be sequenced using a target complimentary region comprising a poly(T) tail by capturing the mRNAs from the sample.

Determining the sequences of the spatial labels and the molecular labels of the plurality of the barcodes (e.g., stochastic barcodes) can include sequencing 0.00001%, 0.0001%, 0.001%, 0.01%, 0.1%, 1%, 2%, 3%, 4%, 5%, 6%, 7%, 8%, 9%, 10%, 20%, 30%, 40%, 50%, 60%, 70%, 80%, 90%, 99%, 100%, or a number or a range between any two of these values, of the plurality of barcodes. Determining the sequences of the labels of the plurality of barcodes, for example the sample labels, the spatial labels, and the molecular labels, can include sequencing 1, 10, 20, 30, 40, 50, 60, 70, 80, 90, 100, $10^3$, $10^4$, $10^5$, $10^6$, $10^7$, $10^8$, $10^9$, $10^{10}$, $10^{11}$, $10^{12}$, $10^{13}$, $10^{14}$, $10^{15}$, $10^{16}$, $10^{17}$, $10^{18}$, $10^{19}$, $10^{20}$, or a number or a range between any two of these values, of the plurality of barcodes. Sequencing some or all of the plurality of barcodes can include generating sequences with read lengths of, of about, of at least, or of at most, 10, 20, 30, 40, 50, 60, 70, 80, 90, 100, 200, 300, 400, 500, 600, 700, 800, 900, 1000, 2000, 3000, 4000, 5000, 6000, 7000, 8000, 9000, 10000, or a number or a range between any two of these values, of nucleotides or bases.

Sequencing can comprise sequencing at least or at least about 10, 20, 30, 40, 50, 60, 70, 80, 90, 100 or more nucleotides or base pairs of the barcoded targets (e.g., stochastically barcoded targets). For example, sequencing can comprise generating sequencing data with sequences with read lengths of 50, 75, or 100, or more nucleotides by performing polymerase chain reaction (PCR) amplification on the plurality of barcoded targets. Sequencing can comprise sequencing at least or at least about 200, 300, 400, 500, 600, 700, 800, 900, 1,000 or more nucleotides or base pairs of the barcoded targets. Sequencing can comprise sequencing at least or at least about 1500, 2000, 3000, 4000, 5000, 6000, 7000, 8000, 9000, or 10000 or more nucleotides or base pairs of the barcoded targets.

Sequencing can comprise at least about 200, 300, 400, 500, 600, 700, 800, 900, 1,000 or more sequencing reads per run. In some embodiments, sequencing comprises sequencing at least or at least about 1500, 2000, 3000, 4000, 5000, 6000, 7000, 8000, 9000, or 10000 or more sequencing reads per run. Sequencing can comprise less than or equal to about 1,600,000,000 sequencing reads per run. Sequencing can comprise less than or equal to about 200,000,000 reads per run.

Samples

In some embodiments, the plurality of targets can be comprised in one or more samples. A sample can comprise one or more cells, or nucleic acids from one or more cells. A sample can be a single cell or nucleic acids from a single cell. The one or more cells can be of one or more cell types. At least one of the one or more cell types can be brain cell, heart cell, cancer cell, circulating tumor cell, organ cell, epithelial cell, metastatic cell, benign cell, primary cell, circulatory cell, or any combination thereof.

A sample for use in the method of the disclosure can comprise one or more cells. A sample can refer to one or more cells. In some embodiments, the plurality of cells can include one or more cell types. At least one of the one or more cell types can be brain cell, heart cell, cancer cell, circulating tumor cell, organ cell, epithelial cell, metastatic cell, benign cell, primary cell, circulatory cell, or any combination thereof. In some embodiments, the cells are cancer cells excised from a cancerous tissue, for example, breast cancer, lung cancer, colon cancer, prostate cancer, ovarian cancer, pancreatic cancer, brain cancer, melanoma and non-melanoma skin cancers, and the like. In some embodiments, the cells are derived from a cancer but collected from a bodily fluid (e.g. circulating tumor cells). Non-limiting examples of cancers can include, adenoma, adenocarcinoma, squamous cell carcinoma, basal cell carcinoma, small cell carcinoma, large cell undifferentiated carcinoma, chondrosarcoma, and fibrosarcoma. The sample can include a tissue, a cell monolayer, fixed cells, a tissue section, or any combination thereof. The sample can include a biological sample, a clinical sample, an environmental sample, a biological fluid, a tissue, or a cell from a subject. The sample can be obtained from a human, a mammal, a dog, a rat, a mouse, a fish, a fly, a worm, a plant, a fungus, a bacterium, a virus, a vertebrate, or an invertebrate.

In some embodiments, the cells are cells that have been infected with virus and contain viral oligonucleotides. In some embodiments, the viral infection can be caused by a virus such as single-stranded (+ strand or "sense") DNA viruses (e.g. parvoviruses), or double-stranded RNA viruses (e.g. reoviruses). In some embodiments, the cells are bacteria. These can include either gram-positive or gram-negative bacteria. In some embodiments, the cells are fungi. In some embodiments, the cells are protozoans or other parasites.

As used herein, the term "cell" can refer to one or more cells. In some embodiments, the cells are normal cells, for example, human cells in different stages of development, or human cells from different organs or tissue types. In some embodiments, the cells are non-human cells, for example, other types of mammalian cells (e.g. mouse, rat, pig, dog, cow, or horse). In some embodiments, the cells are other types of animal or plant cells. In other embodiments, the cells can be any prokaryotic or eukaryotic cells.

Data Analysis and Display Software

*Data Analysis* and *Visualization of Spatial Resolution of Targets*

The disclosure provides for methods for estimating the number and position of targets with barcoding (e.g., stochastic barcoding) and digital counting using spatial labels. The data obtained from the methods of the disclosure can be visualized on a map. A map of the number and location of targets from a sample can be constructed using information generated using the methods described herein. The map can be used to locate a physical location of a target. The map can be used to identify the location of multiple targets. The multiple targets can be the same species of target, or the multiple targets can be multiple different targets. For example a map of a brain can be constructed to show the digital count and location of multiple targets.

The map can be generated from data from a single sample. The map can be constructed using data from multiple samples, thereby generating a combined map. The map can be constructed with data from tens, hundreds, and/or thousands of samples. A map constructed from multiple samples can show a distribution of digital counts of targets associated with regions common to the multiple samples. For example, replicated assays can be displayed on the same map. At least 1, 2, 3, 4, 5, 6, 7, 8, 9, or 10 or more replicates can be displayed (e.g., overlaid) on the same map. At most 1, 2, 3, 4, 5, 6, 7, 8, 9, or 10 or more replicates can be displayed (e.g., overlaid) on the same map. The spatial distribution and number of targets can be represented by a variety of statistics.

Combining data from multiple samples can increase the locational resolution of the combined map. The orientation of multiple samples can be registered by common landmarks, wherein the individual locational measurements across samples are at least in part non-contiguous. A particular example is sectioning a sample using a microtome on one axis and then sectioning a second sample along a different access. The combined dataset will give three dimensional spatial locations associated with digital counts of targets. Multiplexing the above approach will allow for high resolution three dimensional maps of digital counting statistics.

In some embodiments of the instrument system, the system will comprise computer-readable media that includes code for providing data analysis for the sequence datasets generated by performing single cell, barcoding assays (e.g., stochastic barcoding assays). Examples of data analysis functionality that can be provided by the data analysis software include, but are not limited to, (i) algorithms for decoding/demultiplexing of the sample label, cell label, spatial label, and molecular label, and target sequence data provided by sequencing the barcode library (e.g., stochastic barcode library) created in running the assay, (ii) algorithms for determining the number of reads per gene per cell, and the number of unique transcript molecules per gene per cell, based on the data, and creating summary tables, (iii) statistical analysis of the sequence data, e.g. for clustering of cells by gene expression data, or for predicting confidence intervals for determinations of the number of transcript molecules per gene per cell, etc., (iv) algorithms for identifying sub-populations of rare cells, for example, using principal component analysis, hierarchical clustering, k-mean clustering, self-organizing maps, neural networks etc., (v) sequence alignment capabilities for alignment of gene sequence data with known reference sequences and detection of mutation, polymorphic markers and splice variants, and (vi) automated clustering of molecular labels to compensate for amplification or sequencing errors. In some embodiments, commercially-available software can be used to perform all or a portion of the data analysis, for example, the Seven Bridges (https://www.sbgenomics.com/) software can be used to compile tables of the number of copies of one or more genes occurring in each cell for the entire collection of cells. In some embodiments, the data analysis software can include options for outputting the sequencing results in useful graphical formats, e.g. heatmaps that indicate the number of copies of one or more genes occurring in each cell of a collection of cells. In some embodiments, the data analysis software can further comprise algorithms for extracting biological meaning from the sequencing results, for example, by correlating the number of copies of one or more genes occurring in each cell of a collection of cells with a type of cell, a type of rare cell, or a cell derived from a subject having a specific disease or condition. In some embodiment, the data analysis software can further comprise algorithms for comparing populations of cells across different biological samples.

In some embodiments all of the data analysis functionality can be packaged within a single software package. In some embodiments, the complete set of data analysis capabilities can comprise a suite of software packages. In some embodiments, the data analysis software can be a standalone package that is made available to users independently of the assay instrument system. In some embodiments, the software can be web-based, and can allow users to share data.

In some embodiments all of the data analysis functionality can be packaged within a single software package. In some embodiments, the complete set of data analysis capabilities can comprise a suite of software packages. In some embodiments, the data analysis software can be a standalone package that is made available to users independently of the assay instrument system. In some embodiments, the software can be web-based, and can allow users to share data.

*System Processors* and *Networks*

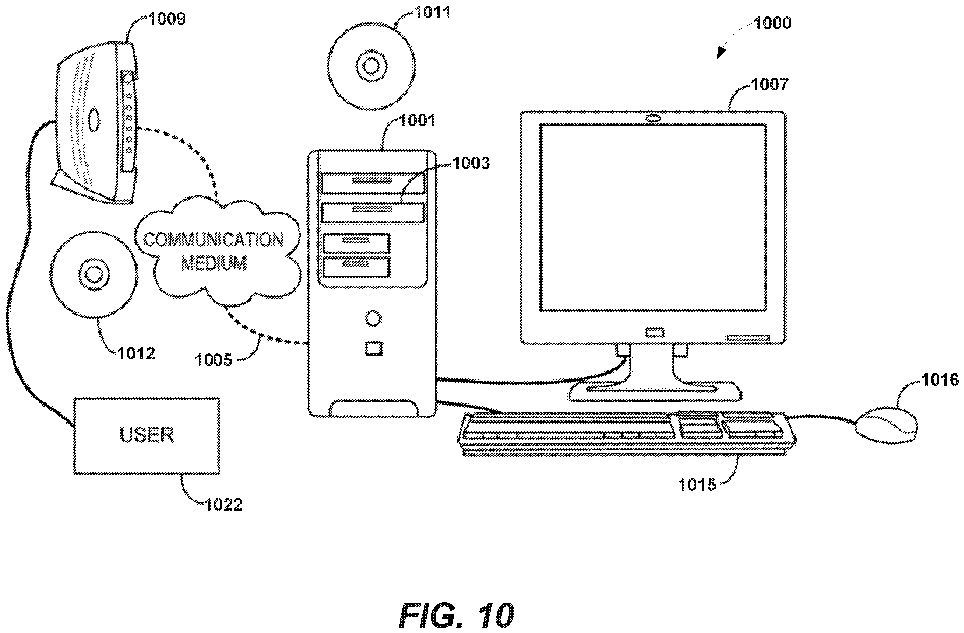

In general, the computer or processor suitable for use in the methods of the presently disclosed instrument systems, as illustrated in FIG. 10, can be further understood as a logical apparatus that can read instructions from media 1011 or a network port 1005, which can optionally be connected to server 1009 having fixed media 1012. The system 1000, such as shown in FIG. 10 can include a CPU 1001, disk drives 1003, optional input devices such as keyboard 1015 or mouse 1016 and optional monitor 1007. Data communication can be achieved through the indicated communication medium to a server at a local or a remote location. The communication medium can include any means of transmitting or receiving data. For example, the communication medium can be a network connection, a wireless connection or an internet connection. Such a connection can provide for communication over the World Wide Web. It is envisioned that data relating to the present disclosure can be transmitted over such networks or connections for reception or review by a party 1022 as illustrated in FIG. 10.

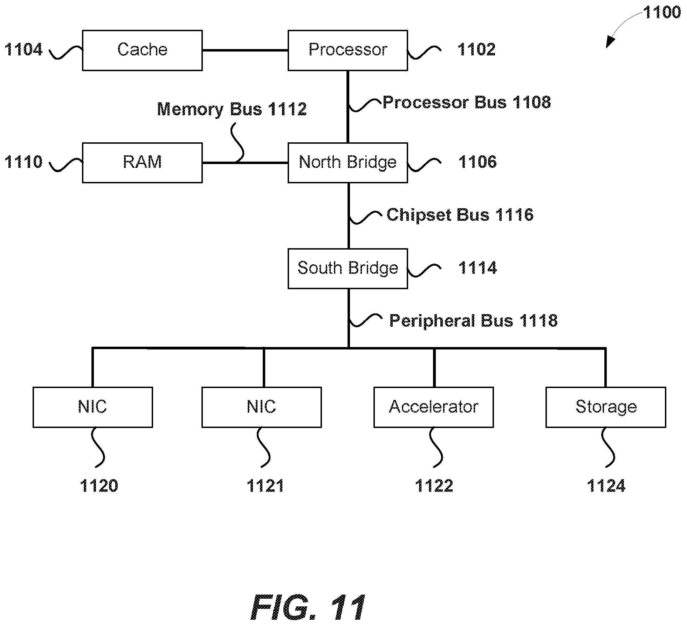

FIG. 11 illustrates an exemplary embodiment of a first example architecture of a computer system 1100 that can be used in connection with example embodiments of the present disclosure. As depicted in FIG. 11, the example computer system can include a processor 1102 for processing instructions. Non-limiting examples of processors include: Intel XeonTM processor, AMD Opteron™ processor, Samsung 32-bit RISC ARM 1176JZ(F)-S v1.0™ processor, ARM Cortex-A8 Samsung S5PC100TM processor, ARM Cortex-A8 Apple A4TM processor, Marvell PXA 930TM processor, or a functionally-equivalent processor. Multiple threads of execution can be used for parallel processing. In some embodiments, multiple processors or processors with multiple cores can also be used, whether in a single computer system, in a cluster, or distributed across systems over a network comprising a plurality of computers, cell phones, or personal data assistant devices.

As illustrated in FIG. 11, a high speed cache 1104 can be connected to, or incorporated in, the processor 1102 to provide a high speed memory for instructions or data that have been recently, or are frequently, used by processor 1102. The processor 1102 is connected to a north bridge 1106 by a processor bus 1108. The north bridge 1106 is connected to random access memory (RAM) 1110 by a memory bus 1112 and manages access to the RAM 1110 by the processor 1102. The north bridge 1106 is also connected to a south bridge 1114 by a chipset bus 1116. The south bridge 1114 is, in turn, connected to a peripheral bus 1118. The peripheral bus can be, for example, PCI, PCI-X, PCI Express, or other peripheral bus. The north bridge and south bridge are often referred to as a processor chipset and manage data transfer between the processor, RAM, and peripheral components on the peripheral bus 1118. In some alternative architectures, the functionality of the north bridge can be incorporated into the processor instead of using a separate north bridge chip.

In some embodiments, system 1100 can include an accelerator card 1122 attached to the peripheral bus 1118. The accelerator can include field programmable gate arrays (FPGAs) or other hardware for accelerating certain processing. For example, an accelerator can be used for adaptive data restructuring or to evaluate algebraic expressions used in extended set processing.

Software and data are stored in external storage 1124 and can be loaded into RAM 1110 or cache 1104 for use by the processor. The system 1100 includes an operating system for managing system resources; non-limiting examples of operating systems include: Linux, Windows™, MACOS™, BlackBerry OS™, iOS™, and other functionally-equivalent operating systems, as well as application software running on top of the operating system for managing data storage and optimization in accordance with example embodiments of the present invention.

In this example, system 1100 also includes network interface cards (NICs) 1120 and 1121 connected to the peripheral bus for providing network interfaces to external storage, such as Network Attached Storage (NAS) and other computer systems that can be used for distributed parallel processing.

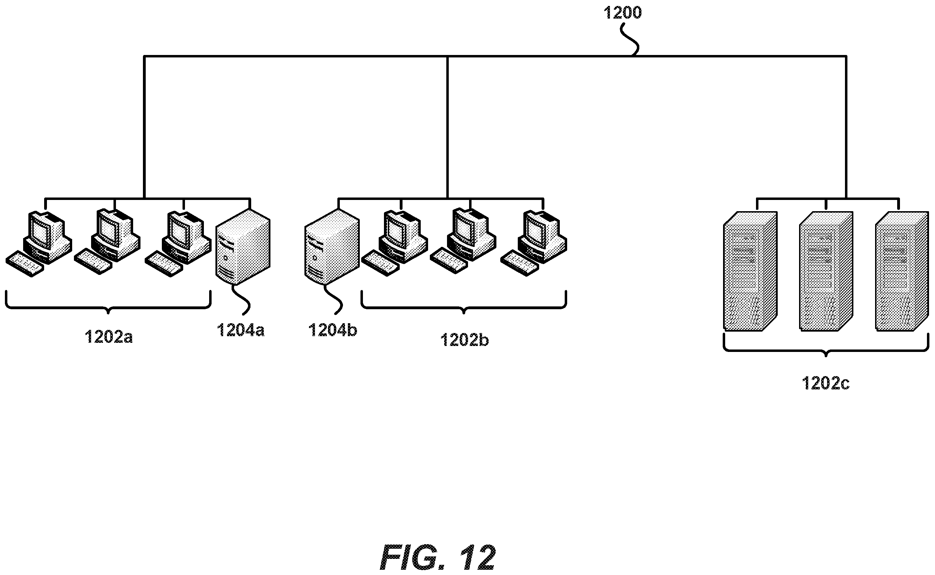

FIG. 12 illustrates an exemplary diagram showing a network 1200 with a plurality of computer systems 1202a, and 1202b, a plurality of cell phones and personal data assistants 1202c, and Network Attached Storage (NAS) 1204a, and 1204b suitable for use in the methods of the disclosure. In example embodiments, systems 1212a, 1212b, and 1212c can manage data storage and optimize data access for data stored in Network Attached Storage (NAS) 1214a and 1214b. A mathematical model can be used for the data and be evaluated using distributed parallel processing across computer systems 1212a, and 1212b, and cell phone and personal data assistant systems 1212c. Computer systems 1212a, and 1212b, and cell phone and personal data assistant systems 1212c can also provide parallel processing for adaptive data restructuring of the data stored in Network Attached Storage (NAS) 1214a and 1214b. FIG. 12 illustrates an example only, and a wide variety of other computer architectures and systems can be used in conjunction with the various embodiments of the present invention. For example, a blade server can be used to provide parallel processing. Processor blades can be connected through a back plane to provide parallel processing. Storage can also be connected to the back plane or as Network Attached Storage (NAS) through a separate network interface.

In some example embodiments, processors can maintain separate memory spaces and transmit data through network interfaces, back plane or other connectors for parallel processing by other processors. In other embodiments, some or all of the processors can use a shared virtual address memory space.

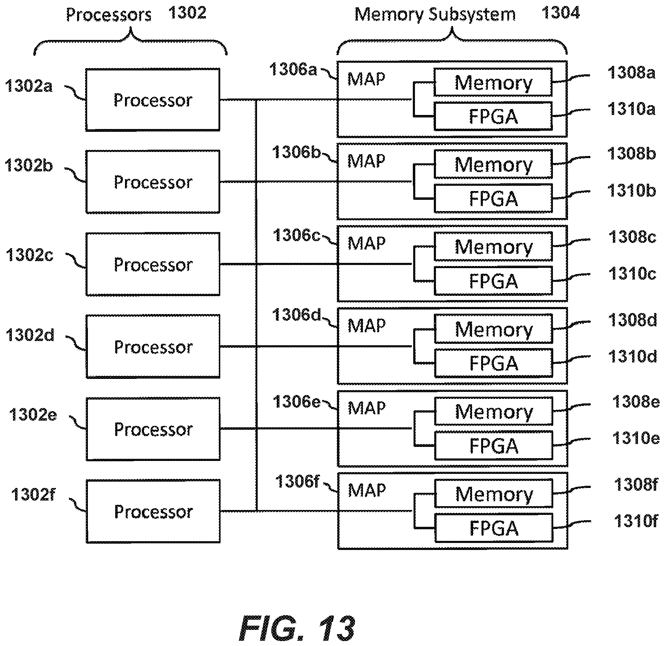

FIG. 13 illustrates an exemplary a block diagram of a multiprocessor computer system 1300 using a shared virtual address memory space in accordance with an example embodiment. The system includes a plurality of processors 1302a-f that can access a shared memory subsystem 1304. The system incorporates a plurality of programmable hardware memory algorithm processors (MAPs) 1306a-f in the memory subsystem 1304. Each MAP 1306a-f can comprise a memory 1308a-f and one or more field programmable gate arrays (FPGAs) 1310a-f. The MAP provides a configurable functional unit and particular algorithms or portions of algorithms can be provided to the FPGAs 1310a-f for processing in close coordination with a respective processor. For example, the MAPs can be used to evaluate algebraic expressions regarding the data model and to perform adaptive data restructuring in example embodiments. In this example, each MAP is globally accessible by all of the processors for these purposes. In one configuration, each MAP can use Direct Memory Access (DMA) to access an associated memory 1308a-f, allowing it to execute tasks independently of, and asynchronously from, the respective microprocessor 1302a-f. In this configuration, a MAP can feed results directly to another MAP for pipelining and parallel execution of algorithms.

The above computer architectures and systems are examples only, and a wide variety of other computer, cell phone, and personal data assistant architectures and systems can be used in connection with example embodiments, including systems using any combination of general processors, co-processors, FPGAs and other programmable logic devices, system on chips (SOCs), application specific integrated circuits (ASICs), and other processing and logic elements. In some embodiments, all or part of the computer system can be implemented in software or hardware. Any variety of data storage media can be used in connection with example embodiments, including random access memory, hard drives, flash memory, tape drives, disk arrays, Network Attached Storage (NAS) and other local or distributed data storage devices and systems.

In example embodiments, the computer subsystem of the present disclosure can be implemented using software modules executing on any of the above or other computer architectures and systems. In other embodiments, the functions of the system can be implemented partially or completely in firmware, programmable logic devices such as field programmable gate arrays (FPGAs), system on chips (SOLs), application specific integrated circuits (ASICs), or other processing and logic elements. For example, the Set Processor and Optimizer can be implemented with hardware acceleration through the use of a hardware accelerator card, such as accelerator card.

EXAMPLES

Some aspects of the embodiments discussed above are disclosed in further detail in the following examples, which are not in any way intended to limit the scope of the present disclosure.

Example 1

ML Coverage of each ML across a Plate for a High expressor gene - ACTB

This example demonstrates distinct distributions of ML errors derived during sequencing or PCR generally have distinct distributions from true MLs.

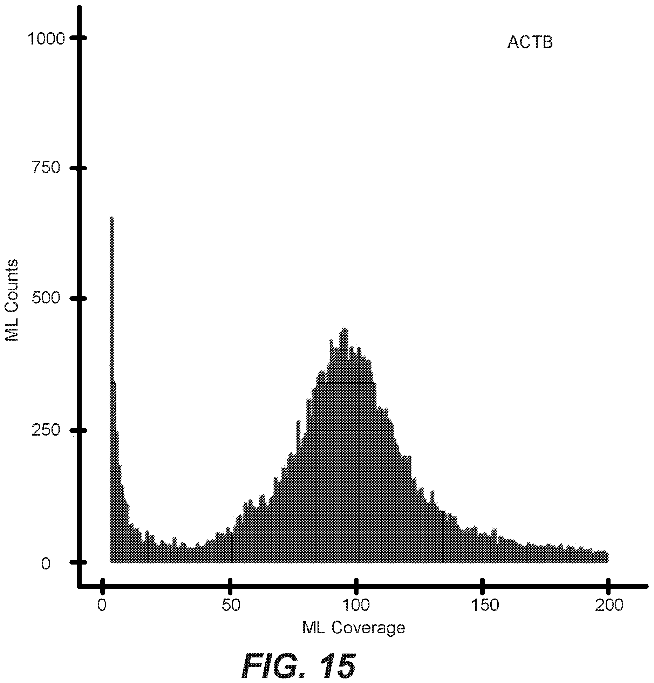

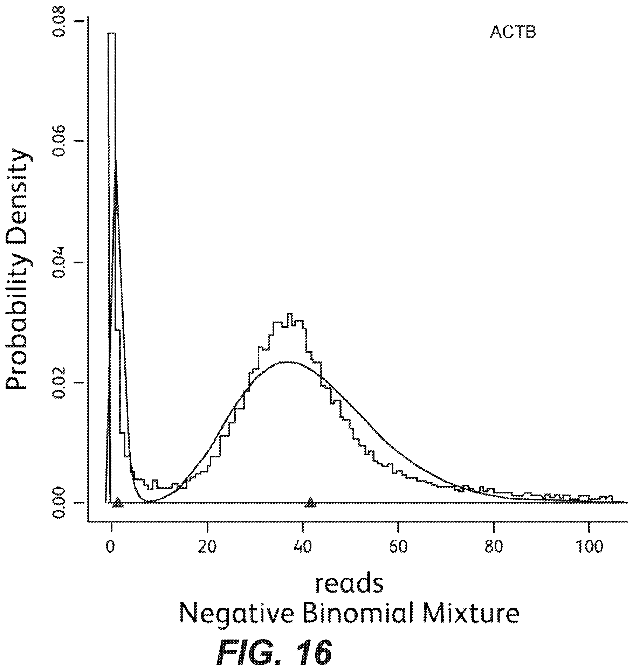

In addition to absolute gene expression counting and PCR bias correction, MLs can provide a better understanding of the statistical quality of the library preparation procedure and sequencing data. When looking at the number of reads presenting the same gene ML - referred to as ML coverage - it is possible to detect for sequencing erroneous base calls or PCR errors generated during library preparation. For example, a gene ML from a given SL that is represented by multiple reads is likely an accurate measurement compared to a gene ML from a given SL that is represented by only a single read. Low ML coverage barcodes in the presence of high ML coverage barcodes in the same library are often artifacts or errors generated during the sequencing run or PCR steps during library preparation. The ML errors derived during sequencing or PCR generally have distinct distributions from the true MLs. FIG. 15 is an exemplary plot showing molecular label coverage of each molecular label across a micro well plate for a high expressor gene - ATCB, where distinct distributions were observed between error molecular labels and real molecular labels. FIG. 16 is an exemplary plot showing fitting two negative binomial distributions to molecular label coverage of each molecular label across a microwell plate for a high expressor gene - ATCB. The fitting of two negative binomial distributions demonstrates that molecular label errors with lower molecular label depth and true molecular label with higher molecular label depth can be statistically distinguished. The $x$ axis is the molecular depth.

Altogether, these data demonstrate the ML errors derived during sequencing or PCR generally have distinct distributions from the true MLs.

Example 2

Correcting Molecular Labels Due to PCR or Sequencing Substitution Errors

This example demonstrates a method for correcting molecular labels due to PCR and sequencing substitution errors that can be applied to whole transcriptome assays without the assumption of uniform coverage and without requiring high sequencing coverage for the complete sequencing status.

Deduplication was performed on the first mapping coordinate and unique molecular labels (UMI) of each read, and reads were assumed to be identical given the same start coordinate, UML, and strand. After deduplication, UMLs with the highest counts per cluster were retained (Table 13).

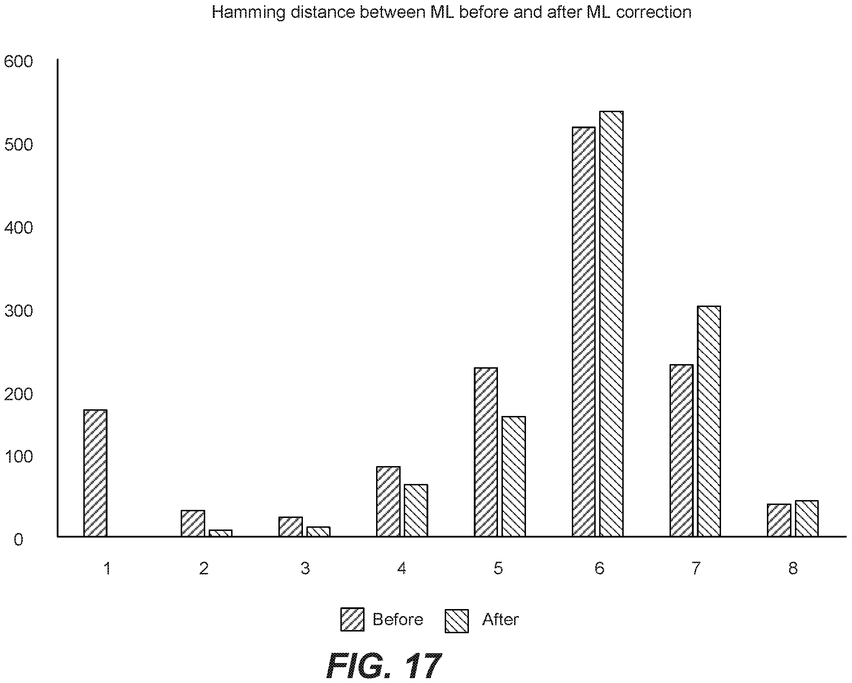

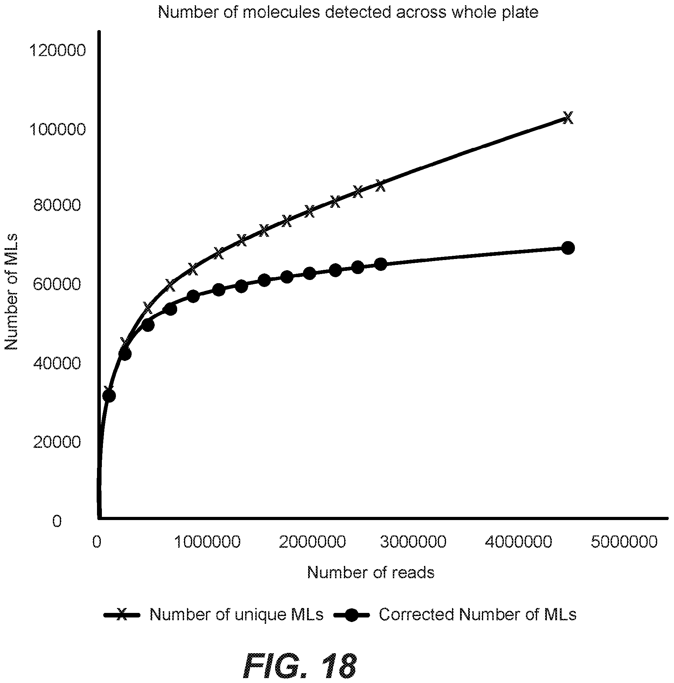

Molecular labels (MLs) were corrected on a per gene basis. For each gene, clusters of MLs were identified with directional adjacency. The directional adjacency method clustered MLs if the MLs were within a Hamming distance of 1 and a parent ML count $\geq 2*$ (child MI count) - l. All MLs within the same cluster were considered to originate from the same parent ML, and children ML counts were collapsed to the parent ML. FIG. 17 shows molecular label correction, where pairwise Hamming distance of 1 was overrepresented. After molecular label correction, molecular labels with Hamming distance of one apart were clustered and collapsed to the same parent molecular label. FIG. 18 shows that the curve of the corrected number of MLs vs. the number of reads converge. Because all the reads were retained, this method can also be used to remove one-base PCR or sequencing errors.

Table 13. After deduplicating molecular labels, only an insufficient number, given a whole transcriptome assay, of unique molecular labels were considered as errors

| | | Raw Counts | | Deduplicate UMLs | |
|---|---|---|---|---|---|
| Sample | Well | Number of Reads | Number of Unique MI | Number of Reads | Number of Unique MI |
| Plate 2 | A02 | 234646 | 3079 | 28925 | 2335 |
| Plate 3 | A02 | 773050 | 14126 | 95023 | 11410 |

Altogether, these data demonstrate a correction method which can be applied to correct or adjust data of whole transcriptome assays because all the reads were retrained.

Example 3

Molecular Label Counting for High Input Samples

This example describes unique molecular labels used as input molecules increase.

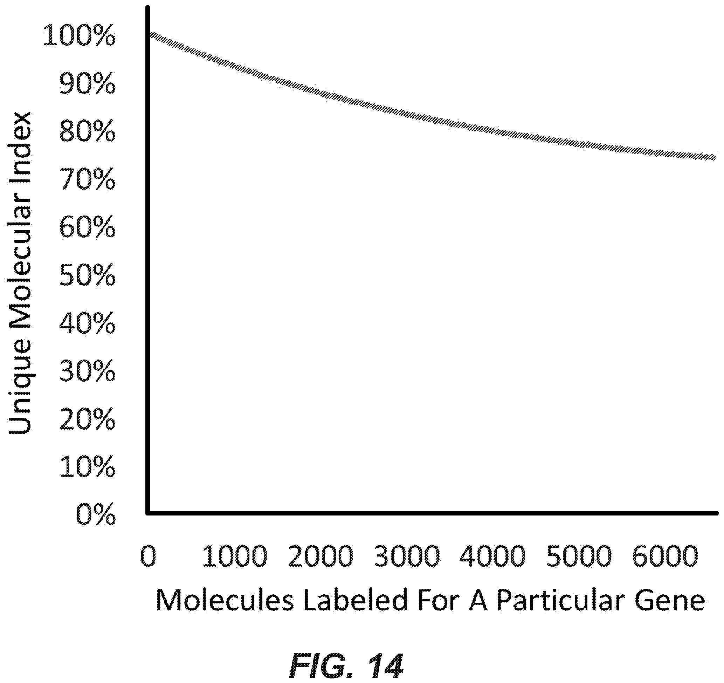

The BD Precise™ Targeted Assay may be the most suitable when used in small sample input - such as in single cells - to allow for stochastic and unique labeling of mRNAs. As the number of transcripts increases relative to the barcode pool in high RNA/cell input experiments, the percentage of MLs being recycled to label the same gene increases and was theoretically calculated using a Poisson distribution (FIG. 14). In these situations, without statistical correction, quantifying gene expression using MLs would underestimate the number of molecules that are initially present without any Poisson corrections or corrections based on two negative binomial distributions.

At extremely high input samples where the number of mRNAs per gene is beyond the entire collection of 6561 barcodes, a Poisson correction or a correction based on two negative binomial distributions is no longer possible. For example, regardless of 65000 or 100000 input molecules, a maximum of 6561 saturated barcodes is expected in either case. Hence, genes and samples that appear to have high sample input can be altered, whereby ML counts would likely be underestimated.

Altogether, these data demonstrate the need to adjust raw data when quantifying gene expression using MLs.

Example 4

Recursive Substitution Error Correction (RSEC)

This example demonstrates recursive substitution error correction.

Two collaborative methods can be employed in the BD Precise™ Targeted Assay analysis pipeline to remove ML errors. In brief, ML errors that are derived from sequencing base calls substitution errors are identified and adjusted to the true ML barcode using Recursive Substitution Error Correction (RSEC). Subsequently, ML errors that are derived from library preparation steps or sequencing base deletion errors are adjusted using Distribution-Based Error Correction (DBEC).

The RSEC algorithm can adjust ML errors that are derived from PCR or sequencing substitution. These rare erroneous events have been observed when examining the ML coverage. For example, the ML coverage for error MLs can be significantly lower than true MLs in adequately sequenced samples (FIG. 15); in cases where two very similar MLs are used during the initial Molecular Indexing™ (reverse transcription) steps, they would generally have similar ML coverage and do not need to be eliminated. As sequencing depth increases, more ML errors appear, hence RSEC can be crucial for adjusting the ML count for highly sequenced barcoded libraries.

In brief, RSEC considers two factors in error correction: 1) Similarity in ML sequence; and 2) and their ML coverage. For each target gene, MLs are connected when both of their ML sequence is within 1 base (Hamming distance = 1) with each other. For each connection between ML *x* and y, if:

$$Coverage(y) > 2*Coverage(x)+1, \qquad Equation\ (5)$$

where y denotes "Parent ML," and x denotes "Child ML."

Based on this assignment, child MLs can be collapsed to their parent ML. This process is recursive until there are no more identifiable parent/child MLs for the gene.

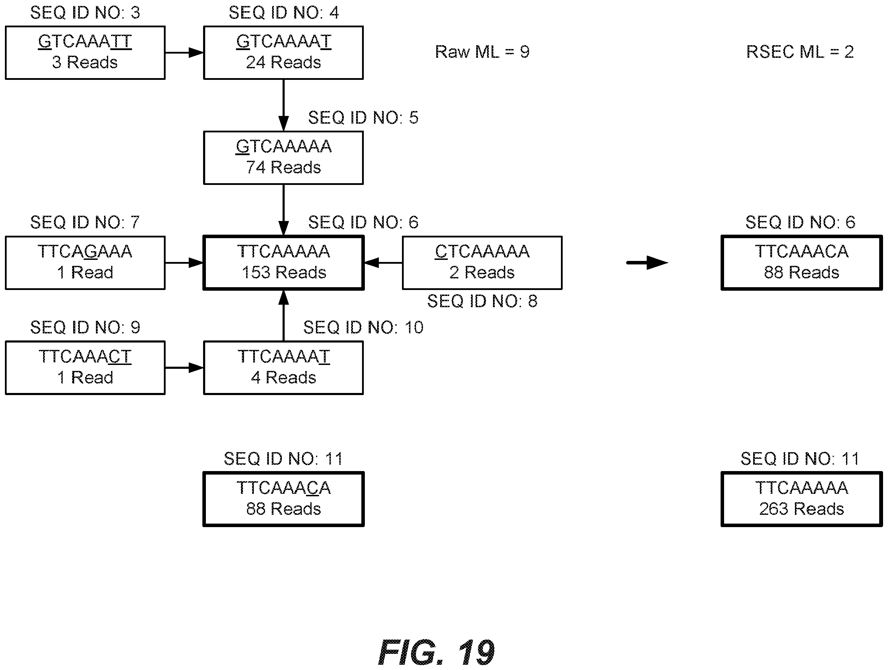

FIG. 19 shows a schematic illustration of an example of recursive substitution error correction outline above. The MLs in the raw data prior to the RSEC correction includes nine unique MLs: GTCAAATT, GTCAAAAT, GTCAAAAA, TTCAAAAA, TTCAGAAA, CTCAAAAA, TTCAAACT, TTCAAAAT, and TTCAAACA (SEQ ID NO: 3-11). By applying RSEC, GTCAAATT (SEQ ID NO: 3) can be collapsed into GTCAAAAT (SEQ ID NO: 4) because the two MLs differ by one nucleotide (underlined) and the ML GTCAAATT (SEQ ID NO: 3) has a lower ML count than that of GTCAAAAT (SEQ ID NO: 4). In turn, the ML GTCAAAAT (SEQ ID NO: 4) can be collapsed into the ML GTCAAAAA (SEQ ID NO: 5) (the difference in ML sequences is underlined), which has a higher ML count than that of GTCAAAAT (SEQ ID NO: 4). Similarly, the MLs TTCAGAAA (SEQ ID NO: 7) and CTCAAAAA (SEQ ID NO: 8) can be collapsed into the ML TTCAAAAA (SEQ ID NO: 6). The ML TTCAAACT (SEQ ID NO: 9) can be collapsed into the ML TTCAAAAT (SEQ ID NO: 10), which can be in turn collapsed into ML TTCAAAAA (SEQ ID NO: 6). The ML TTCAAACA (SEQ ID NO: 11) differs from all other MLs by more than one nucleotide, and thus is not collapsed into any of the other eight MLs. Before RSEC correction, the raw ML count number was nine. After RSEC correction, the ML count number was two: MLs TTCAAAAA (SEQ ID NO: 6) and TTCAAACA (SEQ ID NO: 11).

Altogether, these data demonstrate using RSEC to correct raw ML counts.

Example 5

ML Coverage Calculations

This example describes ML coverage calculation.

After RSEC, gene ML counts per well are evaluated to determine their suitability for further correction. Genes with low ML coverage (< 4 Reads Per ML) bypass subsequent correction steps and are reported in the final ML data table and is logged to be "Low Depth" in the bioinformatics pipeline. For genes with extremely high inputs whereby at least 6557 out of the possible 6561 barcodes are observed, where it becomes challenging to determine the number of molecules due to barcode diversity and genes are flagged as "Saturated." For gene MLs that do not meet either of the 2 decision points move forward to the subsequent DBEC algorithm and are marked as "Pass" in the output log file. In addition, genes with higher than an average of 650 MLs per well are logged to be 'High Input' as >5% of these MLs are recycled based on a Poisson distribution (FIG. 15).

Altogether, this example describes ML coverage calculation.

Example 6

Distribution-Based Error Correction (DBEC)

This example describes distribution-based error correction.

Unlike RSEC, DBEC algorithm is a method to discriminate whether a ML is an error or true signal regardless of its ML sequence. While RSEC can use both ML sequence and ML coverage information to correct for errors, DBEC relies primarily on ML coverage only to correct for non-substitution errors. As aforementioned, error barcodes generally have low ML coverage that is distinct from true barcodes ML coverage; this difference in ML coverage can be observed in a histogram plot of the ML coverage as distinct distributions (FIG. 15). Given this difference, DBEC fits two negative binomial distributions to statistically distinguish between ML errors (with lower ML coverage), and one for true signal with higher ML coverage.

*Removal of Recycled MLs for Optimal Distribution Fitting*

For a given gene, as ML detected increases, the percentage of recycled ML (i.e. the same ML is used to label 2 or more mRNAs from the same gene) increases and can be estimated. Using a Poisson distribution ($\lambda_{non\text{-}unique}$), the number of recycled MLs for well $i$ ($n_{non\text{-}unique,\, i}$) is estimated from the ML recycling rate equation (Equation (6)). If the estimated recycled ML is greater than 5% of total ML for the given gene in well i, this gene in well *i* is flagged as "High Input." For these "High Input" data, the top ML coverage MLs would be eliminated from distribution fitting - but preserved for later counting steps - to obtain a better negative binomial distribution.

$$P(X > 1\,|\,\lambda_{non-unique}),\, \lambda_{non-unique}$$

$$= Number\ of\ ML\ /\ 6561. \qquad \text{Equation (6)}$$

$$n_{non-unique} = \sum_{i=i}^{n\ wells} n_{non-unique,i}. \qquad \text{Equation (7)}$$

*Addition of Pseudopoints for Low Expressing Genes*

If the unique number of ML is less than 10, it is often more challenging for fitting distributions due to the sparseness of the data. To alleviate this problem, DBEC adds pseudopoints at 1% signal counts are used for assisting the distribution fitting, yet does not affect the data.

*Estimation of Parameters*

To fit two negative binomial distributions to separate the error from the signal ML, two sets of starting values for parameter estimation are approximated. The error distribution is assumed to be negative binomial with mean and dispersion of 1.

*Error/Signal Probability Estimation*

Assume the signal and error distributions as NegativeBinomial($\mu_{signal}$, size$_{signal}$) and NegativeBinomial($\mu_{error}$, size$_{error}$), respectively. To determine the number of signal ML, in ascending order, the probabilities that the number of reads from a given ML are from the signal and error distributions are calculated until Equation (8) is satisfied where all preceding ML are considered as error ML.

$$P(X = r \mid \mu = \mu_{error}, size = size_{error})$$
$$< P(X = r \mid \mu = \mu_{signal}, size = size_{signal}). \qquad \text{Equation (8)}$$

Altogether, this example shows calculations for performing distribution-based error correction.

Example 7

Correcting PCR and Sequencing Errors Based on DBEC

This example demonstrates correcting PCR and sequencing errors based on two negative binomial distributions.

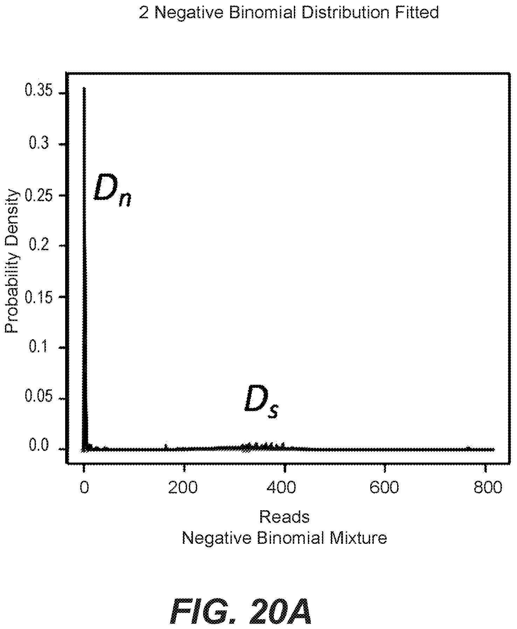

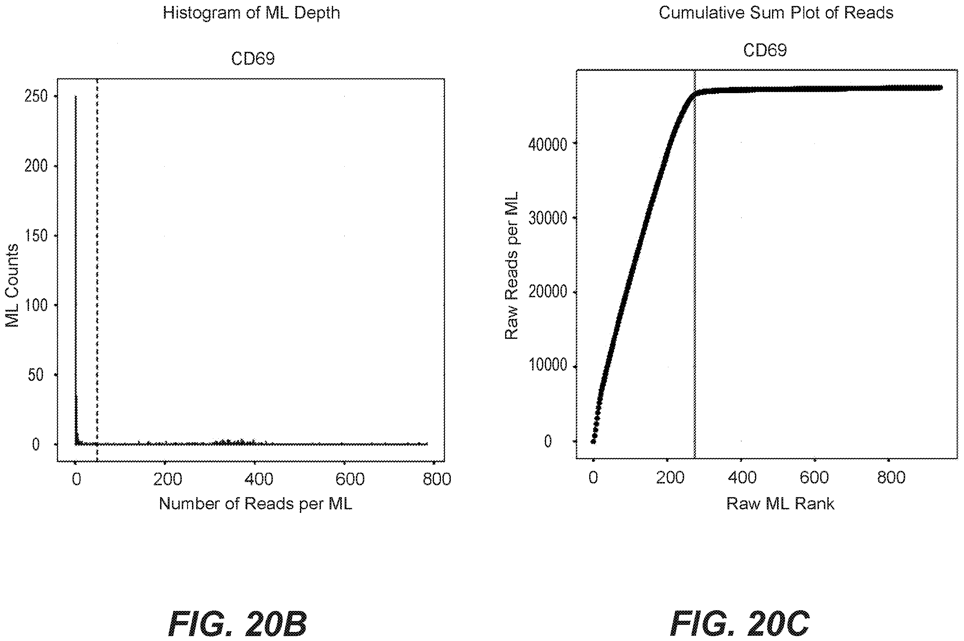

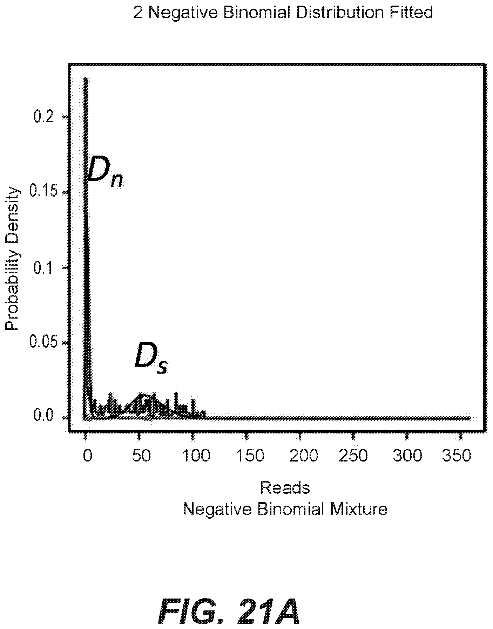

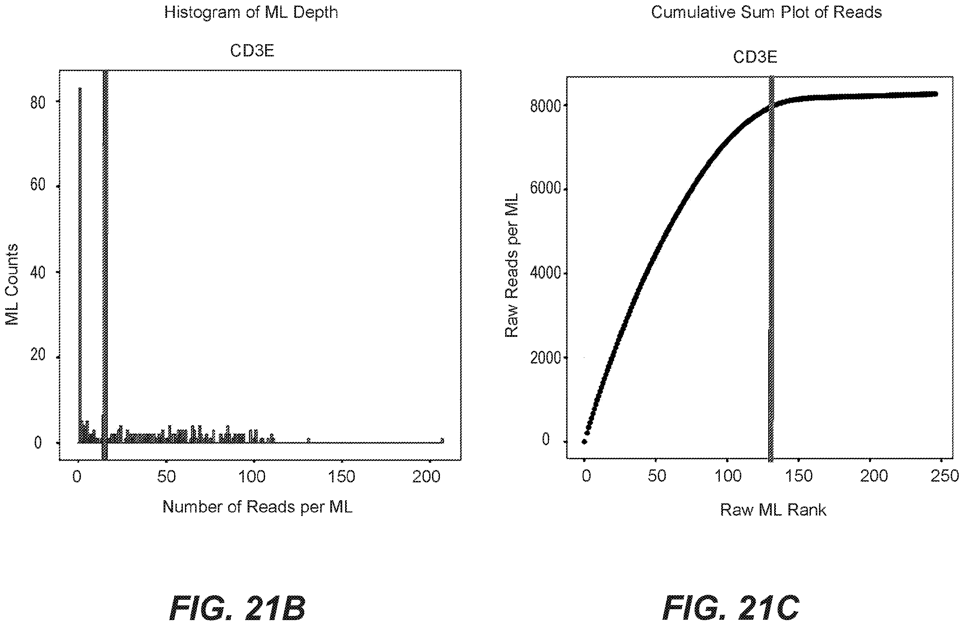

FIGS. 20A-20C show exemplary results of correcting PCR and sequencing errors based on two negative binomial distributions for CD69. FIG. 20A shows the fitting of two negative binomial distributions ($D_n$ for the noise negative binomial distribution and $D_s$ for the signal binomial distribution) for CD69 on the ML count data shown in the histogram of ML depth in FIG. 20B. The dotted line in FIG. 20B shows the separation of ML signals and SL errors determined by the two negative binomial distributions shown in FIG. 20A. The vertical line in FIG. 20C shows the local maximum of second derivatives as determined based on the cumulative sum plot of reads. Similar to FIGS. 20A-20C, FIGS. 21A-21C show exemplary results of correcting PCR and sequencing errors based on two negative binomial distributions for CD3E.

Altogether, these data show that DBEC can be used to correct for PCR and sequencing errors.

Example 8

ML Counts Correction Using Two Negative Binomial Distributions

This example demonstrates ML counts of ten targets corrected using two negative binomial distributions.

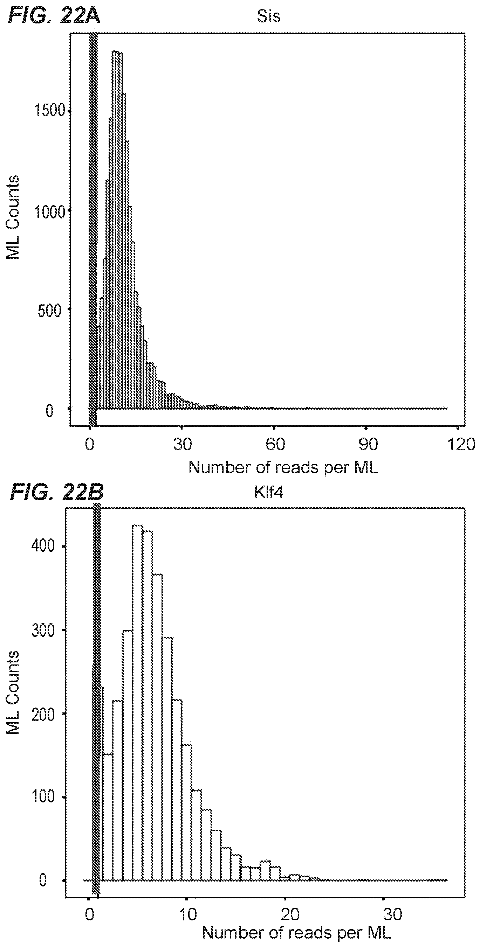

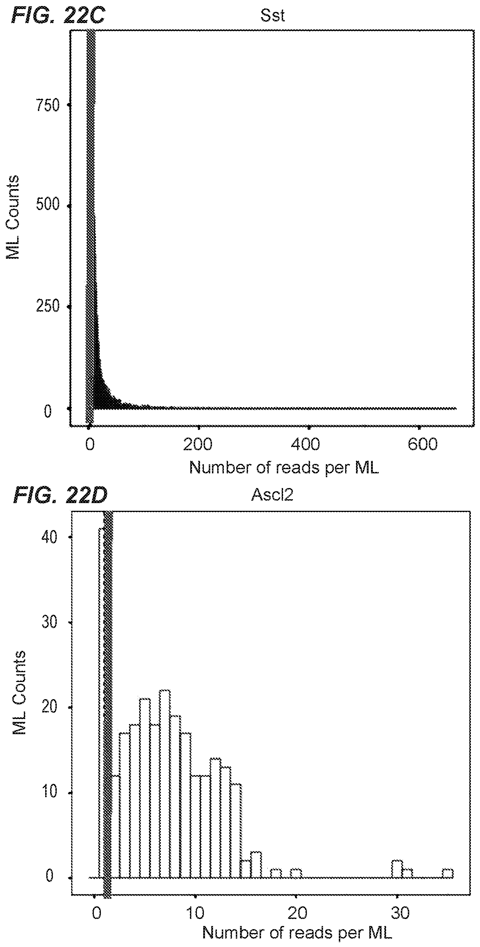

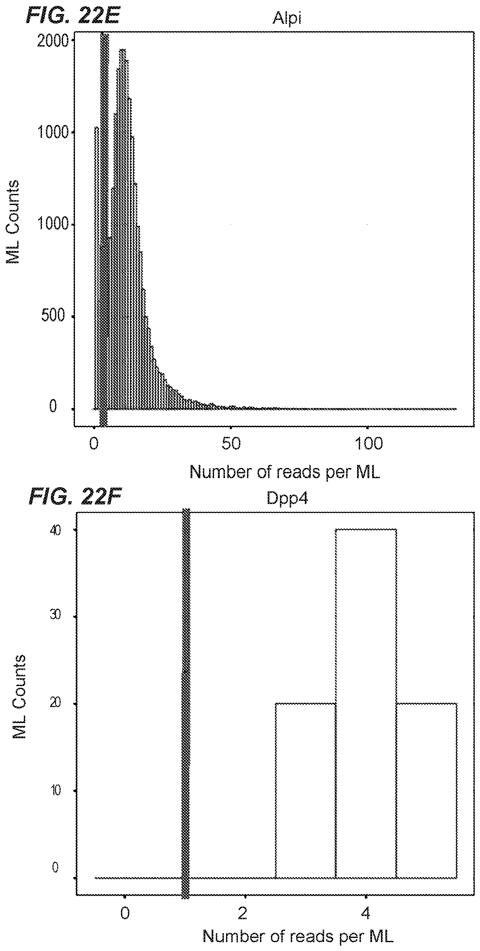

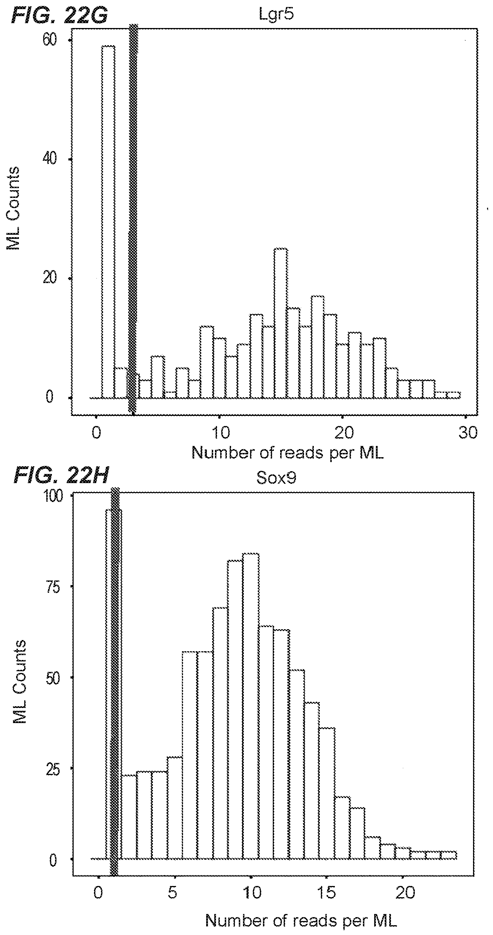

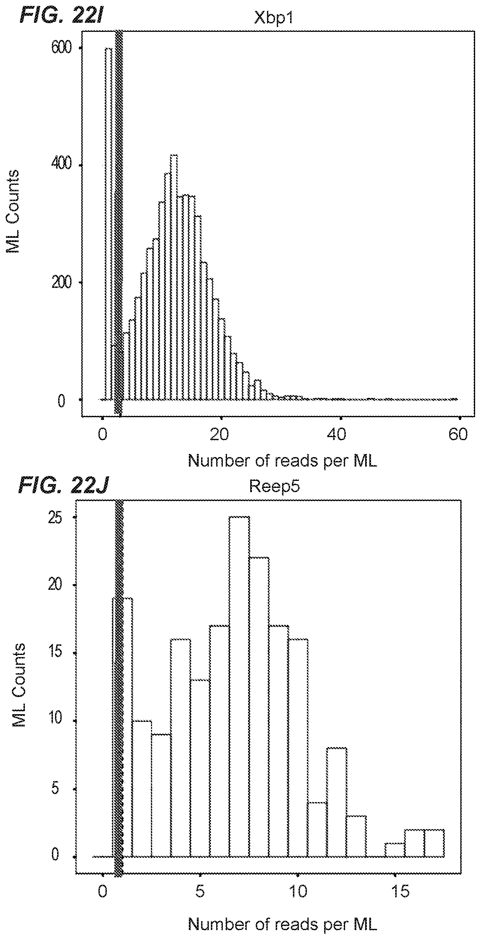

FIGS. 22A-22J show non-limiting exemplary validation of dataset corrected using two negative binomial distributions. ML counts of ten targets were corrected as shown in FIGS. 22A-22J. The vertical line in each of FIGS. 22A-22J shows the separation of ML signals and SL errors for a target determined using two negative binomial distributions.

Altogether, these data validate ML counts correction using two negative binomial distributions.

Example 9

t-Stochastic Neighbor Embedding Visualization of a BD Precise™ Targeted Assay from a 96- Well of Mixed Jurkat and Breast Cancer (BrCa) Single Cells

This example demonstrates a method for correcting PCR and sequencing errors based on recursive substitution error correction and distribution-based error correction for mixed Jurkat and breast cancer (BrCa) single cells.

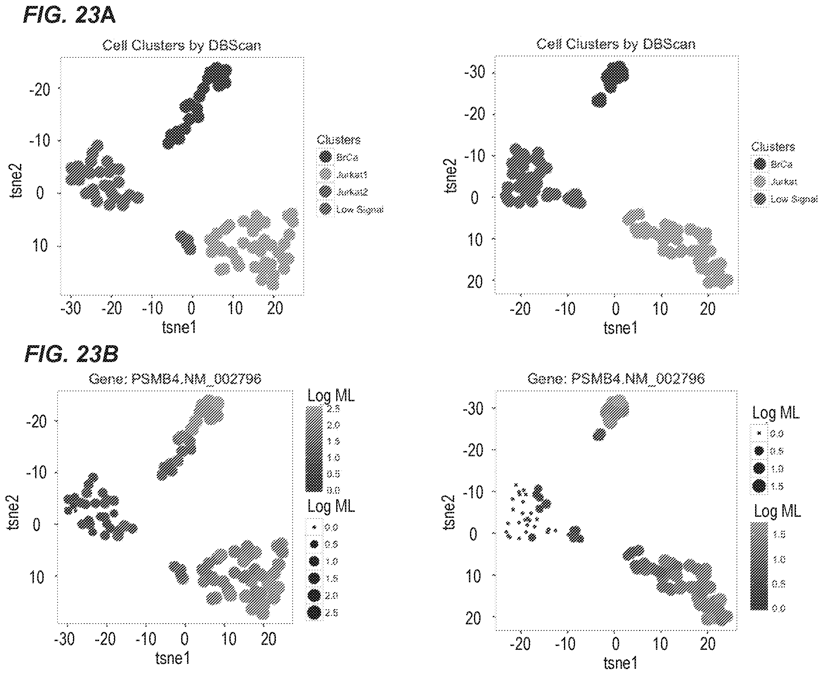

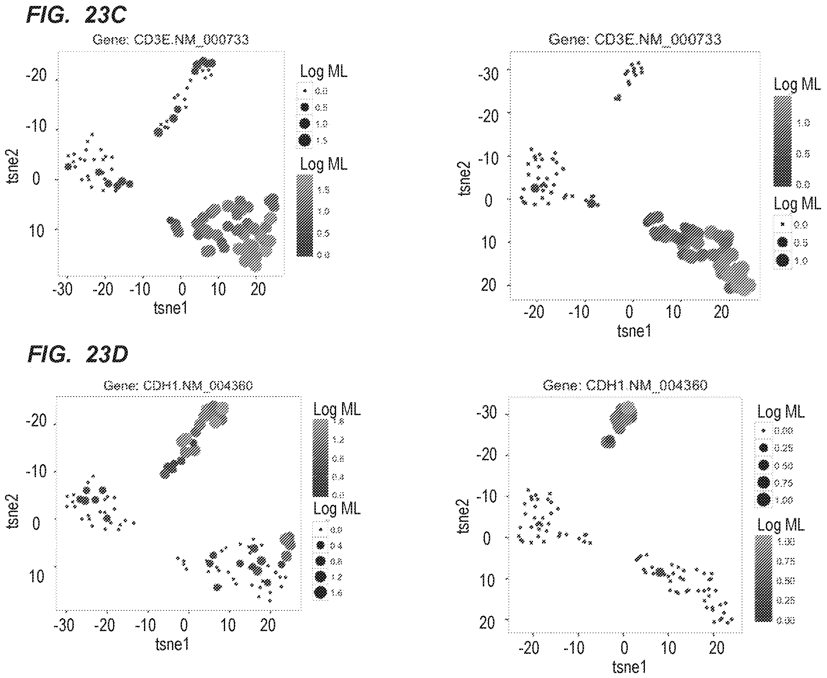

FIGS. 23A-23D show exemplary t-stochastic neighbor embedding (t-SNE) visualizations of Precise™ targeted assay from a 96-well of mixed Jurkat and breast cancer (BrCa) single cells (86 genes examined). FIG. 23A shows that cell clusters were identified using DBScan with the same parameters before and after ML adjustments. FIGS. 23B-23D show individual marker expression scaled both by color and point size. FIG. 23B shows PSMB4, a housekeeping gene that is present in both cell types and after ML adjustments, the lack of PSMB4 signal is highlighted further in the 'Low Signal' cluster. FIG. 23C shows CD3E, a lymphocyte marker that highlights Jurkat cell clusters. FIG. 23D shows CDH1, an epithelial cell marker that highlights the BrCa cluster.

Altogether, these data demonstrate that ML adjustment removed ML noise which allowed for clear differentiation of gene expression between cell clusters.

Example 10

Differential Expression Analysis between Cell Clusters

This example demonstrates a method of correcting PCR and sequencing errors based on recursive substitution error correction and distribution-based error correction for low signal cells and breast cancer (BrCa) cells.

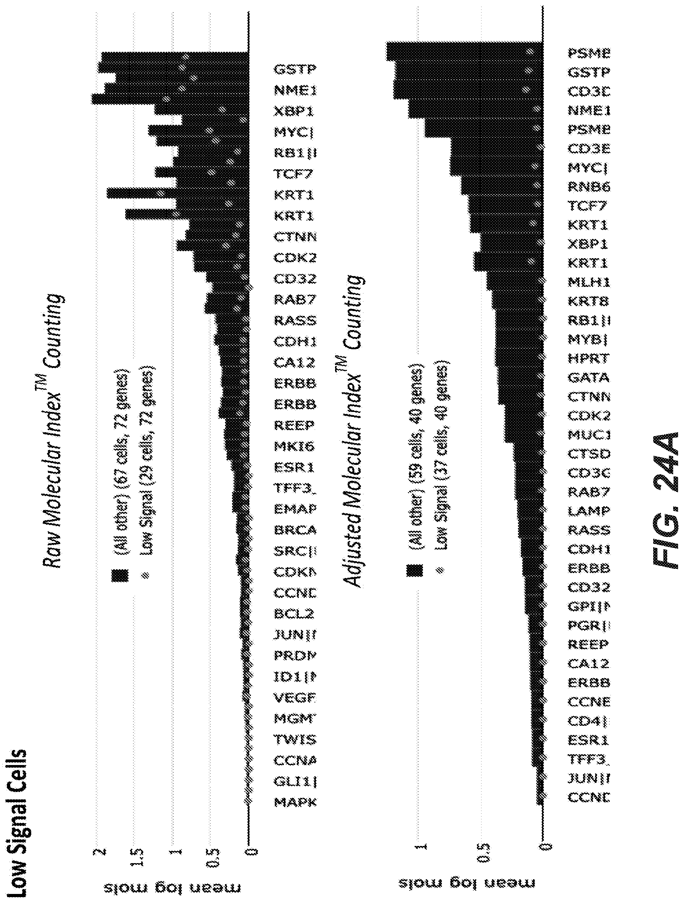

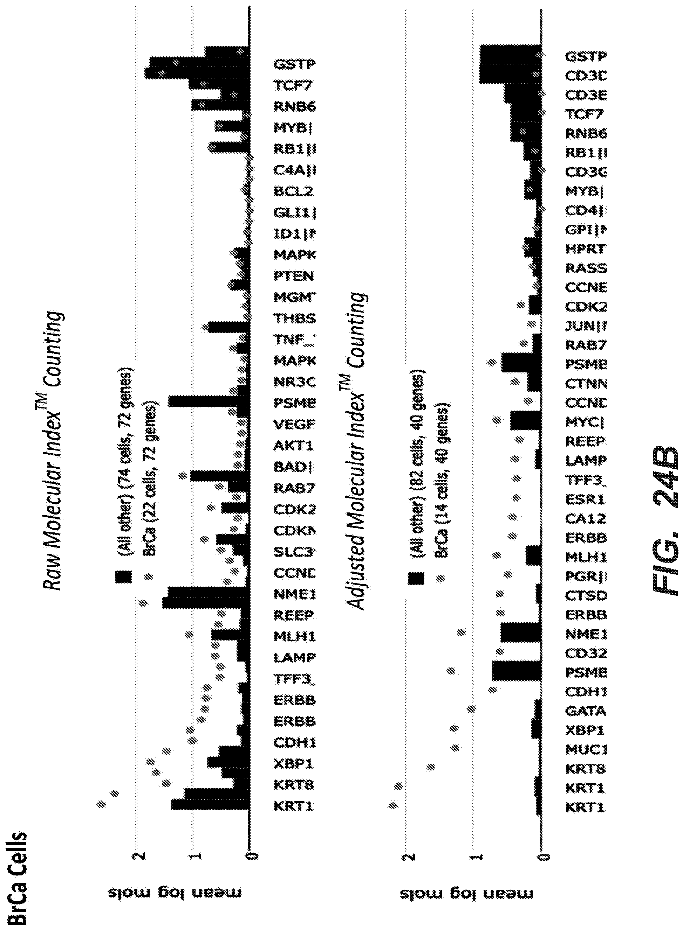

FIGS. 24A-24B are non-limiting exemplary plots showing differential expression analysis between cell clusters for genes with >0 ML in both selected clusters calculated by DBScan and determined by gene marker level in each cluster. FIG. 24A shows 'Low Signal' cluster gene expression compared to the rest of the cells. FIG. 24A, top panel shows raw ML comparison showing that ML noise was generally higher for genes with higher average expression in other cells. FIG. 24A, bottom panel shows that after ML adjustments using RSEC and DBEC, ML noise detected in 'Low Signal' cluster was reduced, allowing clearer distinction of gene expression between clusters. FIG. 24B shows 'BrCa' cluster gene expression compared to the rest of the cells. FIG. 24B, top panel shows raw ML in non-BrCa cells also had a significant ML count of BrCa markers, such as KRT1, MUC1. FIG. 24B, bottom panel shows adjusted MLs of BrCa markers were highly enriched in BrCa cluster than the rest of the cells.

Altogether, these data demonstrate that PCR and sequencing errors can be corrected based on recursive substitution error correction and distribution-based error correction for cells, such as low signal cells and breast cancer cells.

Example 11

Adjusting Molecular Label Counts for Mixed Jurkat and T47D Cells

This example demonstrates a method of adjusting molecular label counts for mixed Jurkat and T47D cells.

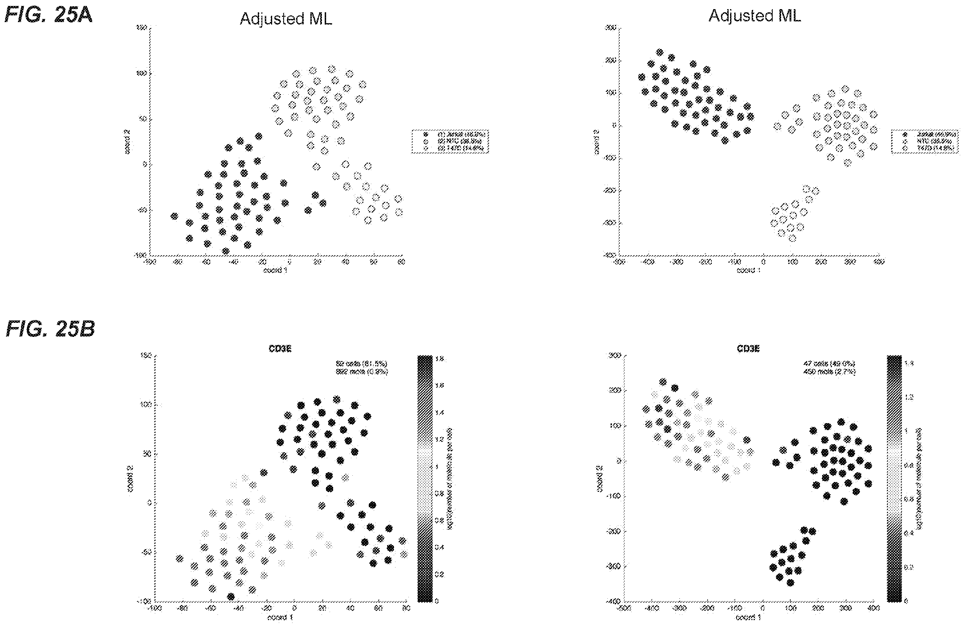

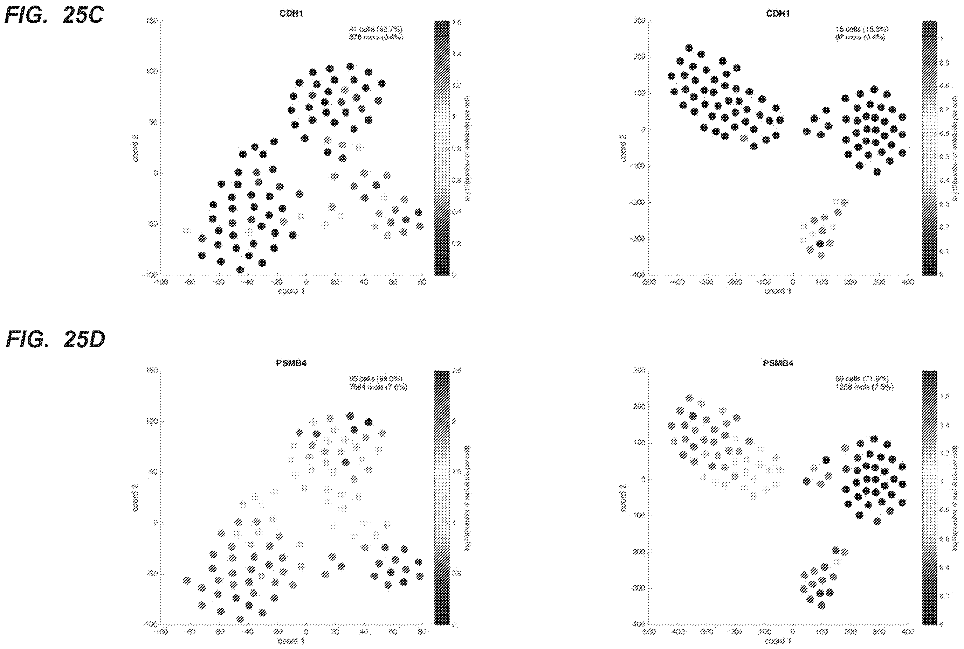

FIGS. 25A-25D are non-limiting exemplary plots showing t-stochastic neighbor embedding (t-SNE) visualization of a BD Precise™ targeted assay from a 96-well plate of mixed Jurkat and breast cancer (T47D) single cells with 86 genes examined. FIG. 25A show that cell clusters were identified using DBScan with the same parameters before and after ML adjustments. FIGS. 25B-25D show that individual marker expression scaled both by color and point size. FIG. 25B shows the scaling of PSMB4, a housekeeping gene that was present in both cell types and after ML adjustments. The lack of PSMB4 signal is highlighted further in the no-template control (NTC) cluster. FIG. 25C shows the scaling of CD3E, a lymphocyte marker that highlights Jurkat cell clusters. FIG. 25D shows the scaling of CDH1, an epithelial cell marker that highlights the T47D cluster.

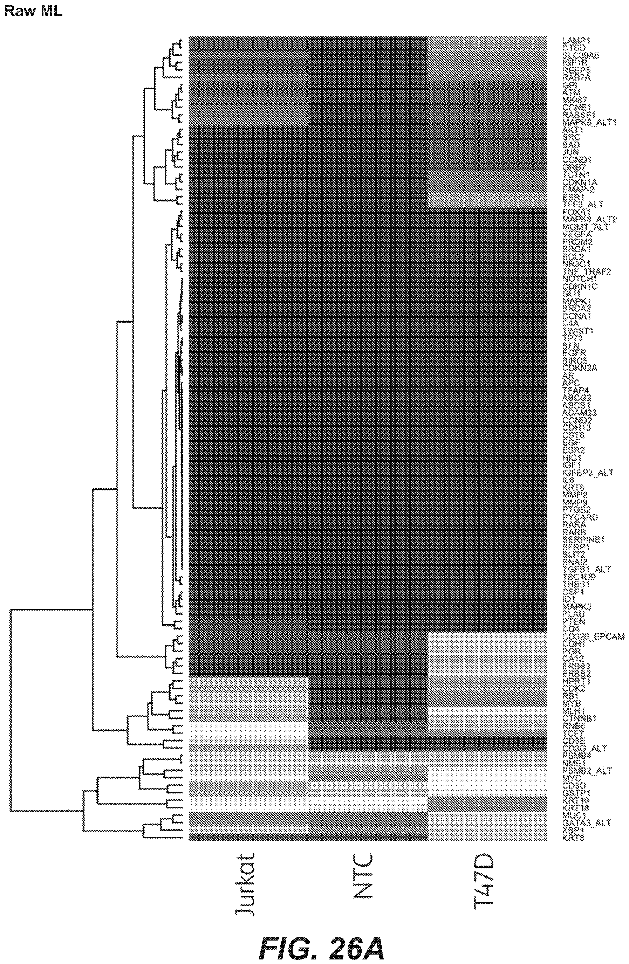

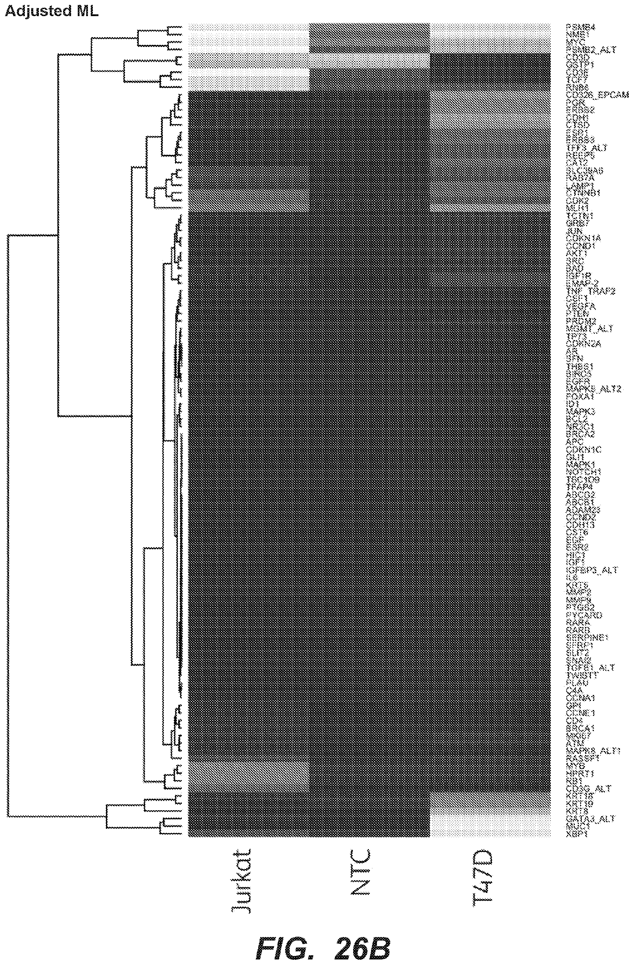

FIGS. 26A-26B are non-limiting exemplary heat maps displaying differential gene expression by molecular label counts between different cell clusters identified in FIGS. 25A-25D before any error correction steps (Raw ML shown in FIG. 26A) and after RSEC and DBEC correction (Adjusted ML shown in FIG. 26B). Genes that were low in expression is in blue, and genes that are high in expression is orange. Genes that are similar in gene expression pattern between these cell types are clustered together. Without error correction, NTC had noise from high expressing genes such as CD3E and KRT18, which are Jurkat and T47D markers, respectively. Moreover, error correction revealed distinct gene expression patterns between Jurkat and T47D.

Altogether, these data demonstrate that ML adjustment can remove MI noise which allows for clear differentiation of gene expression between cell clusters.

Example 12

Immune Receptor-Barcode Error Correction Using Recursive Substitution Error Correction

This example demonstrates immune receptor-barcode error correction based on recursive substitution error correction.

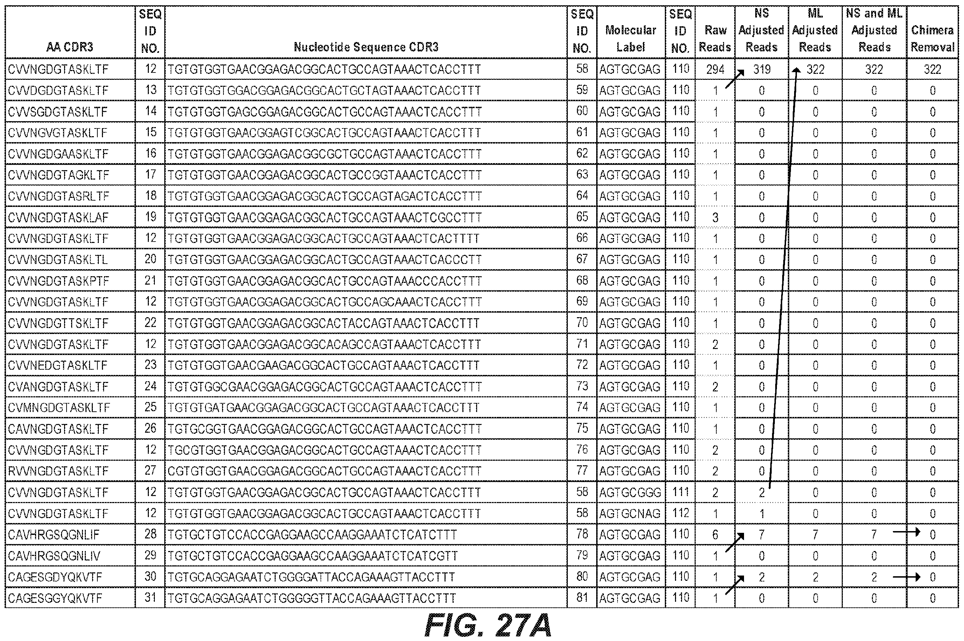

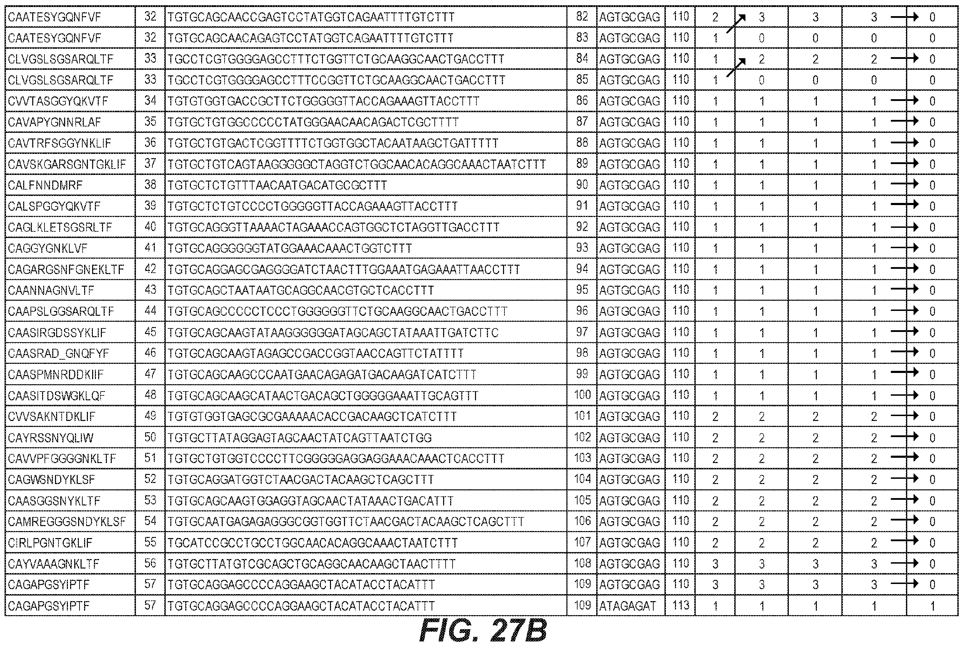

FIGS. 27A-27B show a table illustrating a non-limiting example of immune receptor-barcode error correction based on recursive substitution error correction. Performing immune receptor-barcode error correction included adjusting counts of nucleotide sequences (NSs) of interest (e.g., putative nucleotide sequences of CDR3) by recursive substitution error correction. Multiple clusters of putative sequences of CDR3 were identified. One cluster included sequences that differ from the parent nucleotide sequence TGTGTGGTGAACGGAGACGGCACTGCCAGTAAACTCACCTTT (SEQ ID NO: 58) by one nucleotide (underlined), such as the child nucleotide sequences TGTGTGGTGAACGGAGACGGCACTGCCAGTAAACTCACTTTT (SEQ ID NO: 59) and CGTGTGGTGAACGGAGACGGCACTGCCAGTAAACTCACCTTT (SEQ ID NO: 77). The nucleotide sequence TGTGTGGTGAACGGAGACGGCACTGCCAGTAAACTCACCTTT (SEQ ID NO: 58) was the parent nucleotide sequence because it had the highest raw reads in this cluster, 294, which was the number of molecular labels with different sequences associated with this nucleotide sequence in the sequencing data.

Other clusters included TGTGCTGTCCACCGAGGAAGCCAAGGAAATCTCATCTTT (SEQ ID NO: 78), and TGTGCTGTCCACCGAGGAAGCCAAGGAAATCTCATCGTT (SEQ ID NO: 79); TGTGCAGGAGAATCTGGGGATTACCAGAAAGTTACCTTT (SEQ ID NO: 80), and TGTGCAGGAGAATCTGGGGGTTACCAGAAAGTTACCTTT (SEQ ID NO: 81); TGTGCAGCAACCGAGTCCTATGGTCAGAATTTTGTCTTT (SEQ ID NO: 82), and TGTGCAGCAACAGAGTCCTATGGTCAGAATTTTGTCTTT (SEQ ID NO: 83); and TGCCTCGTGGGGAGCCTTTCTGGTTCTGCAAGGCAACTGACCTTT (SEQ ID NO: 84) and TGCCTCGTGGGGAGCCTTTCCGGTTCTGCAAGGCAACTGACCTTT (SEQ ID NO: 85). The occurrence of each child nucleotide sequence was attributed to the corresponding parent molecular label sequence (shown as the arrows from the column labeled "Raw Reads" to the column labeled "NS Adjusted Reads".

Performing immune receptor-barcode error correction included adjusting counts of the molecular labels (MLs) by recursive substitution error correction. A cluster of molecular label sequence included the parent molecular label sequence AGTGCGAG (SEQ ID NO: 110) and the child molecular label sequences AGTGCGGG and AGTGCNAG (SEQ ID NOs: 111 and 112 respectively), which differ from the parent molecular label sequence by one nucleotide (underlined). In this example, the molecular label sequence AGTGCGAG (SEQ ID NO: 110) associated with the CDR3 sequence TGTGTGGTGAACGGAGACGGCACTGCCAGTAAACTCACCTTT (SEQ ID NO: 58) had the highest nucleotide sequence adjusted reads for all CDR3 sequences associated with the molecular label AGTGCGAG (SEQ ID NO: 110). The occurrence of each child nucleotide sequence (two and one for the child molecular label sequences AGTGCGGG and AGTGCNAG (SEQ ID NOs: 111 and 112 respectively)), was attributed to the corresponding parent molecular label sequence with the highest nucleotide sequence adjusted reads (319 for the parent molecular label AGTGCGAG (SEQ ID NO: 110)). Performing immune receptor-barcode error correction included adjusting counts of the nucleotide sequences and molecular labels at the same time based on RSEC. In this example, no molecular label count was adjusted when each nucleotide sequence and the corresponding molecular label was considered as one sequence when applying RSEC.

Multiple chimeras were identified, and molecular label counts corresponding to chimeras were removed. For example, the molecular label sequence AGTGCGAG (SEQ ID NO: 110) was associated with multiple CDR3 nucleotide sequences, such as TGTGTGGTGAACGGAGACGGCACTGCCAGTAAACTCACCTTT (SEQ ID NO: 58) and TGTGCTGTCCACCGAGGAAGCCAAGGAAATCTCATCTTT (SEQ ID NO: 78). The adjusted occurrence (e.g., the number of occurrence observed in the sequencing data after adjustment described above) of the nucleotide sequence TGTGCTGTCCACCGAGGAAGCCAAGGAAATCTCATCTTT (SEQ ID NO: 78) was 7, lower than the adjusted occurrence of the nucleotide sequence TGTGTGGTGAACGGAGACGGCACTGCCAGTAAACTCACCTTT (SEQ ID NO: 58), 322, which was the highest of all nucleotide sequences with the molecular label sequence AGTGCGAG (SEQ ID NO: 110). The putative sequences of the target corresponding to the chimeric sequences of CDR3 identified were removed (shown as the arrows going from the column labeled "NS and ML Adjusted Reads" to the column labeled "Chimera Removed"). With adjustments and chimera removal, the CDR3 sequence was determined to be TGTGTGGTGAACGGAGACGGCACTGCCAGTAAACTCACCTTT (SEQ ID NO. 58), which was associated with the molecular label sequence AGTGCGAG (SEQ ID NO. 110) having an adjusted molecular label count of 322.

Altogether, these data demonstrate adjusting nucleotide sequence counts, molecular label counts, and nucleotide sequence and molecular counts with RSEC and removing chimeric CDR3 sequences.

Example 13

Immune Receptor-Barcode Error Correction Using Recursive Substitution Error Correction and Distribution-Based Error Correction

This example demonstrates immune receptor-barcode error correction using recursive substitution error correction and distribution-based error correction.

A sample of 500 cells with 75% healthy peripheral blood mononuclear cell (PBMCs) and 25% Jurkat cells were loaded in a Rhapsody™ cartridge and captured in microwells that contained Rhapsody™ beads. The Rhapsody™ beads were magnetic beads with barcodes attached to the bead surfaces. Each bead was attached with barcodes with cell labels with an identical cell label sequence and molecular labels selected from a group of diverse molecular sequences. Barcodes of different beads had cell labels with different cell label sequences. Barcodes of each bead had capture sites for capturing TCR mRNA molecules. The captured TCR mRNA molecules were barcoded and sequenced as described herein. Cell label and molecular labels were used to determine cell and molecule origin of each TCR molecule. The numbers of occurrences or the counts of different TCR molecules were determined using the molecular labels.

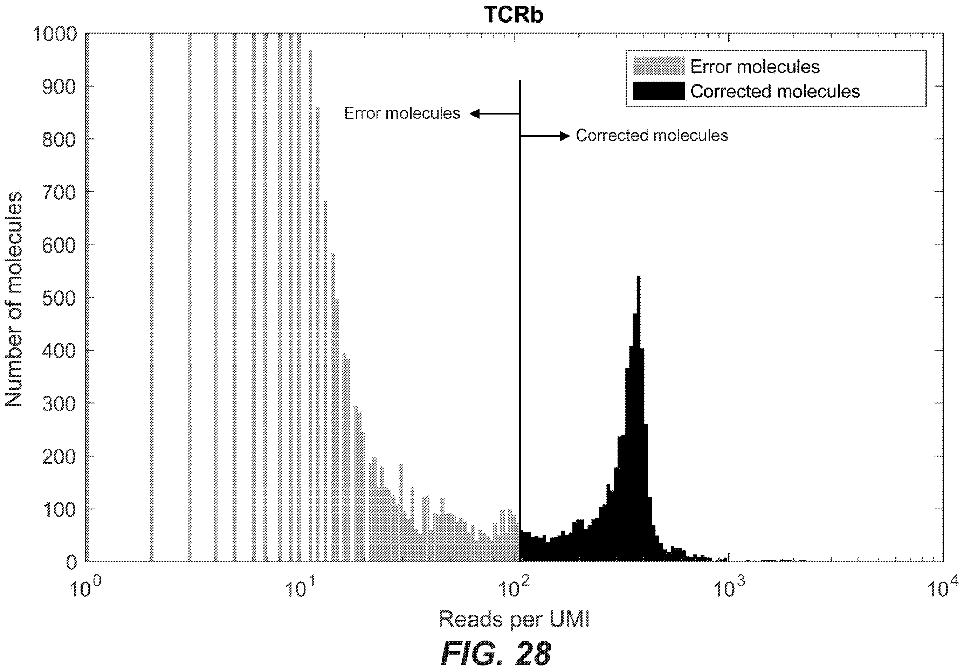

The sequencing data of different TCR molecules were corrected using immune receptor-barcode error correction (described with reference to FIGS. 7, 8, and 27 and Example 12). Briefly, the counts of putative nucleotide sequences of different TCR genes (e.g., TCRb) were adjusted based on recursive substitution error correction. The counts of the molecular labels were adjusted based on recursive substitution error correction. The counts of the nucleotide sequences (e.g., TCRb) and molecular labels were adjusted based on recursive substitution error correction. Molecular label counts that correspond to chimeras were removed. Subsequently, molecular label counts were adjusted using distribution-based error correction described herein. FIG. 28 is a histogram showing non-limiting exemplary results of immune receptor-barcode error correction followed by distribution-based error correction for TCRb. Without error correction, the TCR diversity (including TCRb diversity) would be overestimated.

Altogether, these data demonstrate adjusting TCR nucleotide sequence counts, molecular label counts, and TCR nucleotide sequence and molecular counts with recursive substitution error correction and removing chimeric TCR sequences, followed by distribution-based error correction to avoid overestimation of TCR diversity.

In at least some of the previously described embodiments, one or more elements used in an embodiment can interchangeably be used in another embodiment unless such a replacement is not technically feasible. It will be appreciated by those skilled in the art that various other omissions, additions and modifications may be made to the methods and structures described above without departing from the scope of the claimed subject matter. All such modifications and changes are intended to fall within the scope of the subject matter, as defined by the appended claims.

With respect to the use of substantially any plural and/or singular terms herein, those having skill in the art can translate from the plural to the singular and/or from the singular to the plural as is appropriate to the context and/or application. The various singular/plural permutations may be expressly set forth herein for sake of clarity. As used in this specification and the appended claims, the singular forms "a," "an," and "the" include plural references unless the context clearly dictates otherwise. Any reference to "or" herein is intended to encompass "and/or" unless otherwise stated.

It will be understood by those within the art that, in general, terms used herein, and especially in the appended claims (*e.g.*, bodies of the appended claims) are generally intended as "open" terms (*e.g.*, the term "including" should be interpreted as "including but not limited to," the term "having" should be interpreted as "having at least," the term "includes" should be interpreted as "includes but is not limited to," etc.). It will be further understood by those within the art that if a specific number of an introduced claim recitation is intended, such an intent will be explicitly recited in the claim, and in the absence of such recitation no such intent is present. For example, as an aid to understanding, the following appended claims may contain usage of the introductory phrases "at least one" and "one or more" to introduce claim recitations. However, the use of such phrases should not be construed to imply that the introduction of a claim recitation by the indefinite articles "a" or "an" limits any particular claim containing such introduced claim recitation to embodiments containing only one such recitation, even when the same claim includes the introductory phrases "one or more" or "at least one" and indefinite articles such as "a" or "an" (*e.g.*, "a" and/or "an" should be interpreted to mean "at least one" or "one or more"); the same holds true for the use of definite articles used to introduce claim recitations. In addition, even if a specific number of an introduced claim recitation is explicitly recited, those skilled in the art will recognize that such recitation should be interpreted to mean at least the recited number (*e.g.,* the bare recitation of "two recitations," without other modifiers, means at least two recitations, or two or more recitations). Furthermore, in those instances where a convention analogous to "at least one of A, B, and C, etc." is used, in general such a construction is intended in the sense one having skill in the art would understand the convention (*e.g.*, " a system having at least one of A, B, and C" would include but not be limited to systems that have A alone, B alone, C alone, A and B together, A and C together, B and C together, and/or A, B, and C together, etc.). In those instances where a convention analogous to "at least one of A, B, or C, etc." is used, in general such a construction is intended in the sense one having skill in the art would understand the convention (*e.g.*, " a system having at least one of A, B, or C" would include but not be limited to systems that have A alone, B alone, C alone, A and B together, A and C together, B and C together, and/or A, B, and C together, etc.). It will be further understood by those within the art that virtually any disjunctive word and/or phrase presenting two or more alternative terms, whether in the description, claims, or drawings, should be understood to contemplate the possibilities of including one of the terms, either of the terms, or both terms. For example, the phrase "A or B" will be understood to include the possibilities of "A" or "B" or "A and B."

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, such as in terms of providing a written description, all ranges disclosed herein also encompass any and all possible sub-ranges and combinations of sub-ranges thereof. Any listed range can be easily recognized as sufficiently describing and enabling the same range being broken down into at least equal halves, thirds, quarters, fifths, tenths, etc. As a nonlimiting example, each range discussed herein can be readily broken down into a lower third, middle third and upper third, etc. As will also be understood by one skilled in the art all language such as "up to," "at least," "greater than," "less than," and the like include the number recited and refer to ranges which can be subsequently broken down into sub-ranges as discussed above. Finally, as will be understood by one skilled in the art, a range includes each individual member. Thus, for example, a group having 1-3 articles refers to groups having 1, 2, or 3 articles. Similarly, a group having 1-5 articles refers to groups having 1, 2, 3, 4, or 5 articles, and so forth.

While various aspects and embodiments have been disclosed herein, other aspects and embodiments will be apparent to those skilled in the art. The various aspects and embodiments disclosed herein are for purposes of illustration and are not intended to be limiting, with the true scope and spirit being indicated by the following claims.

The invention also provides the following numbered embodiments:

1. A method for determining occurrences of targets, comprising:

(a) stochastically barcoding a plurality of targets using a plurality of stochastic barcodes to create a plurality of stochastically barcoded targets, wherein each of the plurality of stochastic barcodes comprises a cell label and a molecular label, wherein molecular labels of at least two stochastic barcodes of the plurality of stochastic barcodes comprise different molecular label sequences, and wherein at least two stochastic barcodes of the plurality of stochastic barcodes comprise cell labels with an identical cell label sequence;

(b) obtaining sequencing data of the stochastically barcoded targets; and

(c) for at least one target of the plurality of targets:

(i) identifying putative sequences of the target in the sequencing data;

(ii) counting occurrences of molecular label sequences associated with the putative sequences of the target in the sequencing data identified in (i);

(iii) identifying clusters of the putative sequences of the target;

(iv) collapsing the sequencing data obtained using the clusters of putative sequences of the target identified in (iii);

(v) identifying clusters of the molecular label sequences associated with the putative sequences of the target;

(vi) collapsing the sequencing data using the clusters of molecular label sequences identified in (v);

(vii) identifying clusters of combination sequences, wherein each combination sequence comprises a sequence of the sequences of the target and an associated molecular label sequence of the molecular label sequences;

(viii) collapsing the sequencing data using the clusters of combination sequences identified in (vii);

(ix) identifying one or more putative sequences of the target that correspond to one or more chimeric sequences of the target, wherein occurrences of the one or more putative sequences of the target that correspond to the one or more chimeric sequences of the target are smaller than occurrences of remaining one or more putative sequences of the target that do not correspond to the one or more chimeric sequences of the target;

(x) removing the one or more putative sequences of the target corresponding to the one or more chimeric sequences of the target identified in (ix) from the sequencing data; and

(xi) estimating the occurrence of the target, wherein the occurrence of the target estimated correlates with the number of molecular label sequences counted in (ii) after collapsing the sequencing data in (iv), (vi), and (viii) and removing the one or more putative sequences of the target that correspond to the one or more chimeric sequences of the target in (x).

2. The method of embodiment 1, wherein the plurality of targets comprises targets of the whole transcriptome of a cell.

3. The method of embodiment 2, wherein the plurality of targets comprises a gene.

4. The method of embodiment 3, wherein the gene comprises a variable sequence.

5. The method of embodiment 4, wherein the gene encodes a T-cell Receptor.

6. The method of any one of embodiments 1-5, wherein the putative sequences of the target differ from one another by at least one nucleotide.

7. The method of any one of embodiments 1-6, wherein identifying the clusters of the putative sequences of the target comprises identifying the clusters of the putative sequences of the target using directional adjacency.

8. The method of embodiment 7, wherein putative sequences of the target within a cluster are within a first predetermined directional adjacency threshold of one another.

9. The method of embodiment 8, wherein the first directional adjacency threshold is a Hamming distance of one.

10. The method of any one of embodiments 8-9, wherein the putative sequences of the target within the cluster comprise one or more parent sequences and one or more children sequences of the one or more parent sequences, and wherein an occurrence of the parent sequence is greater than or equal to a first predetermined directional adjacency occurrence threshold.

11. The method of embodiment 10, wherein the first predetermined directional adjacency occurrence threshold is twice an occurrence of a child sequence less one.

12. The method of any one of embodiments 1-11, wherein collapsing the sequencing data obtained in (b) using the clusters of putative sequences of the target identified in (iii) comprises:

attributing an occurrence of a child sequence of the one or more children sequences to the parent sequence of the child sequence.

13. The method of any one of embodiments 1-12, wherein identifying the clusters of the molecular label sequences associated with the putative sequences of the target comprises identifying the clusters of the molecular label sequences associated with the putative sequences of the target using directional adjacency.

14. The method of embodiment 13, wherein molecular label sequences of the target within a cluster are within a second predetermined directional adjacency threshold of one another.

15. The method of embodiment 14, wherein the second directional adjacency threshold is a Hamming distance of one.

16. The method of any one of embodiments 14-15, wherein the putative molecular label sequences of the target within the cluster comprise one or more parent molecular label sequences and one or more children molecular label sequences of the one or more parent molecular label sequences, and wherein the occurrence of the parent molecular label sequence is greater than or equal to a second predetermined directional adjacency occurrence threshold.

17. The method of embodiment 16, wherein the second predetermined directional adjacency occurrence threshold is twice an occurrence of a child molecular label sequence less one.

18. The method of any one of embodiments 1-17, wherein collapsing the sequencing data using the clusters of molecular label sequences associated with the sequences of the target identified in (v) comprises:

attributing an occurrence of a child molecular label sequence of the one or more children molecular label sequences to the parent molecular label of the child molecular label sequence.

19. The method of any one of embodiments 1-18, wherein identifying the clusters of combination sequences comprises identifying clusters of combination sequences using directional adjacency.

20. The method of embodiment 19, wherein combination sequences within a cluster are within a third predetermined directional adjacency threshold of one another.

21. The method of embodiment 20, wherein the third directional adjacency threshold is a Hamming distance of one.

22. The method of any one of embodiments 20-21, wherein the combination sequences within the cluster comprise one or more parent combination sequences and one or more children combination sequences of the one or more parent combination sequences, and wherein an occurrence of the parent combination sequence is greater than or equal to a third predetermined directional adjacency occurrence threshold.

23. The method of embodiment 22, wherein the third predetermined directional adjacency occurrence threshold is twice an occurrence of a child combination sequence less one.

24. The method of any one of embodiments 1-23, wherein collapsing the sequencing data using the clusters of combination sequences identified in (vii) comprises:

attributing an occurrence of a child combination sequence of the one or more children combination sequences to the parent combination sequence of the child combination sequence.

25. The method of any one of embodiments 1-24, wherein identifying the one or more putative sequences of the target corresponding to the one or more chimeric sequences of the target:

identifying putative sequences of the target associated with one molecular label sequence of the plurality of molecular sequences;
identifying a putative sequence of the putative sequences of the target associated with the one molecular label sequence with an occurrence smaller than a chimeric occurrence threshold as corresponding to a chimeric sequence of the one or more chimeric sequences of the target.

26. The method of embodiment 25, wherein a value of the chimeric occurrence threshold is an occurrence of a putative sequence of the putative sequences of the target associated with the one molecular label sequence that is greater than an occurrence of any other sequence of the putative sequences of the target.

27. The method of any one of embodiments 1-26, further comprising:
adjusting the sequencing data after collapsing the sequencing data in (iv), (vi), and (viii) and removing the one or more putative sequences of the target that correspond to the one or more chimeric sequences of the target in (x).

28. The method of embodiment 27, wherein adjusting the sequencing data after collapsing the sequencing data in (iv), (vi), and (viii) and removing the one or more putative sequences of the target that correspond to the one or more chimeric sequences of the target in (x) comprises:
thresholding molecular label sequences associated with the putative sequences of the target to determine signal molecular label sequences and noise molecular label sequences associated with the sequences of the target in the sequencing data counted in (b) after collapsing the sequencing data in (iv), (vi), and (viii) and removing the one or more putative sequences of the target corresponding to the one or more chimeric sequences of the target in (x).

29. The method of embodiment 28, wherein thresholding the molecular label sequences associated with the putative sequences of the target comprises performing a statistical analysis on the molecular label sequences of the target.

30. The method of embodiment 29, wherein performing the statistical analysis comprises:

fitting the molecular label sequences associated with the putative sequences of the target and their occurrences to two negative binomial distributions;

determining an occurrence of signal molecular label sequences n using the two negative binomial distributions; and

removing the noise molecular label sequences from the sequencing data obtained in (b) after collapsing the sequencing data in (iv), (vi), and (viii) and removing the one or more putative sequences of the target that correspond to the one or more chimeric sequences of the target in (x), wherein the noise molecular label sequences comprise molecular label sequences with occurrences lower than an occurrence of the $n$th most abundant molecular label, and wherein the signal molecular label sequences comprise molecular label sequences with occurrences greater than or equal to the occurrence of the $n$th most abundant molecular label.

31. The method of embodiment 30, wherein the two negative binomial distributions comprise a first negative binomial distribution corresponding for the signal molecular label sequences and a second negative binomial distribution for the noise molecular label sequences.

32. A method for determining occurrences of targets, comprising:

(a) receiving sequencing data of a plurality of targets, wherein the sequencing data comprises putative sequences of a target of the plurality of targets and occurrences of molecular label sequences associated with the sequences of the target in the sequencing data;

(b) collapsing putative sequences of the target;

(c) collapsing molecular label sequences associated with the putative sequences of the target; and

(d) estimating the occurrence of the target, wherein the occurrence of the target estimated correlates with the occurrence of molecular label sequences associated with the putative sequences of the target in the sequencing data after collapsing the occurrence of the putative sequences of the target in (b) and the occurrence of noise molecular label sequences determined in (c).

33. The method of embodiment 32, comprising:

identifying the sequences of the target in the sequencing data; and

counting the occurrences of molecular label sequences associated with the sequences of the target in the sequencing data.

34. The method of any one of embodiments 32-33, comprising:

collapsing clusters of combination sequences, wherein each combination sequence comprises a sequence of the sequences of the target and an associated molecular label sequence of the molecular label sequences, wherein the occurrence of the target estimated correlates with the occurrence of molecular label sequences associated with the sequences of the target in the sequencing data after collapsing the occurrence of the combination sequences.

35. The method of embodiment 34, wherein collapsing the clusters of combination sequences comprises: collapsing the clusters of combination sequences using directional adjacency.

36. The method of embodiment 35, wherein collapsing the clusters of combination sequences using directional adjacency comprises:

identifying the clusters of combination sequences using directional adjacency; and

collapsing the sequencing data using the clusters of combination sequences identified.

37. The method of any one of embodiments 32-36, wherein collapsing the putative sequences of the target comprises: collapsing the putative sequences of the target using directional adjacency.

38. The method of embodiment 37, wherein collapsing the putative sequences of the target using directional adjacency comprises:

identifying clusters of the putative sequences of the target using directional adjacency; and

collapsing the sequencing data using the clusters of putative sequences of the target identified.

39. The method of any one of embodiments 32-38, wherein collapsing the molecular label sequences associated with the putative sequences of the target comprises: collapsing the molecular label sequences associated with the putative sequences of the target using directional adjacency.

40. The method of embodiment 39, wherein collapsing the molecular label sequences associated with the putative sequences of the target using directional adjacency comprises:

identifying clusters of the molecular label sequences associated with the putative sequences of the target using directional adjacency; and
collapsing the sequencing data using the clusters of molecular label sequences associated with the putative sequences of the target identified.

41. The method of any one of embodiments 32-40, comprising:

identifying one or more putative sequences of the target that correspond to one or more chimeric sequences of the target, wherein occurrences of the one or more putative sequences of the target that correspond to the one or more chimeric sequences of the target are smaller than occurrences of remaining one or more putative sequences of the target that do not correspond to the one or more chimeric sequences of the target; and
removing from the sequencing data one or more putative sequences of the target that correspond to one or more chimeric sequences of the target.

42. The method of embodiment 41, wherein identifying the one or more putative sequences of the target corresponding to the one or more chimeric sequences of the target comprises:

identifying putative sequences of the target associated with one molecular label sequence of the plurality of molecular sequences;
identifying a putative sequence of the putative sequences of the target associated with the one molecular label sequence with an occurrence smaller than a chimeric occurrence threshold as corresponding to a chimeric sequence of the one or more chimeric sequences of the target.

43. The method of embodiment 42, wherein a value of the chimeric occurrence threshold is an occurrence of a putative sequence of the putative sequences of the target associated with the one molecular label sequence that is greater than an occurrence of any other sequence of the putative sequences of the target.

44. The method of any one of embodiments 32-43, comprising:

determining a sequencing status of the target in the sequencing data; and
determining an occurrence of noise molecular label sequences associated with the putative sequences of the target in the sequencing data,
wherein the occurrence of the target estimated correlates with the occurrence of the molecular label sequences associated with the putative sequences of the target in the sequencing data adjusted according to the occurrence of the noise molecular label sequences.

45. The method of embodiment 44, wherein the sequencing status of the target in the sequencing data is saturated sequencing, under sequencing, or over sequencing.

46. The method of embodiment 45, wherein the under sequencing status is determined by the target having a depth smaller than a predetermined under sequencing threshold, and wherein the depth of the target comprises an average, a minimum, or a maximum depth of the molecular label sequences associated with the putative sequences of the target in the sequencing data.

47. The method of any one of embodiments 45-46, wherein the under sequencing threshold is about four.

48. The method of any one of embodiments 45-47, wherein the under sequencing threshold is independent of the number of molecular label sequences.

49. The method of any one of embodiments 45-48, wherein, if the sequencing status of the target in the sequencing data is the under sequencing status, the number of noise molecular label sequences determined is zero.

50. The method of any one of embodiments 45-49, wherein the saturated sequencing status is determined by the number of molecular label sequences associated with the putative sequences of the target greater than a saturation threshold.

51. The method of embodiment 50, wherein the saturation threshold is about 6557 if a molecular label sequence of the molecular label sequences associated with the putative sequences of the target has a sequence selected from about 6561 molecular label sequences.

52. The method of any one of embodiments 50-51, wherein the predetermined saturation threshold is about 65532 if a molecular label sequence of the molecular label sequences associated with the putative sequences of the target has a sequence selected from about 65536 molecular label sequences

EP 4 379 727 A1

53. The method of any one of embodiments 45-52, wherein, if the sequencing status of the target in the sequencing data is the saturated sequencing status, the number of noise molecular label sequences determined is zero.

54. The method of any one of embodiments 45-53, wherein the over sequencing status is determined by the target having a depth greater than a predetermined over sequencing threshold, wherein the depth of the target comprises an average, a minimum, or a maximum depth of the molecular label sequences associated with the putative sequences of the target in the sequencing data.

55. The method of embodiment 54, wherein the over sequencing threshold is about 250 if a molecular label sequence of the molecular label sequences associated with the putative sequences of the target has a sequence selected from about 6561 molecular label sequences.

56. The method of any one of embodiments 54-55, further comprising, if the sequencing status of the target in the sequencing data is saturated sequencing status or the over sequencing status:

subsampling the number of molecular label sequences associated with the sequences of the target in the sequencing data to about the predetermined over sequencing threshold.

57. The method of any one of embodiments 44-56, wherein determining the occurrence of noise molecular label sequences associated with the putative sequences of the target in the sequencing data comprises:
if a negative binomial distribution fitting condition is satisfied,

fitting a signal negative binomial distribution to the occurrences of the molecular label sequences associated with the sequences of the target in the sequencing data, wherein the signal negative binomial distribution corresponds to an occurrence of molecular label sequences associated with the sequences of the target in the sequencing data being signal molecular label sequences;
fitting a noise negative binomial distribution to the occurrences of the molecular label sequences associated with the sequences of the target in the sequencing data, wherein the noise negative binomial distribution corresponds to an occurrence of molecular label sequences associated with the sequences of the target in the sequencing data being noise molecular label sequences; and
determining the occurrence of the noise molecular label sequences using the signal negative binomial distribution and the noise negative binomial distribution.

58. The method of embodiment 57, wherein the negative binomial distribution fitting condition comprises: the sequencing status of the target in the sequencing data is not the under sequencing status or the over sequencing status.

59. The method of any one of embodiments 57-58, determining the number of noise molecular label sequences using the signal negative binomial distribution and the noise negative binomial distribution comprises:
for each of the molecular label sequences associated with the putative sequences of the target in the sequencing data:

determining a signal probability of the molecular label sequence to be in the signal negative binomial distribution;
determining a noise probability of the molecular label sequence to be in the noise negative binomial distribution; and
determining the molecular label sequence to be a noise molecular label if the signal probability is smaller than the noise probability.

60. The method of any one of embodiments 44-59, wherein determining the occurrence of noise molecular label sequences associated with the sequences of the target in the sequencing data comprises:
adding pseudopoints to the occurrence of molecular label sequences associated with the sequences of the target in the sequencing data prior to determining the occurrence of noise molecular label sequences associated with the sequences of the target in the sequencing data, if the sequencing status of the target in the sequencing data is not the under sequencing status or the over sequencing status and the occurrence of molecular label sequences associated with the sequences of the target in the sequencing data is less than a pseudopoints threshold.

61. The method of embodiment 60, wherein the pseudopoints threshold is ten.

62. The method of any one of embodiments 60-61, wherein determining the occurrence of noise molecular label sequences associated with the sequences of the target in the sequencing data comprises:
removing non-unique molecular label sequences when determining the occurrence of noise molecular label sequences associated with the sequences of the target in the sequencing data, if the sequencing status of the target in the sequencing data is not the under sequencing status or the over sequencing status and the occurrence of molecular label sequences associated with the sequences of the target in the sequencing data is not less than a pseudopoints threshold.

63. The method of any one of embodiments 32-62, wherein receiving the sequencing data of the plurality of targets comprises:

stochastically barcoding a plurality of targets using a plurality of stochastic barcodes to create a plurality of stochastically barcoded targets, wherein each of the plurality of stochastic barcodes comprises a cell label and a molecular label, wherein molecular labels of at least two stochastic barcodes of the plurality of stochastic barcodes comprise different molecular label sequences, and wherein at least two stochastic barcodes of the plurality of stochastic barcodes comprise cell labels with an identical cell label sequence; and
obtaining sequencing data of the stochastically barcoded targets.

64. The method of embodiment 63, wherein stochastically barcoding the plurality of targets in the plurality of cells using the plurality of stochastic barcodes to create the plurality of stochastically barcoded targets for the cells of the plurality of cells comprises:

stochastically barcoding the plurality of targets using the plurality of stochastic barcodes of a particle to create the plurality of stochastically barcoded targets,
wherein the particle comprises a subset of the plurality of stochastic barcodes, wherein each of the subset of stochastic barcodes comprise an identical cell label sequence and with at least 100 different molecular label sequences.

65. The method of embodiment 64, wherein the particle is a bead.
66. The method of embodiment 65, wherein the bead is selected from the group consisting of streptavidin beads, agarose beads, magnetic beads, conjugated beads, protein A conjugated beads, protein G conjugated beads, protein A/G conjugated beads, protein L conjugated beads, oligo(dT) conjugated beads, silica beads, silica-like beads, anti-biotin microbead, anti-fluorochrome microbead, and any combination thereof.
67. The method of any one of embodiments 63-67, wherein the particle comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof.
68. The method of any one of embodiments 63-67, wherein the stochastic barcodes of the particle comprise molecular labels with at least 1000 different molecular label sequences.
69. The method of any one of embodiments 63-68, wherein the stochastic barcodes of the particle comprise molecular labels with at least 10000 different molecular label sequences.
70. The method of any one of embodiments 63-69, wherein the molecular labels of the stochastic barcodes comprise random sequences.
71. The method of any one of embodiments 63-70, wherein the particle comprises at least 10000 stochastic barcodes.
72. The method of any one of embodiments 63-71, wherein stochastically barcoding the plurality of targets using the plurality of stochastic barcodes to create the plurality of stochastically barcoded targets comprises:

(i) contacting copies of the targets with target binding regions of the stochastic barcodes; and
(ii) reverse transcribing the plurality targets using the plurality of stochastic barcodes to create a plurality of reverse transcribed targets.

73. The method of any one of embodiments 63-72, comprising: prior to obtaining the sequencing data of the plurality of stochastically barcoded targets, amplifying the stochastically barcoded targets to generate a plurality of amplified stochastically barcoded targets.
74. The method of embodiment 73, wherein amplifying the stochastically barcoded targets to generate the plurality of amplified stochastically barcoded targets comprises: amplifying the stochastically barcoded targets by polymerase chain reaction (PCR).
75. A computer system for determining the occurrence of targets comprising:

a hardware processor; and
non-transitory memory having instructions stored thereon, which when executed by the hardware processor cause the processor to perform the method of any of embodiments 1-74.

76. A computer readable medium comprising a software program that comprises code for performing the method of any of embodiments 1-74.

# EP 4 379 727 A1

**Claims**

1. A computer-implemented method for determining occurrences of targets, comprising:

   (a) receiving sequencing data of a plurality of targets, wherein the sequencing data comprises putative sequences of a target of the plurality of targets and occurrences of molecular label sequences associated with the sequences of the target in the sequencing data;
   (b) collapsing putative sequences of the target using directional adjacency;
   (c) collapsing molecular label sequences associated with the putative sequences of the target using directional adjacency; and
   (d) estimating the occurrence of the target, wherein the occurrence of the target estimated correlates with the occurrence of molecular label sequences associated with the putative sequences of the target in the sequencing data after collapsing the occurrence of the putative sequences of the target in (b) and the occurrence of the molecular label sequences determined in (c).

2. The method of claim 1, comprising:

   identifying the sequences of the target in the sequencing data; and
   counting the occurrences of molecular label sequences associated with the sequences of the target in the sequencing data.

3. The method of claim 1 or 2, comprising:

   collapsing clusters of combination sequences using directional adjacency, wherein each combination sequence comprises a sequence of the sequences of the target and an associated molecular label sequence of the molecular label sequences,
   wherein the occurrence of the target estimated correlates with the occurrence of molecular label sequences associated with the sequences of the target in the sequencing data after collapsing the occurrence of the combination sequences.

4. The method of claim 3, wherein collapsing the clusters of combination sequences using directional adjacency comprises:

   identifying the clusters of combination sequences using directional adjacency; and
   collapsing the sequencing data using the clusters of combination sequences identified.

5. The method of any one of claims 1-4, wherein

   (a) collapsing the putative sequences of the target using directional adjacency comprises:

      identifying clusters of the putative sequences of the target using directional adjacency; and
      collapsing the sequencing data using the clusters of putative sequences of the target identified, and/or

   (b) collapsing the molecular label sequences associated with the putative sequences of the target using directional adjacency comprises:

      identifying clusters of the molecular label sequences associated with the putative sequences of the target using directional adjacency; and
      collapsing the sequencing data using the clusters of molecular label sequences associated with the putative sequences of the target identified.

6. The method of any one of claims 1-5, comprising:

   identifying one or more putative sequences of the target that correspond to one or more chimeric sequences of the target, wherein occurrences of the one or more putative sequences of the target that correspond to the one or more chimeric sequences of the target are smaller than occurrences of remaining one or more putative sequences of the target that do not correspond to the one or more chimeric sequences of the target; and
   removing from the sequencing data one or more putative sequences of the target that correspond to one or more chimeric sequences of the target.

7. The method of claim 6, wherein identifying the one or more putative sequences of the target corresponding to the one or more chimeric sequences of the target comprises: identifying putative sequences of the target associated with one molecular label sequence of the plurality of molecular sequences;

identifying a putative sequence of the putative sequences of the target associated with the one molecular label sequence with an occurrence smaller than a chimeric occurrence threshold as corresponding to a chimeric sequence of the one or more chimeric sequences of the target,
optionally, wherein a value of the chimeric occurrence threshold is an occurrence of a putative sequence of the putative sequences of the target associated with the one molecular label sequence that is greater than an occurrence of any other sequence of the putative sequences of the target.

8. The method of any one of claims 1-7, comprising:

determining a sequencing status of the target in the sequencing data; and
determining an occurrence of noise molecular label sequences associated with the putative sequences of the target in the sequencing data,
wherein the occurrence of the target estimated correlates with the occurrence of the molecular label sequences associated with the putative sequences of the target in the sequencing data adjusted according to the occurrence of the noise molecular label sequences,
optionally wherein the sequencing status of the target in the sequencing data is saturated sequencing, under sequencing, or over sequencing.

9. The method of claim 8, wherein

(a) the under sequencing status is determined by the target having a depth smaller than a predetermined under sequencing threshold, and wherein the depth of the target comprises an average, a minimum, or a maximum depth of the molecular label sequences associated with the putative sequences of the target in the sequencing data,
(b) the under sequencing threshold is independent of the number of molecular label sequences,
(c) if the sequencing status of the target in the sequencing data is the under sequencing status, the number of noise molecular label sequences determined is zero,
(d) the saturated sequencing status is determined by the number of molecular label sequences associated with the putative sequences of the target greater than a saturation threshold,
(e) if the sequencing status of the target in the sequencing data is the saturated sequencing status, the number of noise molecular label sequences determined is zero, and/or
(f) the over sequencing status is determined by the target having a depth greater than a predetermined over sequencing threshold, wherein the depth of the target comprises an average, a minimum, or a maximum depth of the molecular label sequences associated with the putative sequences of the target in the sequencing data,
optionally the method comprising subsampling the number of molecular label sequences associated with the sequences of the target in the sequencing data to about the predetermined over sequencing threshold.

10. The method of claim 8 or 9, wherein determining the occurrence of noise molecular label sequences associated with the putative sequences of the target in the sequencing data comprises:
if a negative binomial distribution fitting condition is satisfied,

fitting a signal negative binomial distribution to the occurrences of the molecular label sequences associated with the sequences of the target in the sequencing data, wherein the signal negative binomial distribution corresponds to an occurrence of molecular label sequences associated with the sequences of the target in the sequencing data being signal molecular label sequences;
fitting a noise negative binomial distribution to the occurrences of the molecular label sequences associated with the sequences of the target in the sequencing data, wherein the noise negative binomial distribution corresponds to an occurrence of molecular label sequences associated with the sequences of the target in the sequencing data being noise molecular label sequences; and
determining the occurrence of the noise molecular label sequences using the signal negative binomial distribution and the noise negative binomial distribution,
optionally wherein the negative binomial distribution fitting condition comprises: the sequencing status of the target in the sequencing data is not the under sequencing status or the over sequencing status,

optionally wherein determining the number of noise molecular label sequences using the signal negative binomial distribution and the noise negative binomial distribution comprises:

for each of the molecular label sequences associated with the putative sequences of the target in the sequencing data:

determining a signal probability of the molecular label sequence to be in the signal negative binomial distribution;

determining a noise probability of the molecular label sequence to be in the noise negative binomial distribution; and

determining the molecular label sequence to be a noise molecular label if the signal probability is smaller than the noise probability.

11. The method of any one of claims 8-10, wherein determining the occurrence of noise molecular label sequences associated with the sequences of the target in the sequencing data comprises:

adding pseudopoints to the occurrence of molecular label sequences associated with the sequences of the target in the sequencing data prior to determining the occurrence of noise molecular label sequences associated with the sequences of the target in the sequencing data, if the sequencing status of the target in the sequencing data is not the under sequencing status or the over sequencing status and the occurrence of molecular label sequences associated with the sequences of the target in the sequencing data is less than a pseudopoints threshold,

optionally wherein determining the occurrence of noise molecular label sequences associated with the sequences of the target in the sequencing data comprises:

removing non-unique molecular label sequences when determining the occurrence of noise molecular label sequences associated with the sequences of the target in the sequencing data, if the sequencing status of the target in the sequencing data is not the under sequencing status or the over sequencing status and the occurrence of molecular label sequences associated with the sequences of the target in the sequencing data is not less than a pseudopoints threshold.

12. The method of any one of claims 1-11, wherein receiving the sequencing data of the plurality of targets comprises:

stochastically barcoding a plurality of targets using a plurality of stochastic barcodes to create a plurality of stochastically barcoded targets, wherein each of the plurality of stochastic barcodes comprises a cell label and a molecular label, wherein molecular labels of at least two stochastic barcodes of the plurality of stochastic barcodes comprise different molecular label sequences, and wherein at least two stochastic barcodes of the plurality of stochastic barcodes comprise cell labels with an identical cell label sequence; and

obtaining sequencing data of the stochastically barcoded targets.

13. The method of claim 12, wherein

(a) stochastically barcoding the plurality of targets in the plurality of cells using the plurality of stochastic barcodes to create the plurality of stochastically barcoded targets for the cells of the plurality of cells comprises:

(1) stochastically barcoding the plurality of targets using the plurality of stochastic barcodes of a particle to create the plurality of stochastically barcoded targets,

wherein the particle comprises a subset of the plurality of stochastic barcodes, wherein each of the subset of stochastic barcodes comprise an identical cell label sequence and with at least 100 different molecular label sequences,

optionally wherein the particle is a bead, wherein the bead is selected from the group consisting of streptavidin beads, agarose beads, magnetic beads, conjugated beads, protein A conjugated beads, protein G conjugated beads, protein A/G conjugated beads, protein L conjugated beads, oligo(dT) conjugated beads, silica beads, silica-like beads, anti-biotin microbead, anti-fluorochrome microbead, and any combination thereof,

optionally wherein the particle comprises a material selected from the group consisting of polydimethylsiloxane (PDMS), polystyrene, glass, polypropylene, agarose, gelatin, hydrogel, paramagnetic, ceramic, plastic, glass, methylstyrene, acrylic polymer, titanium, latex, sepharose, cellulose, nylon, silicone, and any combination thereof, or

(2)

    (i) contacting copies of the targets with target binding regions of the stochastic barcodes; and
    (ii) reverse transcribing the plurality targets using the plurality of stochastic barcodes to create a plurality of reverse transcribed targets, or

(b) the method comprises prior to obtaining the sequencing data of the plurality of stochastically barcoded targets, amplifying the stochastically barcoded targets to generate a plurality of amplified stochastically barcoded targets.

**14.** A computer system for determining the occurrence of targets comprising:

a hardware processor; and
non-transitory memory having instructions stored thereon, which when executed by the hardware processor cause the processor to perform the method of any one of claims 1-13.

**15.** A computer readable medium comprising a software program that comprises code for performing the method of any one of claims 1-13.

EP 4 379 727 A1

| bead | 5'<br>amine | Universal<br>label | Dimension<br>label | Sample<br>label | Cell label | Molecular<br>label | Target-binding<br>region |
|---|---|---|---|---|---|---|---|

104

105

# FIG. 1

FIG. 2

1. RT

308 SEQ ID NO: 1

302

AAAAAAAAAAAAAAAAAAAA......

TTTTTTTTTTTTTTTTTTTTT

312 SEQ ID NO: 2

318  320  316

314

306

310

304

2. 1st PCR (pooled into 1 tube)

326A

326B

324

326C

318  320  316

306'

328

304

3. Nested PCR

334  332a

332b

330

332c

318  320  316

306''

336

328'

322

4. Index PCR (single tube)

332

318  320  316  336

344

340

342

338

*FIG. 3*

EP 4 379 727 A1

*FIG. 4*

EP 4 379 727 A1

500

504 — ( START )

508 — Determine molecular label counts of Target$_i$ (i = 1 … N$_{\text{# of target}}$)

512 — Determine directional adjacency between all molecular labels

516 — Determine clusters of molecular labels

520 — Collapse clusters to parent molecular labels

524 — Generate output

528 — ( END )

## FIG. 5

600

604 — START

608 — Determine raw molecular label (ML) counts

612 — Saturated?
(e.g., ML COUNT > 6557)

YES

NO

616 — Adjust ML counts by directional adjacency

620 — Determine sequencing status
(e.g., ML coverage)

624 — Undersequenced?
(E.g., ML Coverage < 4)

YES

NO

628 — Filter ML counts

632 — Unique ML < Threshold?
(E.g., Unique ML < 10)

YES

NO

636 — Add pseudopoints

Remove non-unique ML — 640

644 — Adjust ML counts by DBEC

648 — Generate output

652 — END

*FIG. 6*

| | CDR3 | | ML |
|---|---|---|---|

| V \| D* \| J* \| C | ML |
|---|---|

Adjust NS by RSEC

| V \| D \| J \| C | ML* |
|---|---|

Adjust ML by RSEC

| V \| D* \| J* \| C | ML* |
|---|---|

Adjust NS and ML by RSEC

| V \| D \| J \| C | ML |
|---|---|

*FIG. 7*

*FIG. 8*

*FIG. 9*

**FIG. 10**

EP 4 379 727 A1

1100

Processor 1102

Cache 1104

Processor Bus 1108

Memory Bus 1112

North Bridge 1106

RAM 1110

Chipset Bus 1116

South Bridge 1114

Peripheral Bus 1118

NIC 1120

NIC 1121

Accelerator 1122

Storage 1124

*FIG. 11*

**FIG. 12**

EP 4 379 727 A1

*FIG. 13*

FIG. 14

FIG. 15

ACTB

Negative Binomial Mixture

reads

Probability Density

*FIG. 16*

Hamming distance between ML before and after ML correction

FIG. 17

Number of molecules detected across whole plate

FIG. 18

EP 4 379 727 A1

SEQ ID NO: 3

| GTCAAA<u>TT</u> |
| 3 Reads |

SEQ ID NO: 4

| GTCAAAA<u>T</u> |
| 24 Reads |

Raw ML = 9

RSEC ML = 2

SEQ ID NO: 5

| <u>G</u>TCAAAAA |
| 74 Reads |

SEQ ID NO: 7

| TTCA<u>G</u>AAA |
| 1 Read |

SEQ ID NO: 6

| TTCAAAAA |
| 153 Reads |

SEQ ID NO: 8

| <u>C</u>TCAAAAA |
| 2 Reads |

SEQ ID NO: 6

| TTCAAA<u>C</u>A |
| 88 Reads |

SEQ ID NO: 9

| TTCAAA<u>CT</u> |
| 1 Read |

SEQ ID NO: 10

| TTCAAAA<u>T</u> |
| 4 Reads |

SEQ ID NO: 11

| TTCAAA<u>C</u>A |
| 88 Reads |

SEQ ID NO: 11

| TTCAAAAA |
| 263 Reads |

**FIG. 19**

2 Negative Binomial Distribution Fitted

$D_n$

$D_s$

Reads
Negative Binomial Mixture

*FIG. 20A*

Histogram of ML Depth

Cumulative Sum Plot of Reads

*FIG. 20B*

*FIG. 20C*

2 Negative Binomial Distribution Fitted

FIG. 21A

Histogram of ML Depth

CD3E

Cumulative Sum Plot of Reads

CD3E

FIG. 21B

FIG. 21C

*FIG. 22A*

Sis

*FIG. 22B*

Klf4

*FIG. 22C*

Sst

*FIG. 22D*

Ascl2

**FIG. 22E**

Alpi

**FIG. 22F**

Dpp4

**FIG. 22G**

Lgr5

**FIG. 22H**

Sox9

**FIG. 22I**

Xbp1

**FIG. 22J**

Reep5

**FIG. 23A**

Cell Clusters by DBScan

Clusters: BrCa, Jurkat1, Jurkat2, Low Signal

**FIG. 23B**

Gene: PSMB4.NM_002796

Log ML

**FIG. 23A** (right)

Cell Clusters by DBScan

Clusters: BrCa, Jurkat, Low Signal

**FIG. 23B** (right)

Gene: PSMB4.NM_002796

Log ML

FIG. 23C

FIG. 23D

FIG. 24A

FIG. 24B

FIG. 25A

Adjusted ML

Adjusted ML

FIG. 25B

CD3E

CD3E

FIG. 25C

FIG. 25D

*FIG. 26A*

FIG. 26B

| AA CDR3 | SEQ ID NO. | Nucleotide Sequence CDR3 | SEQ ID NO. | Molecular Label | SEQ ID NO. | Raw Reads | NS Adjusted Reads | ML Adjusted Reads | NS and ML Adjusted Reads | Chimera Removal |
|---|---|---|---|---|---|---|---|---|---|---|
| CVVNGDGTASKLTF | 12 | TGTGTGGTGAACGGAGACGGCACTGCCAGTAAACTCACCTTT | 58 | AGTGCGAG | 110 | 294 | 319 | 322 | 322 | 322 |
| CVVDGDGTASKLTF | 13 | TGTGTGGTGGACGGAGACGGCACTGCTAGTAAACTCACCTTT | 59 | AGTGCGAG | 110 | 1 | 0 | 0 | 0 | 0 |
| CVVSGDGTASKLTF | 14 | TGTGTGGTGAGCGGAGACGGCACTGCCAGTAAACTCACCTTT | 60 | AGTGCGAG | 110 | 1 | 0 | 0 | 0 | 0 |
| CVVNGVGTASKLTF | 15 | TGTGTGGTGAACGGAGTCGGCACTGCCAGTAAACTCACCTTT | 61 | AGTGCGAG | 110 | 1 | 0 | 0 | 0 | 0 |
| CVVNGDGAASKLTF | 16 | TGTGTGGTGAACGGAGACGGCGCTGCCAGTAAACTCACCTTT | 62 | AGTGCGAG | 110 | 1 | 0 | 0 | 0 | 0 |
| CVVNGDGTAGKLTF | 17 | TGTGTGGTGAACGGAGACGGCACTGCCGGTAAACTCACCTTT | 63 | AGTGCGAG | 110 | 1 | 0 | 0 | 0 | 0 |
| CVVNGDGTASRLTF | 18 | TGTGTGGTGAACGGAGACGGCACTGCCAGTAGACTCACCTTT | 64 | AGTGCGAG | 110 | 1 | 0 | 0 | 0 | 0 |
| CVVNGDGTASKLAF | 19 | TGTGTGGTGAACGGAGACGGCACTGCCAGTAAACTCGCCTTT | 65 | AGTGCGAG | 110 | 3 | 0 | 0 | 0 | 0 |
| CVVNGDGTASKLTF | 12 | TGTGTGGTGAACGGAGACGGCACTGCCAGTAAACTCACTTTT | 66 | AGTGCGAG | 110 | 1 | 0 | 0 | 0 | 0 |
| CVVNGDGTASKLTL | 20 | TGTGTGGTGAACGGAGACGGCACTGCCAGTAAACTCACCCTT | 67 | AGTGCGAG | 110 | 1 | 0 | 0 | 0 | 0 |
| CVVNGDGTASKPTF | 21 | TGTGTGGTGAACGGAGACGGCACTGCCAGTAAACCCACCTTT | 68 | AGTGCGAG | 110 | 1 | 0 | 0 | 0 | 0 |
| CVVNGDGTASKLTF | 12 | TGTGTGGTGAACGGAGACGGCACTGCCAGCAAACTCACCTTT | 69 | AGTGCGAG | 110 | 1 | 0 | 0 | 0 | 0 |
| CVVNGDGTTSKLTF | 22 | TGTGTGGTGAACGGAGACGGCACTACCAGTAAACTCACCTTT | 70 | AGTGCGAG | 110 | 1 | 0 | 0 | 0 | 0 |
| CVVNGDGTASKLTF | 12 | TGTGTGGTGAACGGAGACGGCACAGCCAGTAAACTCACCTTT | 71 | AGTGCGAG | 110 | 2 | 0 | 0 | 0 | 0 |
| CVVNEDGTASKLTF | 23 | TGTGTGGTGAACGAAGACGGCACTGCCAGTAAACTCACCTTT | 72 | AGTGCGAG | 110 | 1 | 0 | 0 | 0 | 0 |
| CVANGDGTASKLTF | 24 | TGTGTGGCGAACGGAGACGGCACTGCCAGTAAACTCACCTTT | 73 | AGTGCGAG | 110 | 2 | 0 | 0 | 0 | 0 |
| CVMNGDGTASKLTF | 25 | TGTGTGATGAACGGAGACGGCACTGCCAGTAAACTCACCTTT | 74 | AGTGCGAG | 110 | 1 | 0 | 0 | 0 | 0 |
| CAVNGDGTASKLTF | 26 | TGTGCGGTGAACGGAGACGGCACTGCCAGTAAACTCACCTTT | 75 | AGTGCGAG | 110 | 1 | 0 | 0 | 0 | 0 |
| CVVNGDGTASKLTF | 12 | TGCGTGGTGAACGGAGACGGCACTGCCAGTAAACTCACCTTT | 76 | AGTGCGAG | 110 | 2 | 0 | 0 | 0 | 0 |
| RVVNGDGTASKLTF | 27 | CGTGTGGTGAACGGAGACGGCACTGCCAGTAAACTCACCTTT | 77 | AGTGCGAG | 110 | 2 | 0 | 0 | 0 | 0 |
| CVVNGDGTASKLTF | 12 | TGTGTGGTGAACGGAGACGGCACTGCCAGTAAACTCACCTTT | 58 | AGTGCGGG | 111 | 2 | 2 | 0 | 0 | 0 |
| CVVNGDGTASKLTF | 12 | TGTGTGGTGAACGGAGACGGCACTGCCAGTAAACTCACCTTT | 58 | AGTGCNAG | 112 | 1 | 1 | 0 | 0 | 0 |
| CAVHRGSQGNLIF | 28 | TGTGCTGTCCACCGAGGAAGCCAAGGAAATCTCATCTTT | 78 | AGTGCGAG | 110 | 6 | 7 | 7 | 7 | 0 |
| CAVHRGSQGNLIV | 29 | TGTGCTGTCCACCGAGGAAGCCAAGGAAATCTCATCGTT | 79 | AGTGCGAG | 110 | 1 | 0 | 0 | 0 | 0 |
| CAGESGDYQKVTF | 30 | TGTGCAGGAGAATCTGGGGATTACCAGAAAGTTACCTTT | 80 | AGTGCGAG | 110 | 1 | 2 | 2 | 2 | 0 |
| CAGESGGYQKVTF | 31 | TGTGCAGGAGAATCTGGGGGTTACCAGAAAGTTACCTTT | 81 | AGTGCGAG | 110 | 1 | 0 | 0 | 0 | 0 |

**FIG. 27A**

EP 4 379 727 A1

| | | | | | | | | | | | |
|---|---|---|---|---|---|---|---|---|---|---|---|
| CAATESYGQNFVF | 32 | TGTGCAGCAACCGAGTCCTATGGTCAGAATTTTGTCTTT | 82 | AGTGCGAG | 110 | 2 | 3 | 3 | 3 | → | 0 |
| CAATESYGQNFVF | 32 | TGTGCAGCAACAGAGTCCTATGGTCAGAATTTTGTCTTT | 83 | AGTGCGAG | 110 | 1 | 0 | 0 | 0 | | 0 |
| CLVGSLSGSARQLTF | 33 | TGCCTCGTGGGGAGCCTTTCTGGTTCTGCAAGGCAACTGACCTTT | 84 | AGTGCGAG | 110 | 1 | 2 | 2 | 2 | → | 0 |
| CLVGSLSGSARQLTF | 33 | TGCCTCGTGGGGAGCCTTTCCGGTTCTGCAAGGCAACTGACCTTT | 85 | AGTGCGAG | 110 | 1 | 0 | 0 | 0 | | 0 |
| CVVTASGGYQKVTF | 34 | TGTGTGGTGACCGCTTCTGGGGGGTTACCAGAAAGTTACCTTT | 86 | AGTGCGAG | 110 | 1 | 1 | 1 | 1 | → | 0 |
| CAVAPYGNNRLAF | 35 | TGTGCTGTGGCCCCCTATGGGAACAACAGACTCGCTTTT | 87 | AGTGCGAG | 110 | 1 | 1 | 1 | 1 | → | 0 |
| CAVTRFSGGYNKLIF | 36 | TGTGCTGTGACTCGGTTTTCTGGTGGCTACAATAAGCTGATTTTT | 88 | AGTGCGAG | 110 | 1 | 1 | 1 | 1 | → | 0 |
| CAVSKGARSGNTGKLIF | 37 | TGTGCTGTCAGTAAGGGGGGCTAGGTCTGGCAACACAGGCAAACTAATCTTT | 89 | AGTGCGAG | 110 | 1 | 1 | 1 | 1 | → | 0 |
| CALFNNDMRF | 38 | TGTGCTCTGTTTAACAATGACATGCGCTTT | 90 | AGTGCGAG | 110 | 1 | 1 | 1 | 1 | → | 0 |
| CALSPGGYQKVTF | 39 | TGTGCTCTGTCCCCTGGGGGGTTACCAGAAAGTTACCTTT | 91 | AGTGCGAG | 110 | 1 | 1 | 1 | 1 | → | 0 |
| CAGLKLETSGSRLTF | 40 | TGTGCAGGGTTAAAACTAGAAACCAGTGGCTCTAGGTTGACCTTT | 92 | AGTGCGAG | 110 | 1 | 1 | 1 | 1 | → | 0 |
| CAGGYGNKLVF | 41 | TGTGCAGGGGGGTATGGAAACAAACTGGTCTTT | 93 | AGTGCGAG | 110 | 1 | 1 | 1 | 1 | → | 0 |
| CAGARGSNFGNEKLTF | 42 | TGTGCAGGAGCGAGGGGATCTAACTTTGGAAATGAGAAATTAACCTTT | 94 | AGTGCGAG | 110 | 1 | 1 | 1 | 1 | → | 0 |
| CAANNAGNVLTF | 43 | TGTGCAGCTAATAATGCAGGCAACGTGCTCACCTTT | 95 | AGTGCGAG | 110 | 1 | 1 | 1 | 1 | → | 0 |
| CAAPSLGGSARQLTF | 44 | TGTGCAGCCCCCTCCCTGGGGGGTTCTGCAAGGCAACTGACCTTT | 96 | AGTGCGAG | 110 | 1 | 1 | 1 | 1 | → | 0 |
| CAASIRGDSSYKLIF | 45 | TGTGCAGCAAGTATAAGGGGGGGATAGCAGCTATAAATTGATCTTC | 97 | AGTGCGAG | 110 | 1 | 1 | 1 | 1 | → | 0 |
| CAASRAD_GNQFYF | 46 | TGTGCAGCAAGTAGAGCCGACCGGTAACCAGTTCTATTTT | 98 | AGTGCGAG | 110 | 1 | 1 | 1 | 1 | → | 0 |
| CAASPMNRDDKIIF | 47 | TGTGCAGCAAGCCCAATGAACAGAGATGACAAGATCATCTTT | 99 | AGTGCGAG | 110 | 1 | 1 | 1 | 1 | → | 0 |
| CAASITDSWGKLQF | 48 | TGTGCAGCAAGCATAACTGACAGCTGGGGGAAATTGCAGTTT | 100 | AGTGCGAG | 110 | 1 | 1 | 1 | 1 | → | 0 |
| CVVSAKNTDKLIF | 49 | TGTGTGGTGAGCGCGAAAAACACCGACAAGCTCATCTTT | 101 | AGTGCGAG | 110 | 2 | 2 | 2 | 2 | → | 0 |
| CAYRSSNYQLIW | 50 | TGTGCTTATAGGAGTAGCAACTATCAGTTAATCTGG | 102 | AGTGCGAG | 110 | 2 | 2 | 2 | 2 | → | 0 |
| CAVVPFGGGGNKLTF | 51 | TGTGCTGTGGTCCCCTTCGGGGGGAGGAGGAAACAAACTCACCTTT | 103 | AGTGCGAG | 110 | 2 | 2 | 2 | 2 | → | 0 |
| CAGWSNDYKLSF | 52 | TGTGCAGGATGGTCTAACGACTACAAGCTCAGCTTT | 104 | AGTGCGAG | 110 | 2 | 2 | 2 | 2 | → | 0 |
| CAASGGSNYKLTF | 53 | TGTGCAGCAAGTGGAGGTAGCAACTATAAACTGACATTT | 105 | AGTGCGAG | 110 | 2 | 2 | 2 | 2 | → | 0 |
| CAMREGGGSNDYKLSF | 54 | TGTGCAATGAGAGAGGGCGGTGGTTCTAACGACTACAAGCTCAGCTTT | 106 | AGTGCGAG | 110 | 2 | 2 | 2 | 2 | → | 0 |
| CIRLPGNTGKLIF | 55 | TGCATCCGCCTGCCTGGCAACACAGGCAAACTAATCTTT | 107 | AGTGCGAG | 110 | 2 | 2 | 2 | 2 | → | 0 |
| CAYVAAAGNKLTF | 56 | TGTGCTTATGTCGCAGCTGCAGGCAACAAGCTAACTTTT | 108 | AGTGCGAG | 110 | 3 | 3 | 3 | 3 | → | 0 |
| CAGAPGSYIPTF | 57 | TGTGCAGGAGCCCCAGGAAGCTACATACCTACATTT | 109 | AGTGCGAG | 110 | 3 | 3 | 3 | 3 | → | 0 |
| CAGAPGSYIPTF | 57 | TGTGCAGGAGCCCCAGGAAGCTACATACCTACATTT | 109 | ATAGAGAT | 113 | 1 | 1 | 1 | 1 | | 1 |

## FIG. 27B

FIG. 28

Europäisches
Patentamt

European
Patent Office

Office européen
des brevets

# EUROPEAN SEARCH REPORT

Application Number

EP 23 20 9657

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/118719 A1 (QIAGEN SCIENCES LLC [US]) 28 July 2016 (2016-07-28) | 1,2,5, 8-11,14, 15 | **INV.** G16B40/00 G16B40/20 C12Q1/6848 C12Q1/6869 |
| Y | * p.1 l.15-16, p.3 l.11-14, p.7 l.19-22, p.12 l.5-22, p.13 l.17-29, p.23 l.17-18, p.29 l.18-p.30 l.17, p.31 l.7-14, p.32 l.2-3; table 1 * | 3,4,12, 13 | |
| Y,D | WO 2015/031691 A1 (CELLULAR RES INC [US]) 5 March 2015 (2015-03-05) * [0003], [0010], [00582]; claims 1-3; figure 55 * | 3,4,12, 13 | |
| Y | US 2016/333417 A1 (TALASAZ AMIRALI [US]) 17 November 2016 (2016-11-17) * [0004], [0037], [0085], [0089], [0090], [0137], [0139], [0159] * | 3,4 | |
| Y | MACOSKO E Z ET AL: "Highly Parallel Genome-wide Expression Profiling of Individual Cells Using Nanoliter Droplets", CELL, vol. 161, no. 5, 1 May 2015 (2015-05-01), pages 1202-1214, XP055323004, AMSTERDAM, NL ISSN: 0092-8674, DOI: 10.1016/j.cell.2015.05.002 * abstract; figures 2C-D * * Supplemental Information, "Read Alignment and Generation of Digital Expression Data". * | 12,13 | |

-/--

| | | | TECHNICAL FIELDS SEARCHED (IPC) |
|---|---|---|---|
| | | | G06F C12Q |

The present search report has been drawn up for all claims

| Place of search | Date of completion of the search | Examiner |
|---|---|---|
| Munich | 26 April 2024 | Werner, Andreas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

**EP 23 20 9657**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | SMITH T ET AL: "UMI-tools: modeling sequencing errors in Unique Molecular Identifiers to improve quantification accuracy", GENOME RESEARCH, vol. 27, no. 3, 18 January 2017 (2017-01-18), pages 491-499, XP055517852, US ISSN: 1088-9051, DOI: 10.1101/gr.209601.116 * abstract * * p.493 col.1 par.1-col.2 par.1; figure 1E * * p.495-496, "Comparing methods with single-cell RNA-seq data" * * p.497 col.2 par.7-p.498 col.1 par.3, "Single-cell RNA-seq" * -& SMITH T ET AL: "Supplementary Figures. UMI-tools: modeling sequencing errors in Unique Molecular Identifiers to improve quantification accuracy.", GENOME RESEARCH, 18 January 2017 (2017-01-18), pages 1-5, XP055535791, Retrieved from the Internet: URL:https://genome.cshlp.org/content/suppl /2017/02/23/gr.209601.116.DC1/Supplementar y_Figures.pdf [retrieved on 2018-12-18] ----- -/-- | 12,13 | |
| | | | **TECHNICAL FIELDS SEARCHED (IPC)** |

The present search report has been drawn up for all claims

| Place of search | Date of completion of the search | Examiner |
|---|---|---|
| Munich | 26 April 2024 | Werner, Andreas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

**EP 23 20 9657**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | KLEIN A M ET AL: "Droplet Barcoding for Single-Cell Transcriptomics Applied to Embryonic Stem Cells", CELL, CELL PRESS, AMSTERDAM, NL, vol. 161, no. 5, 21 May 2015 (2015-05-21), pages 1187-1201, XP029129138, ISSN: 0092-8674, DOI: 10.1016/J.CELL.2015.04.044 * the whole document * * figure 1 * | | |
| A | PENG Q ET AL: "Reducing amplification artifacts in high multiplex amplicon sequencing by using molecular barcodes", BMC GENOMICS, BIOMED CENTRAL LTD, LONDON, UK, vol. 16, no. 1, 7 August 2015 (2015-08-07), page 589, XP021224943, ISSN: 1471-2164, DOI: 10.1186/S12864-015-1806-8 * abstract * * p.2, par. across col.1-2, p.6 col.1 par.3, p.6 col.2 par.3-p.7 col.2 par.1, p.8 col.2 par.2, p.8 col.2 par.2-p.9 col.1 par.2 * | 1-15 | |

| | |
|---|---|
| | TECHNICAL FIELDS SEARCHED (IPC) |

The present search report has been drawn up for all claims

| Place of search | Date of completion of the search | Examiner |
|---|---|---|
| Munich | 26 April 2024 | Werner, Andreas |

EPO FORM 1503 03.82 (P04C01)

## ANNEX TO THE EUROPEAN SEARCH REPORT
## ON EUROPEAN PATENT APPLICATION NO.

EP 23 20 9657

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2024

| Patent document cited in search report | | Publication date | Patent family member(s) | | Publication date |
|---|---|---|---|---|---|
| WO 2016118719 | A1 | 28-07-2016 | EP | 3247804 A1 | 29-11-2017 |
| | | | EP | 3763825 A1 | 13-01-2021 |
| | | | US | 2018002738 A1 | 04-01-2018 |
| | | | US | 2020354773 A1 | 12-11-2020 |
| | | | WO | 2016118719 A1 | 28-07-2016 |
| WO 2015031691 | A1 | 05-03-2015 | AU | 2014312208 A1 | 10-03-2016 |
| | | | AU | 2019204928 A1 | 25-07-2019 |
| | | | AU | 2022291534 A1 | 02-02-2023 |
| | | | CN | 105705659 A | 22-06-2016 |
| | | | CN | 110964796 A | 07-04-2020 |
| | | | DK | 3039158 T3 | 04-03-2019 |
| | | | EP | 3039158 A1 | 06-07-2016 |
| | | | EP | 3480321 A1 | 08-05-2019 |
| | | | EP | 3842542 A1 | 30-06-2021 |
| | | | ES | 2711168 T3 | 30-04-2019 |
| | | | ES | 2857908 T3 | 29-09-2021 |
| | | | GB | 2525104 A | 14-10-2015 |
| | | | GB | 2546833 A | 02-08-2017 |
| | | | JP | 6545682 B2 | 17-07-2019 |
| | | | JP | 6891199 B2 | 18-06-2021 |
| | | | JP | 2016533187 A | 27-10-2016 |
| | | | JP | 2019106998 A | 04-07-2019 |
| | | | KR | 20160055168 A | 17-05-2016 |
| | | | KR | 20200133281 A | 26-11-2020 |
| | | | KR | 20220075240 A | 07-06-2022 |
| | | | KR | 20230074639 A | 30-05-2023 |
| | | | SG | 10201806890V A | 27-09-2018 |
| | | | SG | 11201601188T A | 30-03-2016 |
| | | | US | 2015299784 A1 | 22-10-2015 |
| | | | US | 2016010151 A1 | 14-01-2016 |
| | | | US | 2016145683 A1 | 26-05-2016 |
| | | | US | 2016265069 A1 | 15-09-2016 |
| | | | US | 2018002764 A1 | 04-01-2018 |
| | | | US | 2018258500 A1 | 13-09-2018 |
| | | | US | 2018291470 A1 | 11-10-2018 |
| | | | US | 2018327866 A1 | 15-11-2018 |
| | | | US | 2018327867 A1 | 15-11-2018 |
| | | | US | 2019040474 A1 | 07-02-2019 |
| | | | US | 2019085412 A1 | 21-03-2019 |
| | | | US | 2021198754 A1 | 01-07-2021 |
| | | | US | 2023295742 A1 | 21-09-2023 |
| | | | WO | 2015031691 A1 | 05-03-2015 |
| US 2016333417 | A1 | 17-11-2016 | US | 2014066317 A1 | 06-03-2014 |
| | | | US | 2016333417 A1 | 17-11-2016 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 23 20 9657

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2024

| Patent document cited in search report | Publication date | Patent family member(s) | Publication date |
|---|---|---|---|
|  |  | US 2019078164 A1 | 14-03-2019 |
|  |  | US 2024102101 A1 | 28-03-2024 |

EPO FORM P0459

For more details about this annex : see Official Journal of the European Patent Office, No. 12/82

**REFERENCES CITED IN THE DESCRIPTION**

**Patent documents cited in the description**

- US 62562978
- US 20150299784 A
- WO 2015031691 A

**Non-patent literature cited in the description**

- **SINGLETON et al.** Dictionary of Microbiology and Molecular Biology 2nd. J. Wiley & Sons, 1994
- **SAMBROOK et al.** Molecular Cloning, A Laboratory Manual. Cold Spring Harbor Press, 1989
- **FU et al.** *Proc Natl Acad Sci U.S.A.,* 31 May 2011, vol. 108 (22), 9026-31